(19) Europäisches Patentamt / European Patent Office / Office européen des brevets

(11) **EP 4 077 315 B1**

(12) **EUROPÄISCHE PATENTSCHRIFT**


(45) Veröffentlichungstag und Bekanntmachung des Hinweises auf die Patenterteilung:
**03.01.2024 Patentblatt 2024/01**

(21) Anmeldenummer: **20824236.2**

(22) Anmeldetag: **15.12.2020**

(51) Internationale Patentklassifikation (IPC):
***C07D 405/14*** *(2006.01)* ***C07D 409/14*** *(2006.01)*
***H10K 85/60*** *(2023.01)* ***H10K 101/10*** *(2023.01)*
***H10K 101/20*** *(2023.01)*

(52) Gemeinsame Patentklassifikation (CPC):
**C07D 405/14; C07D 409/14; H10K 85/654; H10K 85/6572; H10K 85/6574;** H10K 50/11; H10K 50/16; H10K 2101/10; H10K 2101/20

(86) Internationale Anmeldenummer:
**PCT/EP2020/086130**

(87) Internationale Veröffentlichungsnummer:
**WO 2021/122535 (24.06.2021 Gazette 2021/25)**


(54) **MATERIALIEN FÜR ORGANISCHE ELEKTROLUMINESZENZVORRICHTUNGEN**

MATERIALS FOR ORGANIC ELECTROLUMINESCENT APPARATUS

MATÉRIAUX POUR DISPOSITIFS ÉLECTROLUMINESCENTS ORGANIQUES


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2019 EP 19216933**

(43) Veröffentlichungstag der Anmeldung:
**26.10.2022 Patentblatt 2022/43**



(73) Patentinhaber: **Merck Patent GmbH**
**64293 Darmstadt (DE)**

(72) Erfinder:
- **LINGE, Rouven**
  **64293 DARMSTADT (DE)**
- **PARHAM, Amir Hossain**
  **64293 DARMSTADT (DE)**
- **MEYER, Sebastian**
  **64293 DARMSTADT (DE)**
- **HAYER, Anna**
  **64293 DARMSTADT (DE)**
- **KOENEN, Nils**
  **64293 DARMSTADT (DE)**

(74) Vertreter: **Merck Patent Association**
**Merck Patent GmbH**
**64271 Darmstadt (DE)**



(56) Entgegenhaltungen:
**WO-A1-2015/169412**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung beschreibt Dibenzofuran- und Dibenzothiophenderivate, welche mit elektronenarmen Heteroaromaten substituiert sind, und elektronische Vorrichtungen enthaltend diese Verbindungen, insbesondere organische Elektrolumineszenzvorrichtungen enthaltend diese Verbindungen als Triplettmatrixmaterialien.

In organischen Elektrolumineszenzvorrichtungen (OLEDs) werden häufig phosphoreszierende metallorganische Komplexe eingesetzt. Generell gibt es bei OLEDs immer noch Verbesserungsbedarf. Dabei werden die Eigenschaften phosphoreszierender OLEDs nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Carbazolderivate, und Dibenzofuranderivate, insbesondere solche, die mit elektronenarmen Heteroaromaten wie Triazin substituiert sind, als Matrixmaterialien für phosphoreszierende Emitter verwendet, wie zum Beispiel in WO 2015/169412 offenbart. Generell besteht bei diesen Materialklassen, insbesondere wenn diese aus Lösung verarbeitet werden sollen, noch Verbesserungsbedarf bezüglich der Löslichkeit und der Lebensdauer. Aufgabe der vorliegenden Erfindung ist daher die Bereitstellung von Verbindungen, welche sich gut aus Lösung verarbeiten lassen und als Matrixmaterial in phosphoreszierenden OLEDs, welche aus Lösung hergestellt werden, zu einer Verbesserung der Lebensdauer führen.

Es wurde überraschend gefunden, dass organische Elektrolumineszenzvorrichtungen, die Verbindungen gemäß der folgenden Formel (1) enthalten, eine hohe Löslichkeit und eine hohe Glasübergangstemperatur aufweisen, sich dadurch sehr gut aus Lösung verarbeiten lassen und beim Einsatz als Matrixmaterial für phosphoreszierende Dotanden eine verbesserte Lebensdauer gegenüber dem Stand der Technik aufweisen. Gegenstand der Erfindung ist daher eine Verbindung gemäß der folgenden Formel (1),

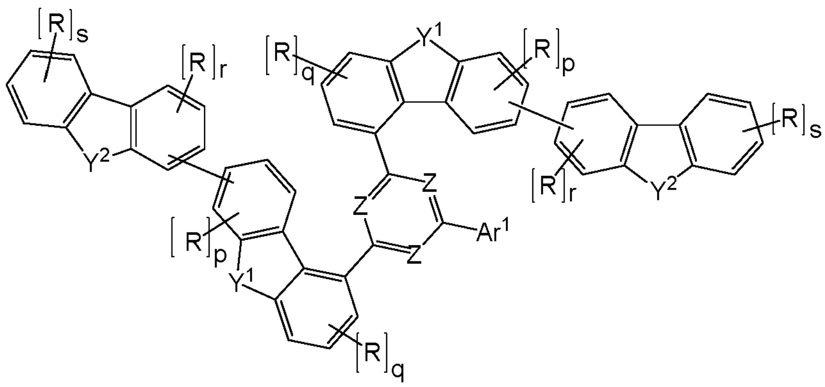

Formel (1)

wobei für die verwendeten Symbole und Indizes gilt:

- $Y^1$ ist bei jedem Auftreten gleich oder verschieden O oder S;
- $Y^2$ ist bei jedem Auftreten gleich oder verschieden $NAr^2$, O, S oder $CR_2$;
- Z ist bei jedem Auftreten gleich oder verschieden CR oder N mit der Maßgabe, dass mindestens zwei Z für N stehen;
- $Ar^1$, $Ar^2$ ist bei jedem Auftreten gleich oder verschieden ein aromatisches oder heteroaromatisches Ringsystem mit 5 bis 40 aromatischen Ringatomen, welches mit einem oder mehreren Resten R substiutiert sein kann;
- R ist bei jedem Auftreten gleich oder verschieden H, D, F, Cl, Br, I, $N(Ar')_2$, $N(R^1)_2$, OAr', SAr', CN, $NO_2$, $OR^1$, $SR^1$, $COOR^1$, $C(=O)N(R^1)_2$, $Si(R^1)_3$, $B(OR^1)_2$, $C(=O)R^1$, $P(=O)(R^1)_2$, $S(=O)R^1$, $S(=O)_2R^1$, $OSO_2R^1$, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^1$ substituiert sein kann, wobei eine oder mehrere nicht benachbarte $CH_2$-Gruppen durch $Si(R^1)_2$, C=O, $NR^1$, O, S oder $CONR^1$ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste $R^1$ substituiert sein kann;

dabei können zwei Reste R auch miteinander ein Ringsystem bilden;

Ar' ist bei jedem Auftreten gleich oder verschieden ein aromatisches oder heteroaromatisches Ringsystem mit 5 bis 40 aromatischen Ringatomen, das durch einen oder mehrere Reste $R^1$ substituiert sein kann; dabei können zwei Reste Ar', welche an dasselbe N-Atom binden, auch durch eine Einfachbindung oder eine Brücke, ausgewählt aus $N(R^1)$, $C(R^1)_2$, O oder S, miteinander verbrückt sein;

$R^1$ ist bei jedem Auftreten gleich oder verschieden H, D, F, Cl, Br, I, $N(R^2)_2$, CN, $NO_2$, $OR^2$, $SR^2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^2$ substituiert sein kann, wobei eine oder mehrere nicht benachbarte $CH_2$-Gruppen durch $Si(R^2)_2$, C=O, $NR^2$, O, S oder $CONR^2$ ersetzt sein können und wobei ein oder mehrere H-Atome in der Alkyl-, Alkenyl- oder Alkinylgruppe durch D, F, Cl, Br, I oder CN ersetzt sein können, oder ein aromatisches oder heteroaromatisches Ringsystem mit 5 bis 40 aromatischen Ringatomen, das jeweils durch einen oder mehrere Reste $R^2$ substituiert sein kann; dabei können zwei oder mehrere Reste $R^1$ miteinander ein aliphatisches Ringsystem bilden;

$R^2$ ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;

p, q, r ist bei jedem Auftreten gleich oder verschieden 0, 1, 2 oder 3;

s ist bei jedem Auftreten gleich oder verschieden 0, 1, 2, 3 oder 4.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 39 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw.

Heteroarylgruppen sowie Gruppen, in denen zwei oder mehr Aryl- bzw. Heteroarylgruppen direkt miteinander verknüpft sind, beispielsweise Biphenyl oder Bipyridin, sowie Fluoren oder Spirobifluoren.

Im Rahmen der vorliegenden Erfindung wird der Begriff Alkylgruppe als Oberbegriff für lineare, verzweigte und cyclische Alkylgruppen verwendet. Analog werden die Begriffe Alkenylgruppe bzw. Alkinylgruppe als Oberbegriffe sowohl für lineare wie auch für verzweigte und cyclische Alkenyl- bzw. Alkinylgruppen verwendet.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder $CH_2$-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer

Alkoxygruppe $OR^1$ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe $SR^1$ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte $CH_2$-Gruppen durch die oben genannten Gruppen ersetzt sein können; weiterhin können auch ein oder mehrere H-Atome durch D, F, Cl, Br, I, CN oder $NO_2$, bevorzugt F, Cl oder CN, besonders bevorzugt F oder CN ersetzt sein.

Unter einem aromatischen oder heteroaromatischen Ringsystem mit 5 - 40 aromatischen Ringatomen, welches noch jeweils mit den oben genannten Resten $R^2$ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter der Formulierung, dass zwei oder mehr Reste miteinander ein Ringsystem bilden können, wird die Bildung eines aliphatischen, heteroaliphatischen, aromatischen oder heteroaromatischen Ringsystems verstanden, und es soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

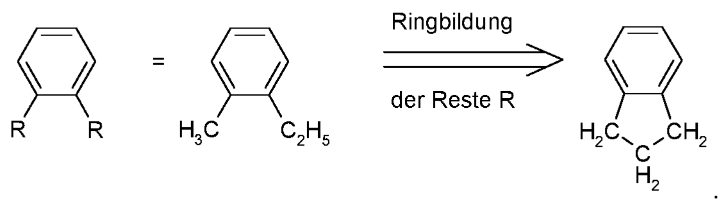


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

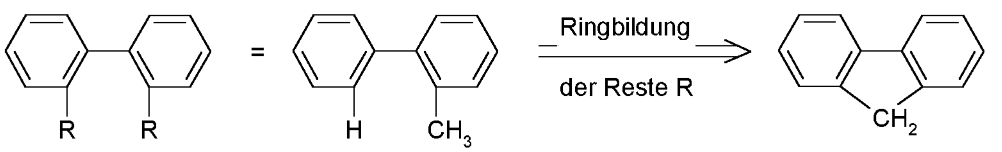


In einer bevorzugten Ausführungsform der Erfindung ist die Gruppe

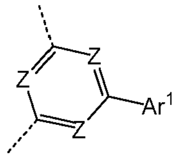,

die in Formel (1) gebunden ist, ausgewählt aus den folgenden Gruppen (HetAr-1), (HetAr-2) oder (HetAr-3),

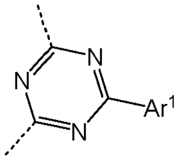

(HetAr-1)

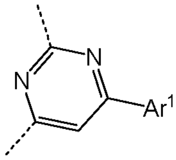

(HetAr-2)

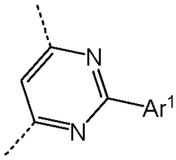

(HetAr-3)

wobei $Ar^1$ die oben genannten Bedeutungen aufweist und die gestrichelte Bindung die Verknüpfung dieser Gruppe innerhalb der Verbindung der Formel (1) andeutet.

In einer bevorzugten Ausführungsform der Erfindung stehen alle drei Gruppen Z für N, so dass es sich bevorzugt um eine Verbindung der folgenden Formel (2) handelt,

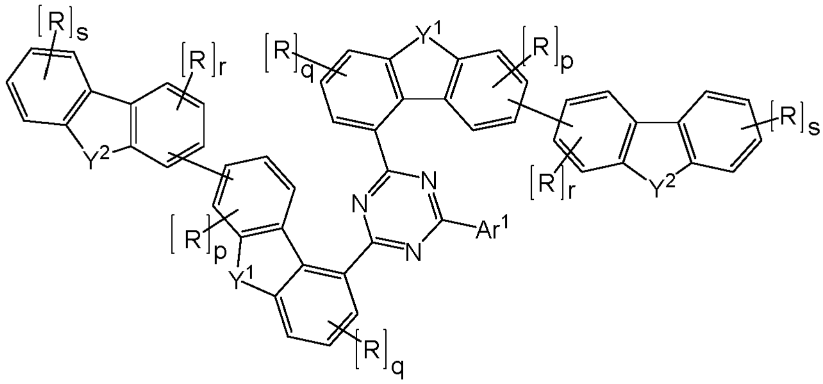

Formel (2)

wobei die verwendeten Symbole und Indizes die oben genannten Bedeutungen aufweisen.

In einer weiteren bevorzugten Ausführungsform der Erfindung steht $Y^1$ für O, so dass es sich um eine Verbindung der folgenden Formel (3) handelt,

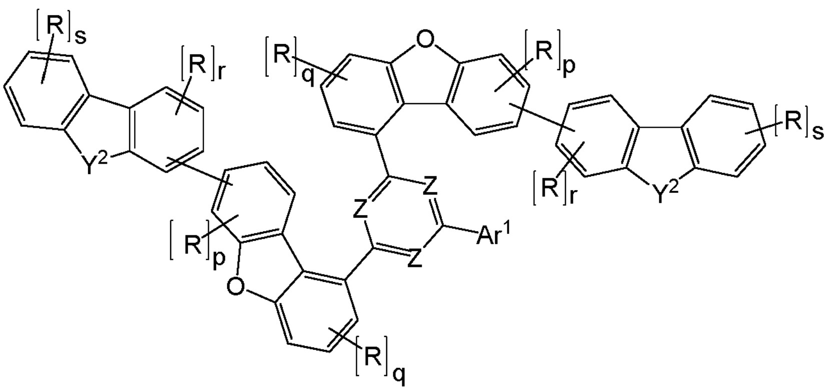

Formel (3)

wobei die verwendeten Symbole und Indizes die oben genannten Bedeutungen aufweisen.

In einer weiteren bevorzugten Ausführungsform der Erfindung steht $Y^2$ für $NAr^2$, so dass es sich um eine Verbindung gemäß der folgenden Formel (4) handelt,

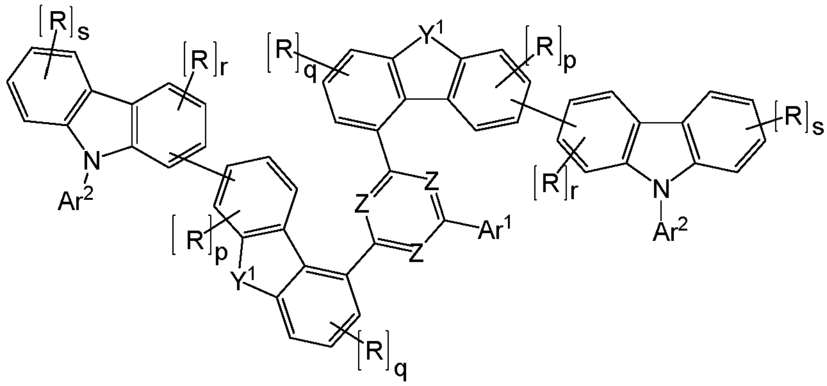

Formel (4)

wobei die verwendeten Symbole und Indizes die oben genannten Bedeutungen aufweisen.

Wenn $Y^2$ für $NAr^2$ steht und die Carbazolgruppe mindestens zwei benachbarte Reste R aufweist, die miteinander ein Ringsystem bilden, so sind bevorzugte Carbazolgruppen gewählt aus den Gruppen der folgenden Formeln (CARB-1) bis (CARB-6),

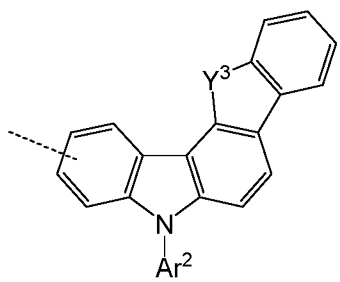

(CARB-1)

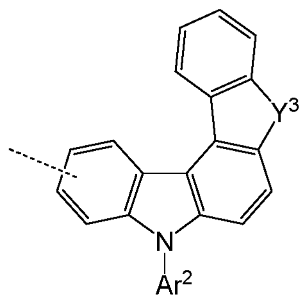

(CARB-2)

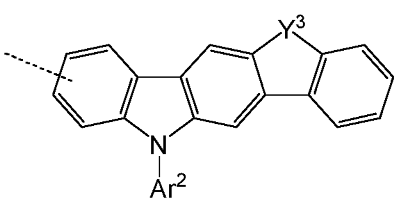

(CARB-3)

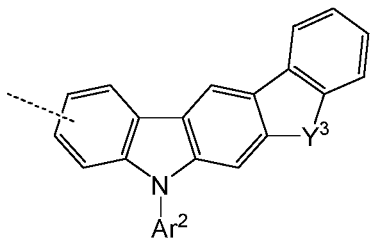

(CARB-4)

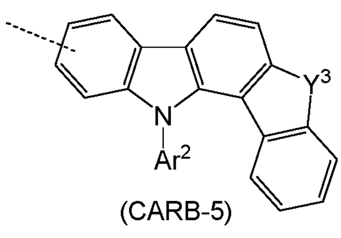

(CARB-5)

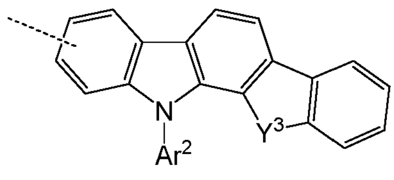

(CARB-6)

wobei die Strukturen jeweils am Carbazol durch einen oder mehrere Reste R und an der ankondensierten Struktur durch einen oder mehrere Reste $R^1$ substituiert sein können, bevorzugt aber unsubstituiert sind, $Y^3$ für $C(R^1)_2$, $NR^1$, O oder

S steht, bevorzugt für $C(R^1)_2$ oder $NR^1$ und besonders bevorzugt für $C(R^1)_2$ und die Strukturen über die gestrichelte Bindung mit dem Dibenzofuran bzw. Dibenzothiophen verknüpft sind.

In einer weiteren bevorzugten Ausführungsform der Erfindung stehen alle Z für N, und gleichzeitig stehen alle $Y^1$ für O. In nochmals einer weiteren bevorzugten Ausführungsform der Erfindung stehen alle Z für N, und gleichzeitig stehen alle $Y^2$ für $NAr^2$. In nochmals einer weiteren bevorzugten Ausführungsform der Erfindung stehen alle $Y^1$ für O, und gleichzeitig stehen alle $Y^2$ für $NAr^2$. In einer besonders bevorzugten Ausführungsform der Erfindung stehen alle Z für N, und gleichzeitig stehen alle $Y^1$ für O, und gleichzeitig stehen alle $Y^2$ für $NAr^2$, so dass es sich um eine Verbindung der folgenden Formel (5) handelt,

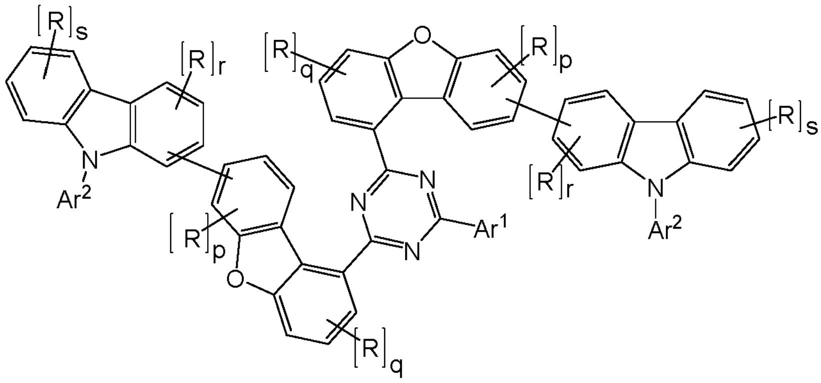

Formel (5)

wobei die verwendeten Symbole und Indizes die oben genannten Bedeutungen aufweisen.

Dabei erfolgt die Verknüpfung der Gruppe enthaltend $Y^1$, also dem Dibenzofuran bzw. Dibenzothiophen, bevorzugt über die 8-Position, also die Position para zu $Y^1$. Weiterhin erfolgt die Verknüpfung der Gruppe enthaltend $Y^2$ bevorzugt über die 3-Position für $Y^2 = NAr^2$ und über die 2-Position für $Y^2$ = O oder S, also jeweils über die Position para zu $Y^2$ und über die 2-Position für $Y^2 = CR_2$.

Die Nummerierung des Dibenzofurans und des Carbazols ist dabei im folgenden Schema dargestellt, wobei die Nummerierung des Dibenzothiophens analog zum Dibenzofuran und die Nummerierung des Fluorens analog zum Carbazol erfolgt:

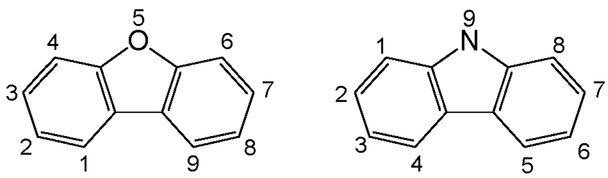

Bevorzugte Ausführungsformen der Verbindungen der Formeln (1) bis (5) sind somit die Verbindungen der folgenden Formeln (1a) bis (5a),

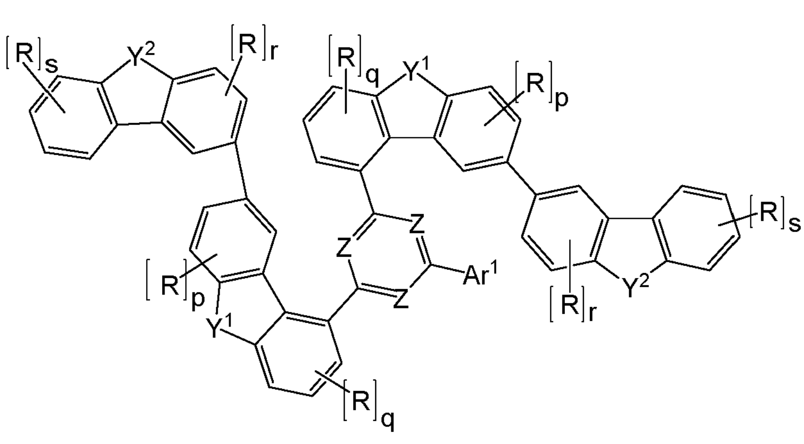

Formel (1a)

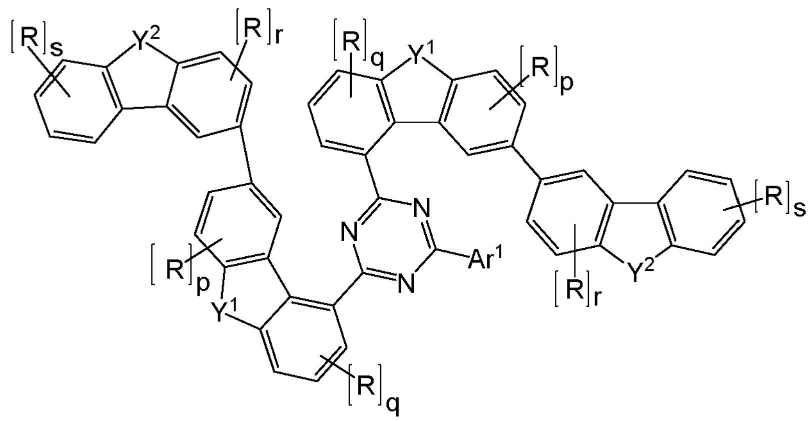

Formel (2a)

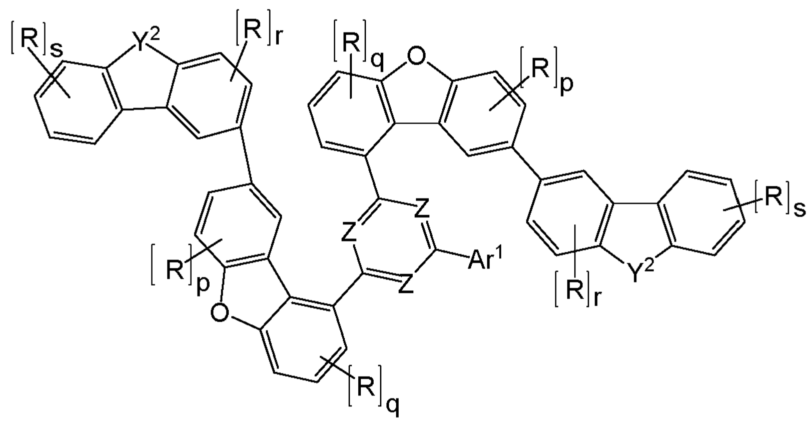

Formel (3a)

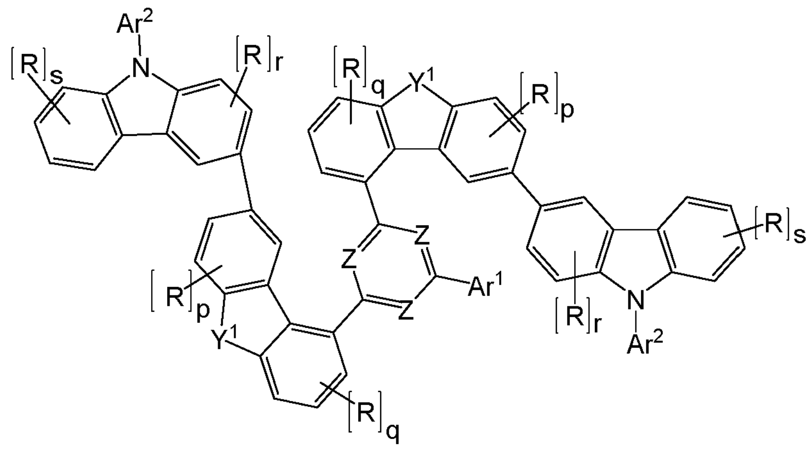

Formel (4a)

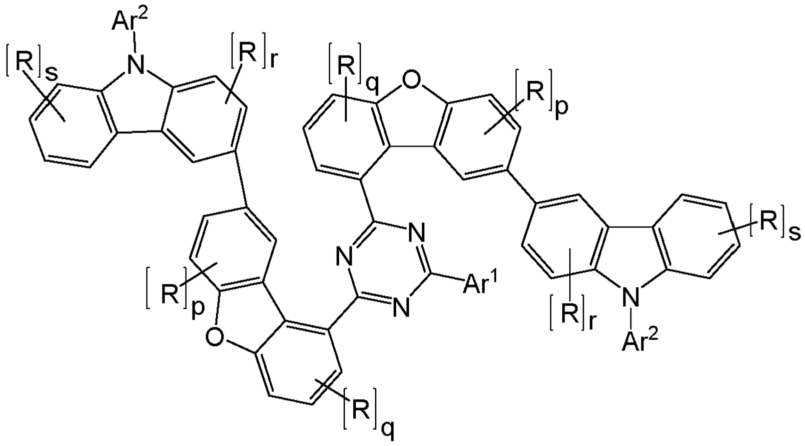

Formel (5a)

wobei die verwendeten Symbole und Indizes die oben genannten Bedeutungen aufweisen.

In einer weiteren bevorzugten Ausführungsform der Verbindungen gemäß Formel (1), (2), (3), (4), (5), (1a), (2a), (3a), (4a) und (5a) stehen p, q und r gleich oder verschieden bei jedem Auftreten für 0 oder 1, und besonders bevorzugt für 0. In nochmals einer weiteren bevorzugten Ausführungsform der Verbindungen gemäß Formel (1), (2), (3), (4), (5), (1a), (2a), (3a), (4a) und (5a) steht s gleich oder verschieden bei jedem Auftreten für 0, 1 oder 2, besonders bevorzugt für 0 oder 1 und ganz besonders bevorzugt für 0. Besonders bevorzugt handelt es sich somit um eine Verbindung der folgenden Formel (6),

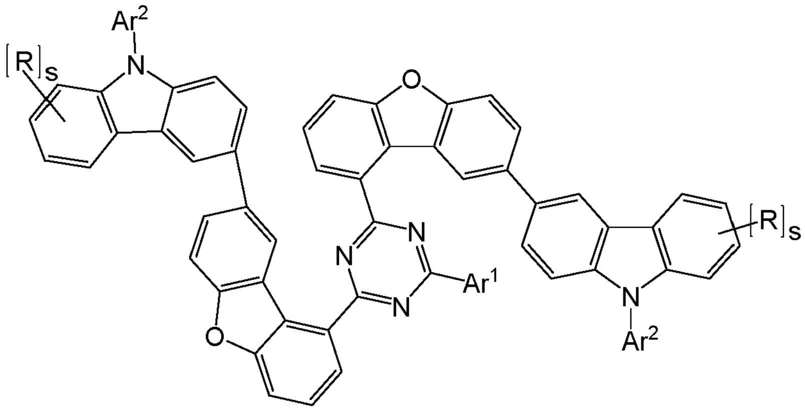

Formel (6)

wobei die verwendeten Symbole die oben genannten Bedeutungen aufweisen und s gleich oder verschieden bei jedem Auftreten 0 oder 1 ist.

Eine besonders bevorzugte Ausführungsform ist die Verbindung der folgenden Formel (6a),

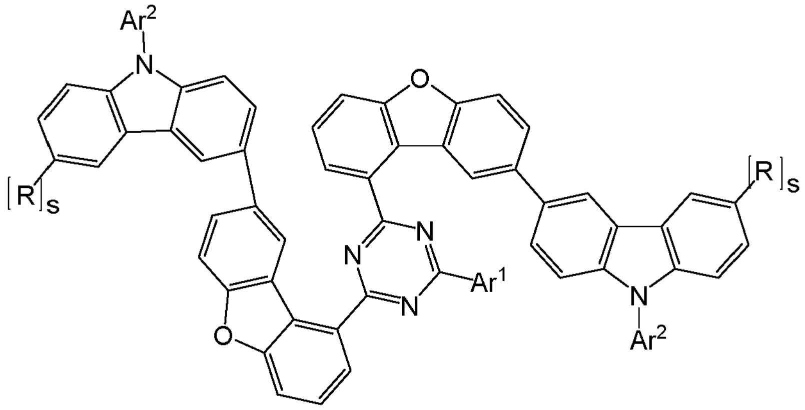

Formel (6a)

wobei die verwendeten Symbole die oben genannten Bedeutungen aufweisen und s gleich oder verschieden bei jedem Auftreten 0 oder 1 ist, bevorzugt 0.

In einer weiteren bevorzugten Ausführungsform der Erfindung ist $Ar^1$, also der Substituent am Triazin bzw. Pyrimidin, gleich oder verschieden bei jedem Auftreten ein aromatisches oder heteroaromatisches Ringsystem mit 6 bis 30 aromatischen Ringatomen, das durch einen oder mehrere Reste R substituiert sein kann. Besonders bevorzugt ist $Ar^1$ gleich oder verschieden bei jedem Auftreten ein aromatisches oder heteroaromatisches Ringsystem, insbesondere ein aromatisches Ringsystem, mit 6 bis 24 aromatischen Ringatomen, insbesondere mit 6 bis 13 aromatischen Ringatomen, das durch einen oder mehrere, bevorzugt nicht-aromatische, Reste R substituiert sein kann. Wenn $Ar^1$ für eine Heteroarylgruppe, insbesondere für Triazin, Pyrimidin, Chinazolin oder Carbazol, steht, können auch aromatische oder heteroaromatische Substituenten R an dieser Heteroarylgruppe bevorzugt sein.

Geeignete aromatische bzw. heteroaromatische Ringsysteme $Ar^1$ sind bei jedem Auftreten gleich oder verschieden ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Phenanthren, Triphenylen oder einer Kombination aus zwei oder drei dieser Gruppen, welche jeweils mit einem oder mehreren Resten R, bevorzugt nicht-aromatischen Resten R, substituiert sein können. Wenn $Ar^1$ für eine Heteroarylgruppe, insbesondere für Triazin, Pyrimidin, Chinazolin oder Carbazol steht, können auch aromatische oder heteroaromatische Reste R an dieser Heteroarylgruppe bevorzugt sein.

Dabei ist $Ar^1$ bevorzugt bei jedem Auftreten gleich oder verschieden gewählt aus den Gruppen der folgenden Formeln Ar-1 bis Ar-81,

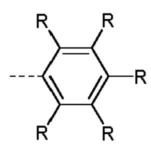

Ar-1

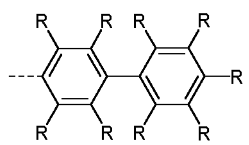

Ar-2

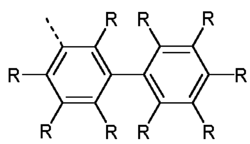

Ar-3

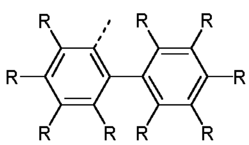

Ar-4

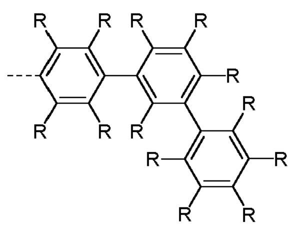

Ar-5

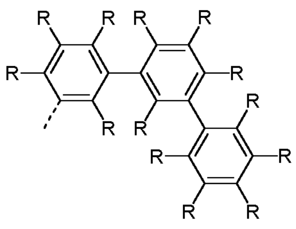

Ar-6

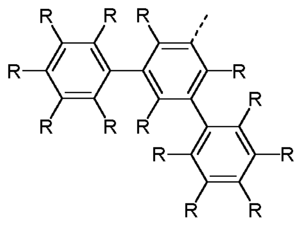

Ar-7

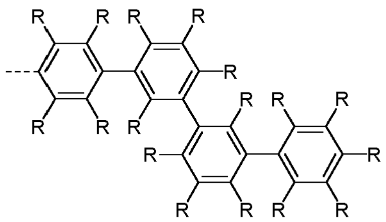

Ar-8

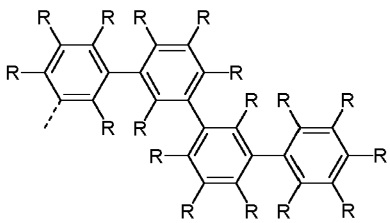

Ar-9

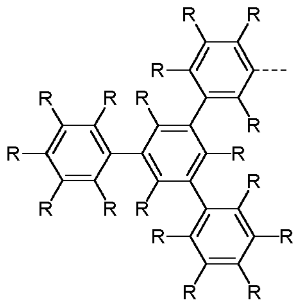

Ar-10

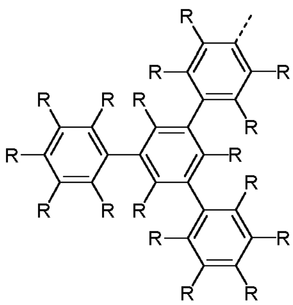

Ar-11

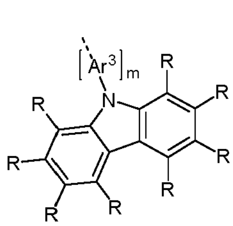

Ar-12

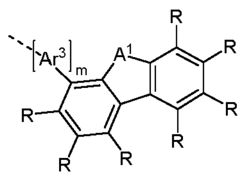

Ar-13

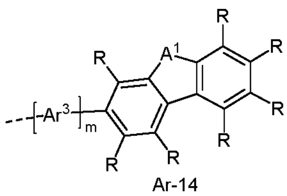

Ar-14

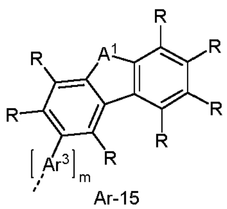

Ar-15

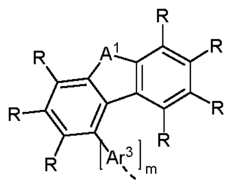

Ar-16

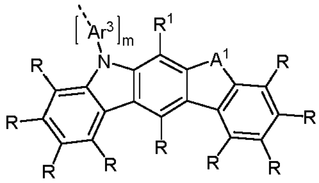

Ar-17

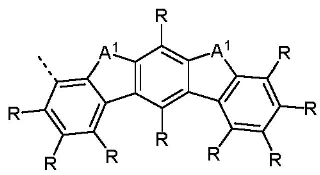

Ar-18

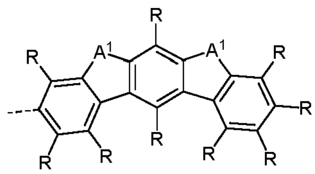

Ar-19

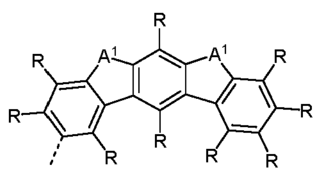

Ar-20

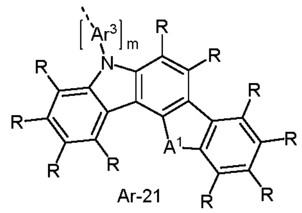

Ar-21

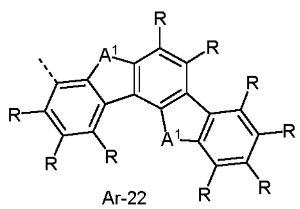

Ar-22

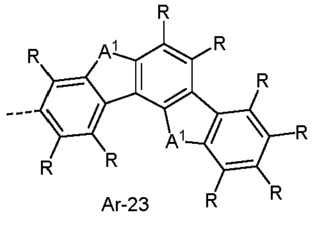

Ar-23

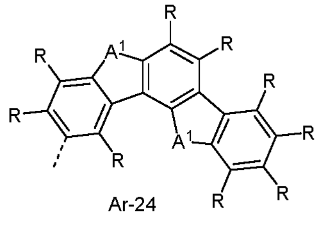

Ar-24

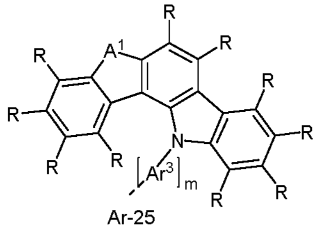

Ar-25

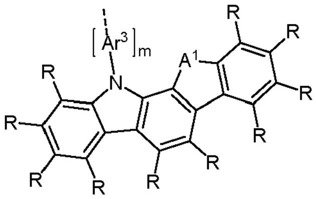
Ar-26

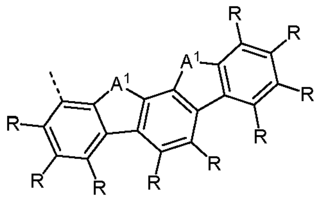
Ar-27

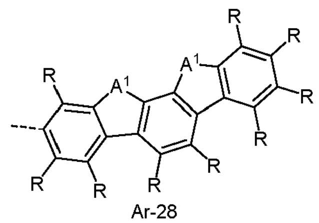
Ar-28

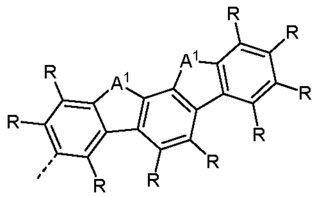
Ar-29

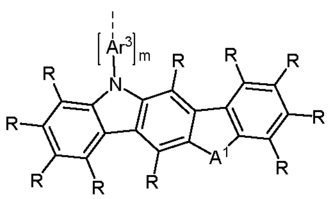
Ar-30

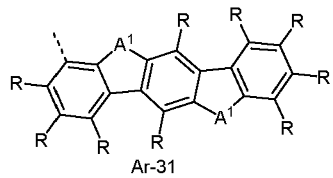
Ar-31

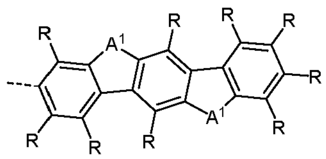
Ar-32

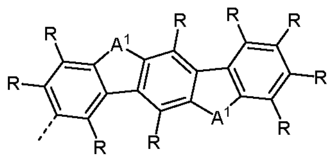
Ar-33

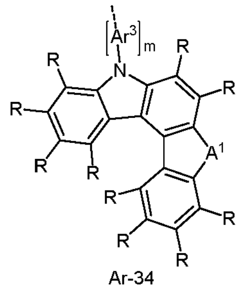
Ar-34

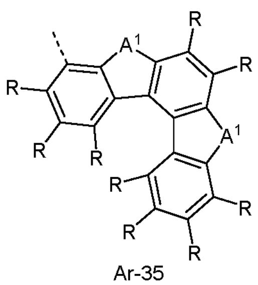
Ar-35

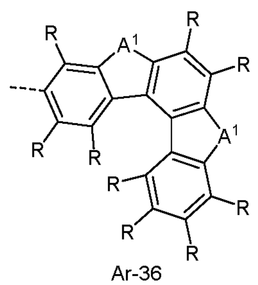
Ar-36

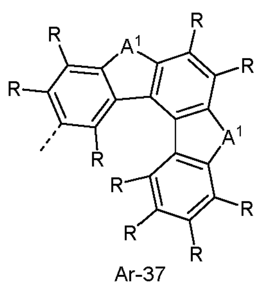
Ar-37

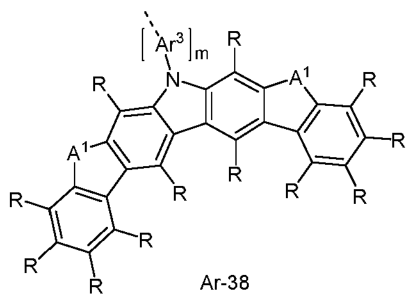
Ar-38

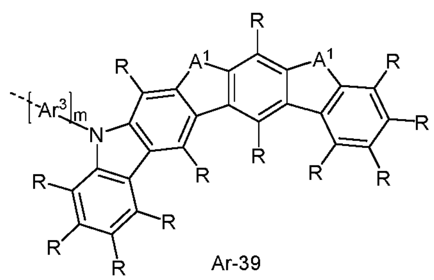
Ar-39

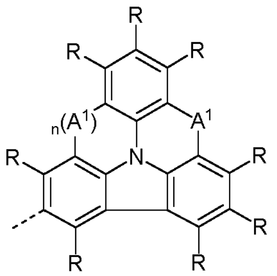
Ar-40

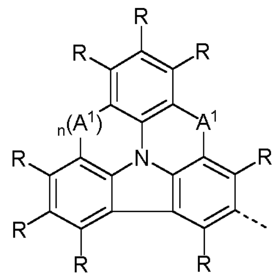
Ar-41

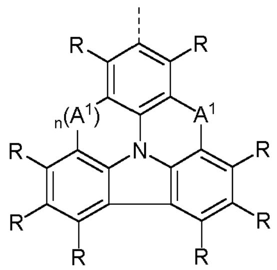
Ar-42

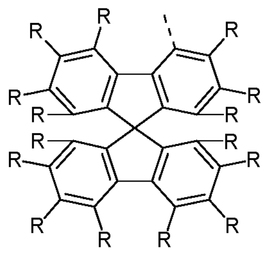
Ar-43

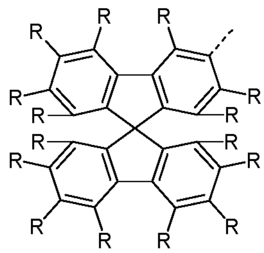
Ar-44

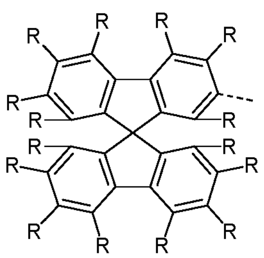
Ar-45

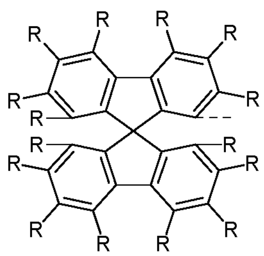
Ar-46

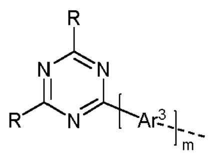
Ar-47

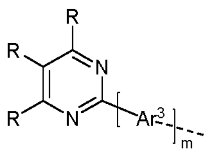
Ar-48

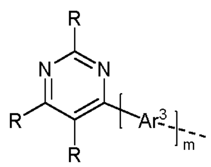
Ar-49

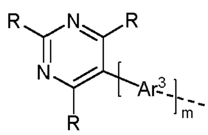
Ar-50

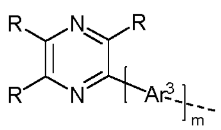
Ar-51

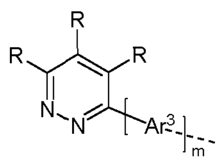
Ar-52

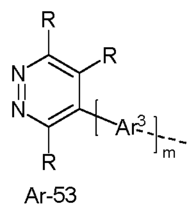
Ar-53

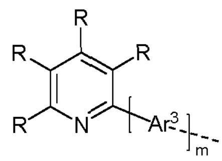
Ar-54

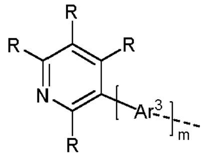
Ar-55

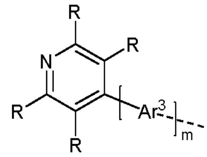
Ar-56

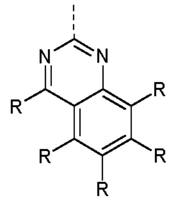
Ar-57

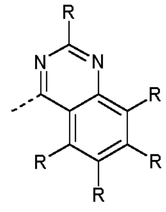
Ar-58

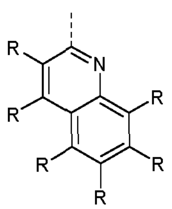
Ar-59

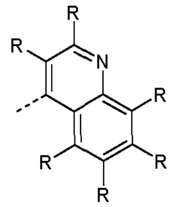
Ar-60

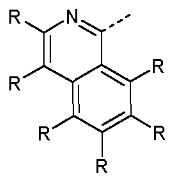
Ar-61

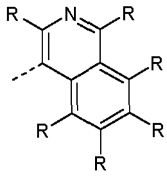
Ar-62

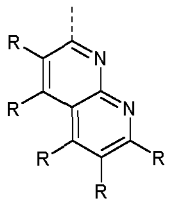
Ar-63

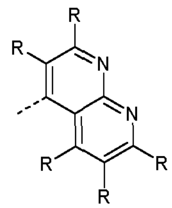
Ar-64

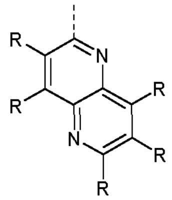
Ar-65

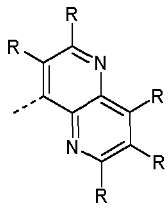
Ar-66

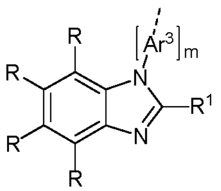
Ar-67

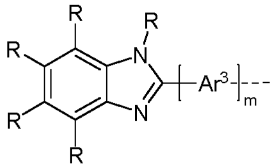
Ar-68

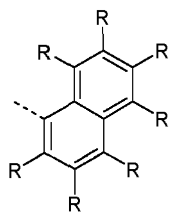
Ar-69

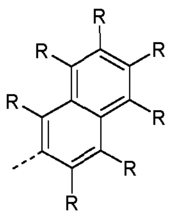
Ar-70

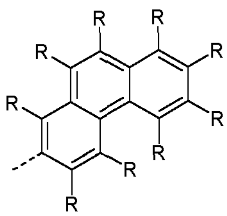
Ar-71

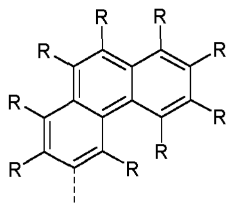
Ar-72

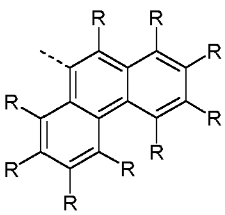
Ar-73

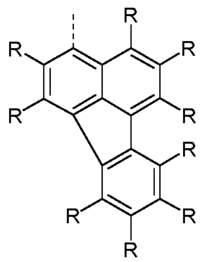
Ar-74

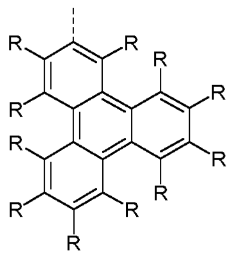
Ar-75

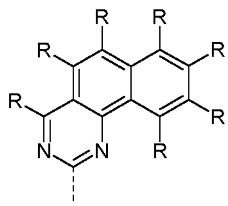
Ar-76

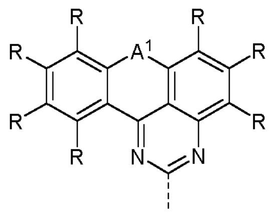

Ar-77

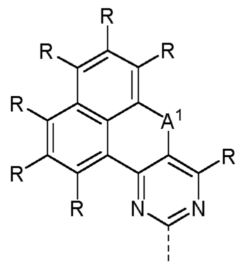

Ar-78

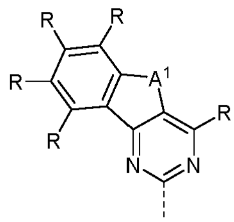

Ar-79

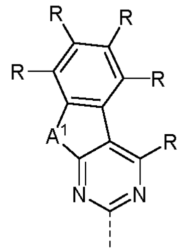

Ar-80

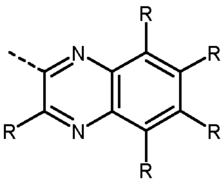

Ar-81

wobei R die oben genannten Bedeutungen aufweist, die gestrichelte Bindung die Bindung an die Heteroarylgruppe darstellt und weiterhin gilt:

$A^1$ ist bei jedem Auftreten gleich oder verschieden $CR_2$, NR, O oder S;

$Ar^3$ ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;

n ist 0 oder 1, wobei n = 0 bedeutet, dass an dieser Position keine Gruppe $A^1$ gebunden ist und an die entsprechenden Kohlenstoffatome statt dessen Reste R gebunden sind;

m ist 0 oder 1, wobei m = 0 bedeutet, dass die Gruppe $Ar^3$ nicht vorhanden ist und dass die entsprechende aromatische bzw. heteroaromatische Gruppe direkt an die Triazin- bzw. Pyrimidingruppe in Formel (1) gebunden ist.

Besonders bevorzugte Gruppen $Ar^1$ sind gewählt aus der Gruppe bestehend aus 1-Dibenzofuranyl, also eine Gruppe der Formel Ar-16 mit $A^1$ = O und m = 0, Phenyl-1-dibenzofuranyl, also eine Gruppe der Formel Ar-16 mit $A^1$ = O, m = 1 und $Ar^3$ = para-Phenylen, Phenyl, ortho-Biphenyl, meta-Biphenyl, para-Biphenyl, Quaterphenyl, para-tert-Butylphenyl, Naphthyl, Fluorenyl, insbesondere 9,9-Dimethyl-2-fluorenyl, und 1-Dibenzothienyl.

In einer weiteren bevorzugten Ausführungsform der Erfindung ist $Ar^2$, also der Substituent am Carbazol für $Y^2$ = $NAr^2$, gleich oder verschieden bei jedem Auftreten ein aromatisches oder heteroaromatisches Ringsystem mit 6 bis 30 aromatischen Ringatomen, das durch einen oder mehrere Reste R substituiert sein kann. Besonders bevorzugt ist $Ar^2$ gleich oder verschieden bei jedem Auftreten ein aromatisches oder heteroaromatisches Ringsystem, insbesondere ein aromatisches Ringsystem, mit 6 bis 24 aromatischen Ringatomen, insbesondere mit 6 bis 13 aromatischen Ringatomen, das durch einen oder mehrere, bevorzugt nicht-aromatische, Reste R substituiert sein kann. Wenn $Ar^2$ für eine Heteroarylgruppe, insbesondere für Triazin, Pyrimidin, Chinazolin oder Carbazol, steht, können auch aromatische oder heteroaromatische Substituenten R an dieser Heteroarylgruppe bevorzugt sein.

Geeignete aromatische bzw. heteroaromatische Ringsysteme $Ar^2$ sind bei jedem Auftreten gleich oder verschieden ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Phenanthren, Triphenylen oder einer Kombination aus zwei oder drei dieser Gruppen, welche jeweils mit einem oder mehreren Resten R, bevorzugt nicht-aromatischen Resten R, substituiert sein können. Wenn $Ar^2$ für eine Heteroarylgruppe, insbesondere für Triazin, Pyrimidin, Chinazolin oder Carbazol steht, können auch aromatische oder heteroaromatische Reste R an dieser Heteroarylgruppe bevorzugt sein.

Dabei ist $Ar^2$ bevorzugt bei jedem Auftreten gleich oder verschieden gewählt aus den oben aufgeführten Gruppen der Formeln Ar-1 bis Ar-81.

Besonders bevorzugte Gruppen $Ar^2$ sind gewählt aus der Gruppe bestehend aus 1-Dibenzofuranyl, also eine Gruppe der Formel Ar-16 mit $A^1$ = O und m = 0, Phenyl-1-dibenzofuranyl, also eine Gruppe der Formel Ar-16 mit $A^1$ = O, m = 1 und $Ar^3$ = para-Phenylen, Phenyl, ortho-Biphenyl, meta-Biphenyl, para-Biphenyl, ortho-para-Terphenyl, Quaterphenyl, para-tert-Butylphenyl, Triphenylen und N-Phenyl-3-carbazolyl.

In einer bevorzugten Ausführungsform der Erfindung ist R bei jedem Auftreten gleich oder verschieden ausgewählt aus der Gruppe bestehend aus H, D, F, $N(Ar')_2$, CN, $OR^1$, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten $R^1$ substituiert sein kann, bevorzugt jedoch unsubstituiert ist, und wobei eine oder mehrere nicht benachbarte $CH_2$-Gruppen durch O ersetzt sein können, oder einem aromatischen oder heteroaromatischen Ringsystem mit 6 bis 30 aromatischen Ringatomen, das jeweils durch einen oder mehrere Reste $R^1$ substituiert sein kann; dabei können zwei Reste R auch miteinander ein aliphatisches, aromatisches oder heteroaromatisches Ringsystem bilden. Besonders bevorzugt ist R bei jedem Auftreten gleich oder verschieden ausgewählt aus der Gruppe bestehend aus H, $N(Ar')_2$, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten $R^1$ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste $R^1$, bevorzugt nicht-aromatische Reste $R^1$, substituiert sein kann. Ganz besonders bevorzugt ist R bei jedem Auftreten gleich oder verschieden ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils durch einen oder mehrere Reste $R^1$, bevorzugt nicht-aromatische Reste $R^1$, substituiert sein kann.

In einer weiteren bevorzugten Ausführungsform der Erfindung ist Ar' ein aromatisches oder heteroaromatisches Ringsystem mit 6 bis 30 aromatischen Ringatomen, das durch einen oder mehrere Reste $R^1$ substituiert sein kann. In einer besonders bevorzugten Ausführungsform der Erfindung ist Ar' ein aromatisches oder heteroaromatisches Ringsystem mit 6 bis 24 aromatischen Ringatomen, ganz besonders bevorzugt mit 6 bis 18 aromatischen Ringatomen, insbesondere mit 6 bis 13 aromatischen Ringatomen, das durch einen oder mehrere, bevorzugt nicht-aromatische, Reste $R^1$ substituiert sein kann.

Geeignete aromatische bzw. heteroaromatische Ringsysteme R bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren nicht-aromatischen Resten $R^1$ substituiert sein können. Wenn R bzw. Ar' für eine Heteroarylgruppe, insbesondere für Triazin, Pyrimidin oder Chinazolin steht, können auch aromatische oder heteroaromatische Reste $R^1$ an dieser Heteroarylgruppe bevorzugt sein.

Dabei sind die Gruppen R, wenn sie für ein aromatisches bzw. heteroaromatisches Ringsystem stehen, bzw. Ar' bevorzugt gewählt aus den Gruppen der folgenden Formeln R-1 bis R-81,

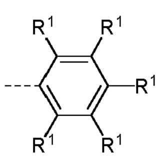
R-1

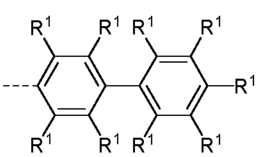
R-2

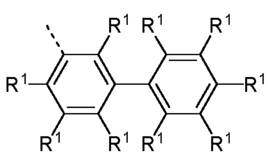
R-3

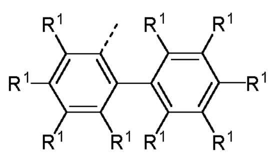
R-4

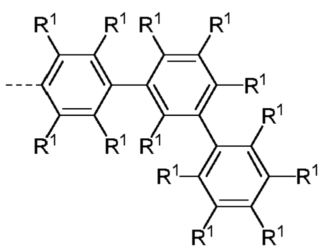
R-5

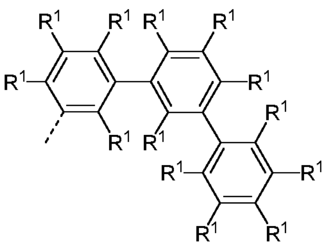
R-6

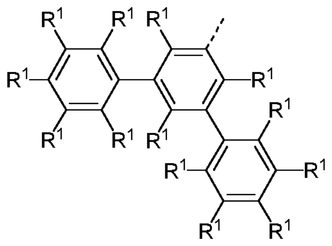
R-7

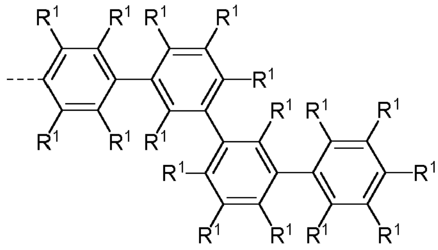
R-8

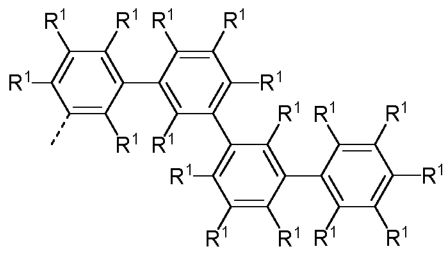
R-9

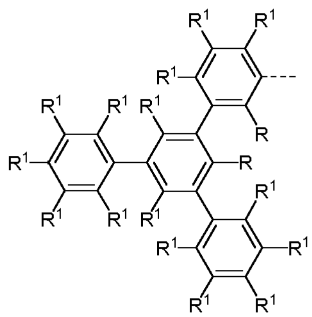
R-10

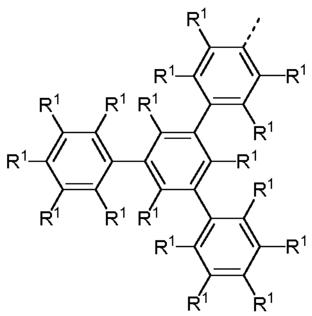
R-11

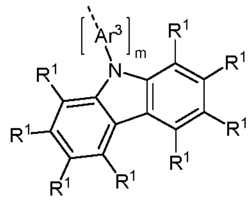
R-12

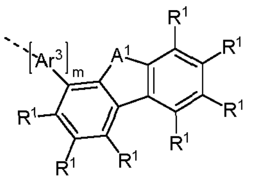
R-13

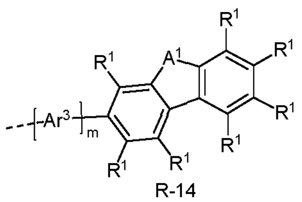
R-14

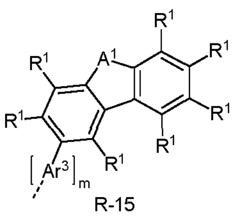
R-15

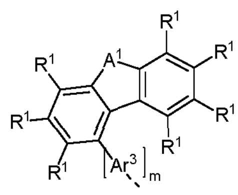
R-16

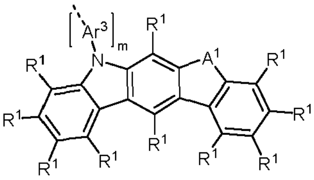
R-17

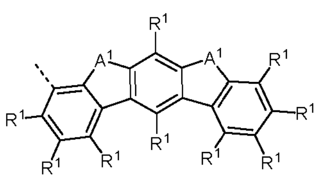
R-18

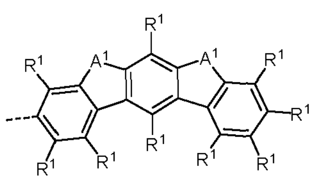
R-19

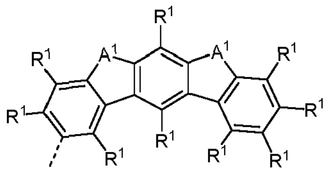
R-20

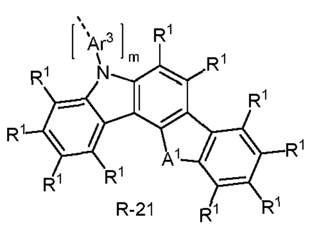
R-21

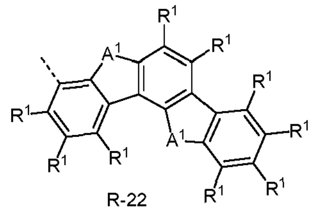
R-22

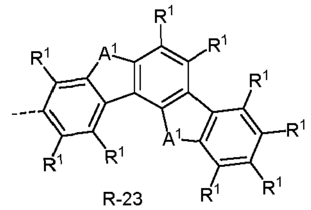
R-23

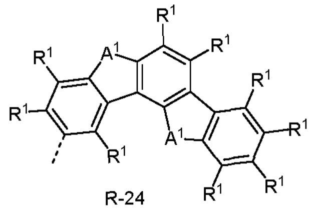
R-24

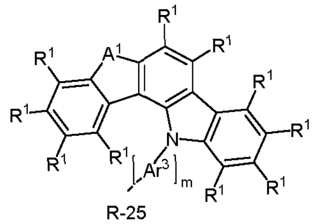
R-25

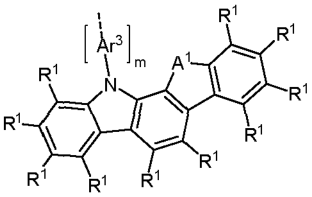
R-26

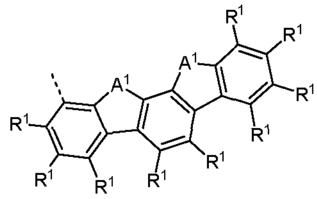
R-27

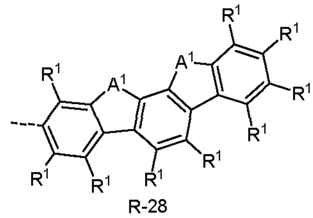
R-28

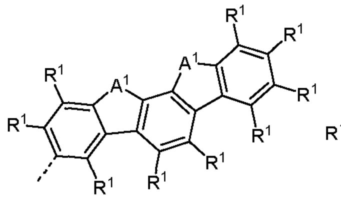
R-29

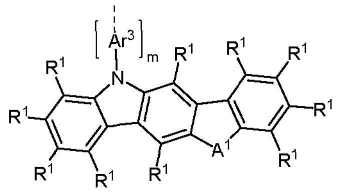
R-30

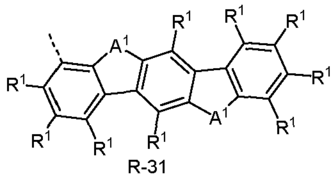
R-31

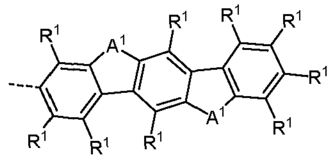
R-32

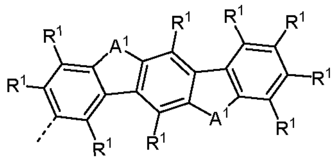
R-33

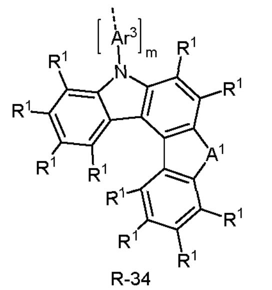
R-34

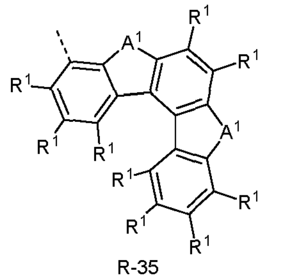
R-35

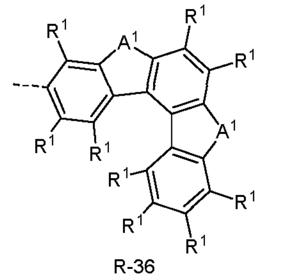
R-36

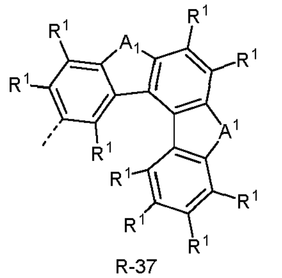
R-37

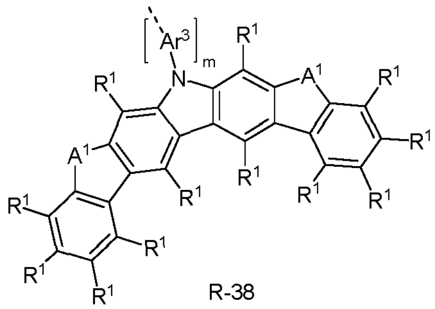
R-38

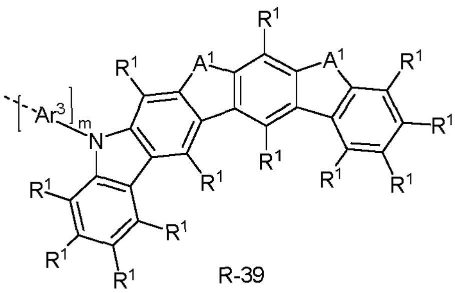
R-39

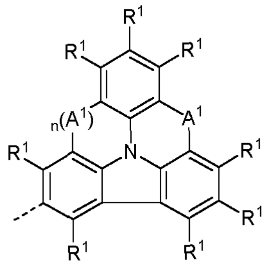
R-40

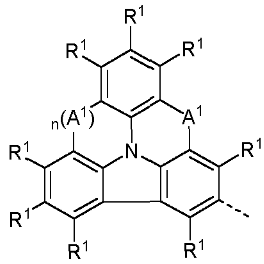
R-41

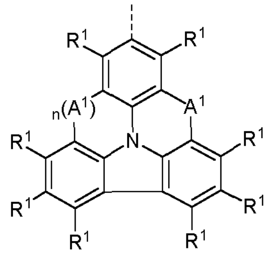
R-42

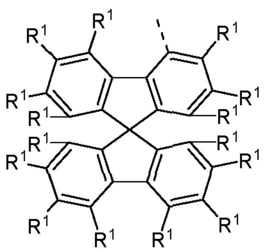
R-43

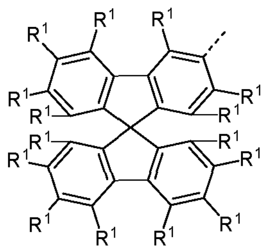
R-44

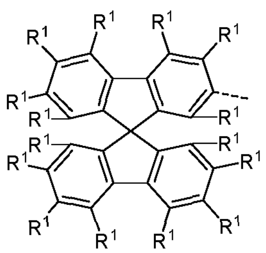
R-45

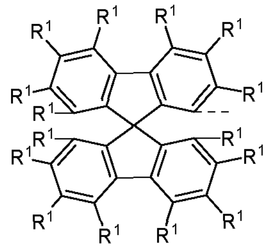
R-46

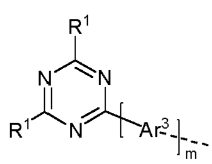
R-47

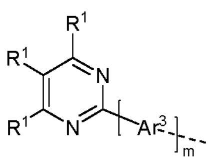
R-48

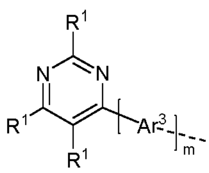
R-49

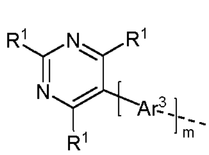
R-50

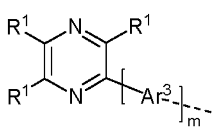
R-51

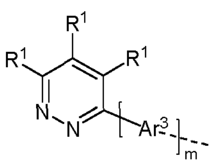
R-52

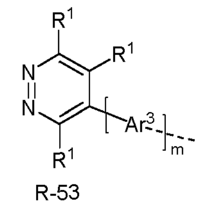
R-53

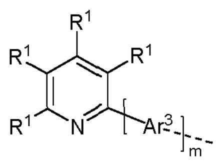
R-54

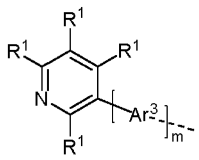

R-55

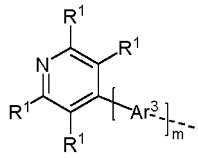

R-56

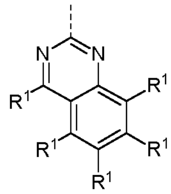

R-57

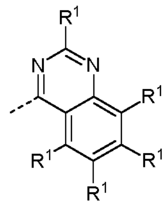

R-58

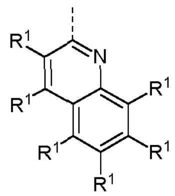

R-59

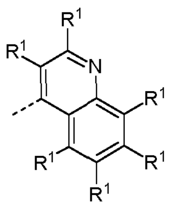

R-60

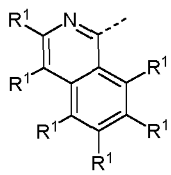

R-61

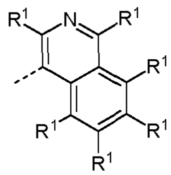

R-62

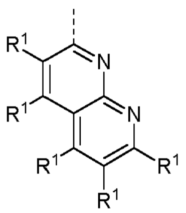

R-63

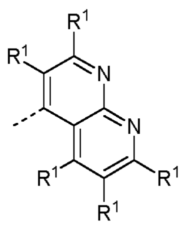

R-64

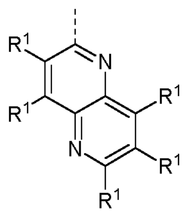

R-65

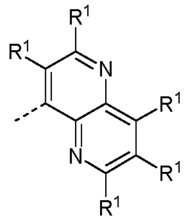

R-66

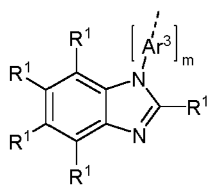

R-67

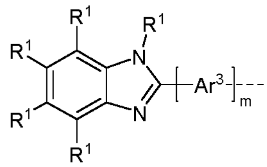

R-68

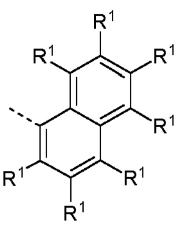

R-69

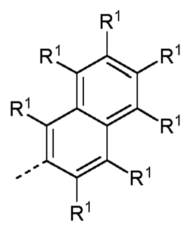

R-70

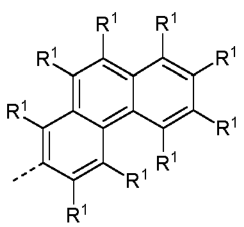

R-71

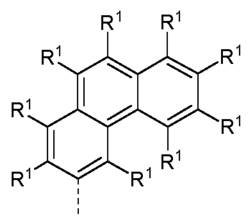

R-72

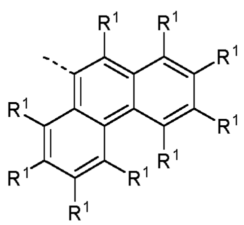

R-73

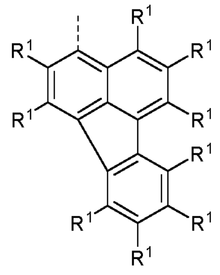

R-74

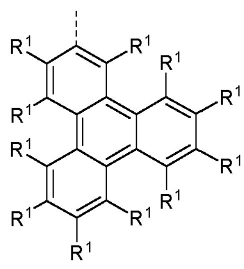

R-75

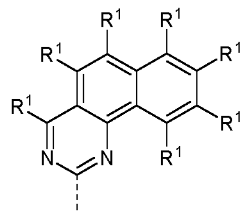

R-76

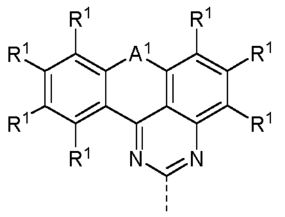
R-77

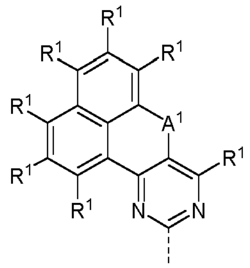
R-78

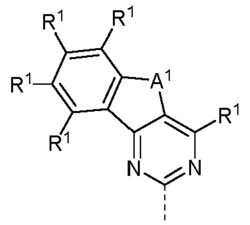
R-79

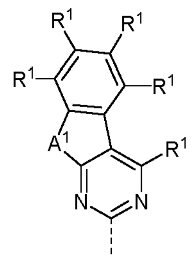
R-80

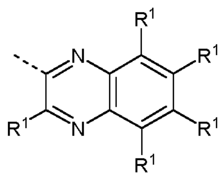
R-81

wobei $R^1$ die oben genannten Bedeutungen aufweist, die gestrichelte Bindung die Bindung an das Grundgerüst in Formel (1) bzw. die Bindung an $Ar^1$ bzw. $Ar^2$ bzw. an das Stickstoffatom in der Gruppe $N(Ar')_2$ darstellt und weiterhin gilt:

$A^1$ ist bei jedem Auftreten gleich oder verschieden $C(R^1)_2$, $NR^1$, O oder S;

$Ar^3$ ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten $R^1$ substituiert sein kann;

n ist 0 oder 1, wobei n = 0 bedeutet, dass an dieser Position keine Gruppe $A^1$ gebunden ist und an die entsprechenden Kohlenstoffatome statt dessen Reste $R^1$ gebunden sind;

m ist 0 oder 1, wobei m = 0 bedeutet, dass die Gruppe $Ar^3$ nicht vorhanden ist und dass die entsprechende aromatische bzw. heteroaromatische Gruppe direkt an das Grundgerüst in Formel (1) bzw. in den bevorzugten Ausführungsformen bzw. an das Stickstoffatom in der Gruppe $N(Ar')_2$ gebunden ist; mit der Maßgabe, dass m = 1 ist für die Strukturen (R-12), (R-17), (R-21), (R-25), (R-26), (R-30), (R-34), (R-38) und (R-39), wenn es sich bei diesen Gruppen um Ausführungsformen von Ar' handelt.

Wenn die oben genannten Gruppen Ar-1 bis Ar-81 für $Ar^1$ bzw. $Ar^2$ und R-1 bis R-81 für R bzw. Ar' mehrere Gruppen $A^1$ aufweisen, so kommen hierfür alle Kombinationen aus der Definition von A' in Frage. Bevorzugte Ausführungsformen sind dann solche, in denen eine Gruppe $A^1$ für NR bzw. $NR^1$ und die andere Gruppe $A^1$ für $C(R)_2$ bzw. $C(R^1)_2$ steht oder in denen beide Gruppen $A^1$ für NR bzw. $NR^1$ stehen oder in denen beide Gruppen $A^1$ für O stehen. In einer besonders bevorzugten Ausführungsform der Erfindung steht in Gruppen $Ar^1$, $Ar^2$, R bzw. Ar', die mehrere Gruppen $A^1$ aufweisen, mindestens eine Gruppe $A^1$ für $C(R)_2$ bzw. $C(R^1)_2$ oder für NR bzw. $NR^1$.

Wenn $A^1$ für NR bzw. $NR^1$ steht, steht der Substituent R bzw. $R^1$, der an das Stickstoffatom gebunden ist, bevorzugt für ein aromatisches oder heteroaromatisches Ringsystem mit 5 bis 24 aromatischen Ringatomen, welches auch durch einen oder mehrere Reste $R^1$ bzw. $R^2$ substituiert sein kann. In einer besonders bevorzugten Ausführungsform steht dieser Substituent R bzw. $R^1$ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-RingGruppen direkt aneinander ankondensiert sind, aufweist, und welches jeweils auch durch einen oder mehrere Reste $R^1$ bzw. $R^2$ substituiert sein kann. Besonders bevorzugt sind Phenyl, Biphenyl, Terphenyl und Quaterphenyl mit Verknüpfungsmustern, wie vorne für Ar-1 bis Ar-11 bzw. R-1 bis R-11 aufgeführt, wobei diese Strukturen durch einen oder mehrere Reste $R^1$ bzw. $R^2$ substituiert sein können, bevorzugt aber unsubstituiert sind.

Wenn $A^1$ für $C(R)_2$ bzw. $C(R^1)_2$ steht, stehen die Substituenten R bzw. $R^1$, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste $R^1$ bzw. $R^2$ substituiert sein kann. Ganz besonders bevorzugt steht R bzw. $R^1$ für eine Methylgruppe oder für eine

Phenylgruppe. Dabei können die Reste R bzw. $R^1$ auch miteinander ein Ringsystem bilden, was zu einem Spirosystem führt.

In einer weiteren bevorzugten Ausführungsform der Erfindung ist $R^1$ gleich oder verschieden bei jedem Auftreten ausgewählt aus der Gruppe bestehend aus H, D, F, CN, $OR^2$, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten $R^2$ substituiert sein kann und wobei eine oder mehrere nicht benachbarte $CH_2$-Gruppen durch O ersetzt sein können, oder einem aromatischen oder heteroaromatischen Ringsystem mit 6 bis 30 aromatischen Ringatomen, das jeweils durch einen oder mehrere Reste $R^2$ substituiert sein kann; dabei können zwei oder mehrere Reste $R^1$ miteinander ein aliphatisches Ringsystem bilden. In einer besonders bevorzugten Ausführungsform der Erfindung ist $R^1$ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils durch einen oder mehrere Reste $R^2$ substituiert sein kann, bevorzugt aber unsubstituiert ist.

In einer weiteren bevorzugten Ausführungsform der Erfindung ist $R^2$ gleich oder verschieden bei jedem Auftreten H, F, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Die oben genannten Bevorzugungen können einzeln oder gemeinsam auftreten. Es ist bevorzugt, wenn die oben genannten Bevorzugungen gemeinsam auftreten.

Beispiele für geeignete erfindungsgemäße Verbindungen sind die nachstehend abgebildeten Strukturen.

| | | |
|---|---|---|
| 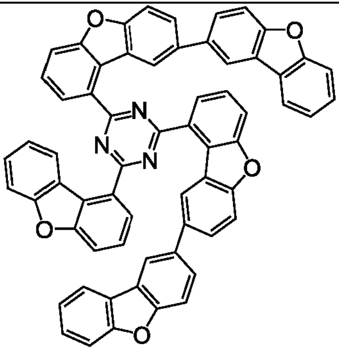 | 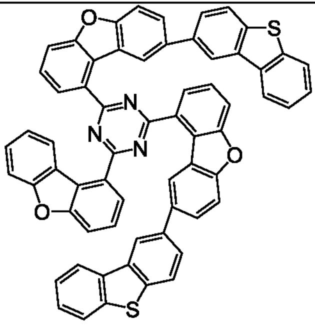 | 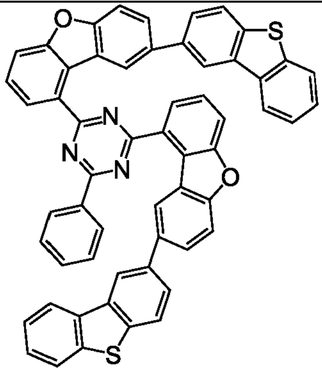 |
| 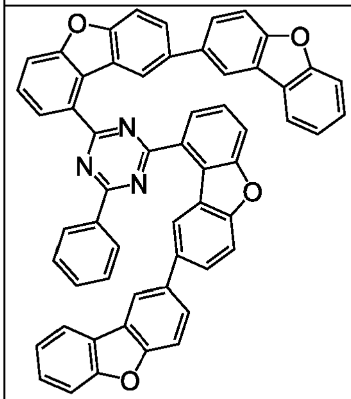 | 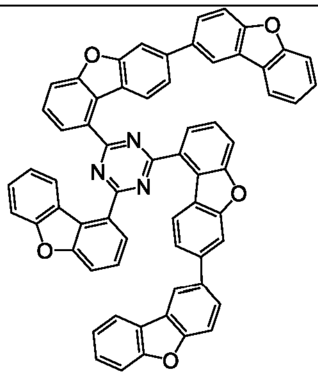 | 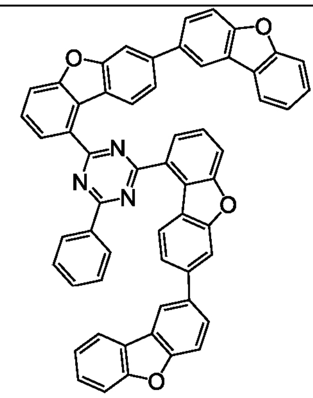 |
| 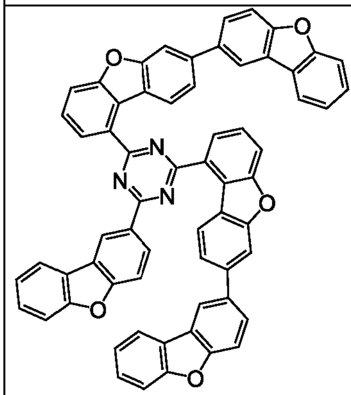 | 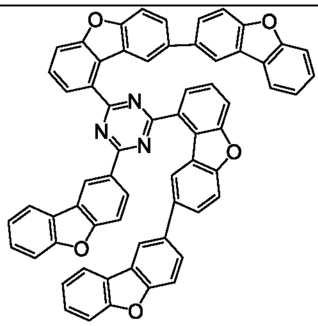 | 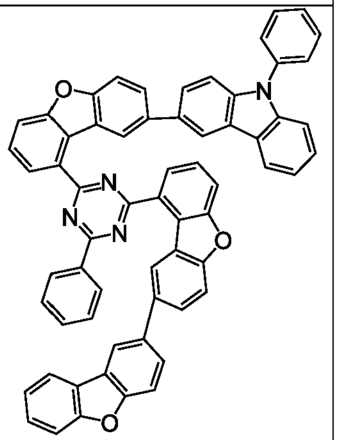 |
| 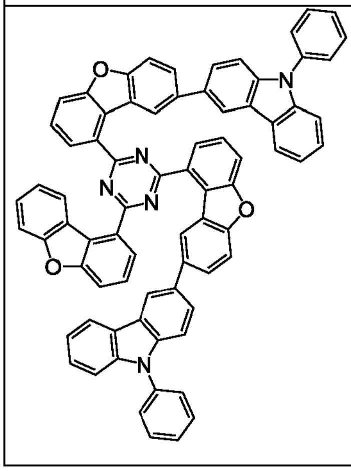 | 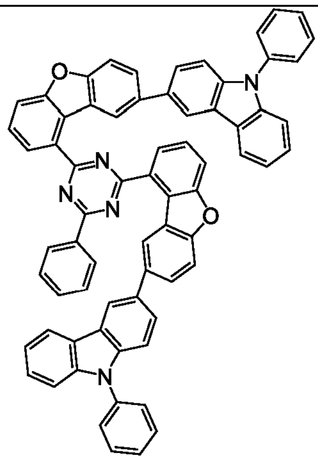 | |

| | | |
|---|---|---|
| 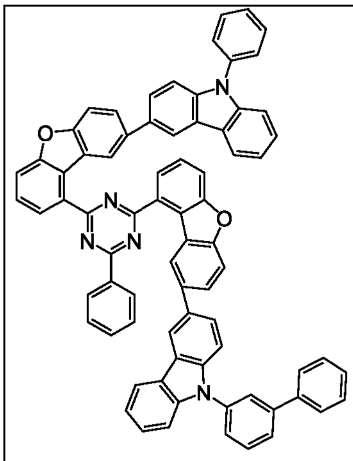 | 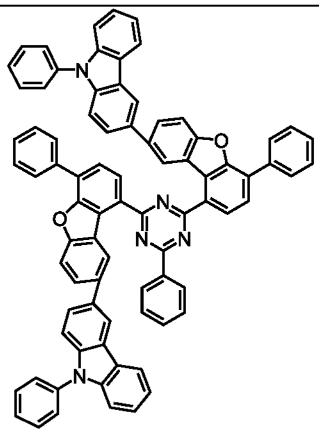 | 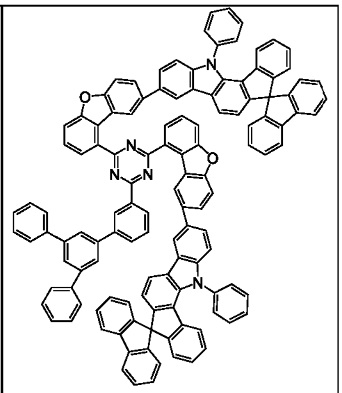 |
| 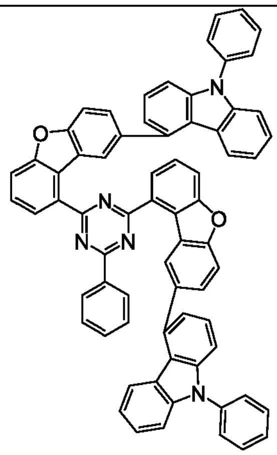 | 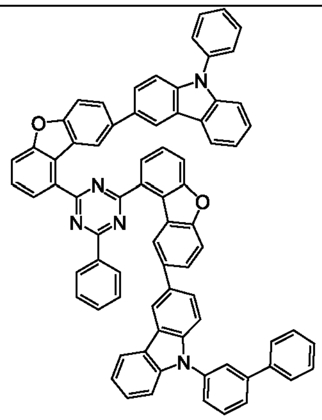 | 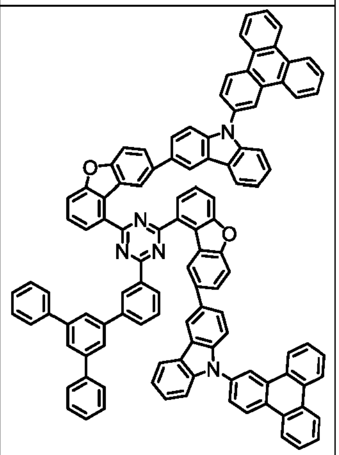 |
| 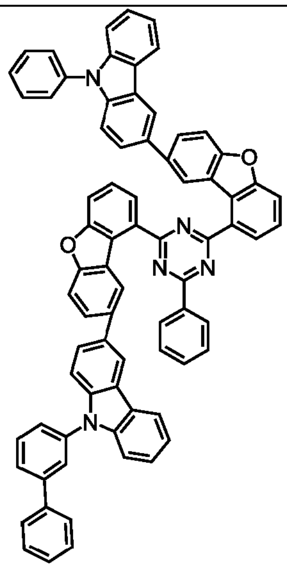 | 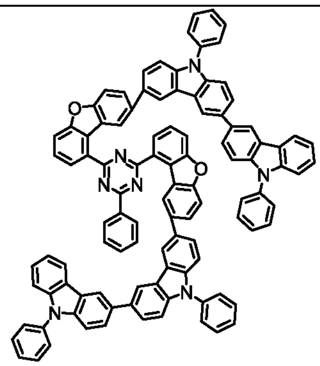 | 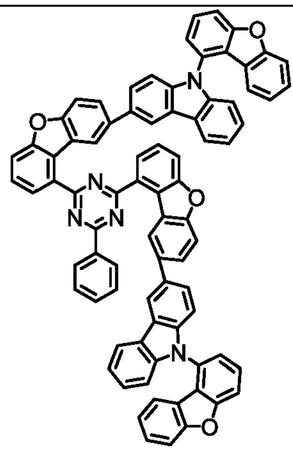 |

| | | |
|---|---|---|
| 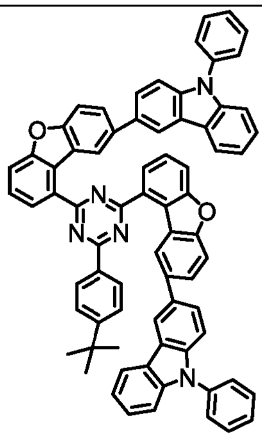 | 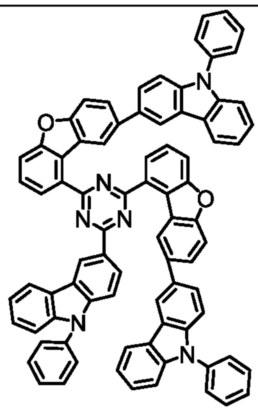 | 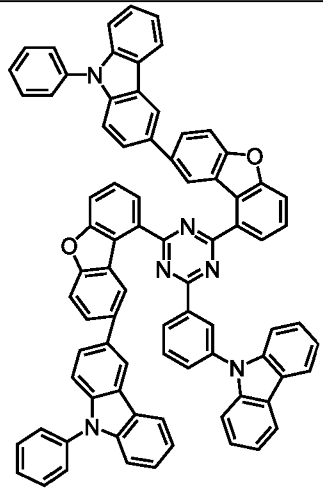 |
| 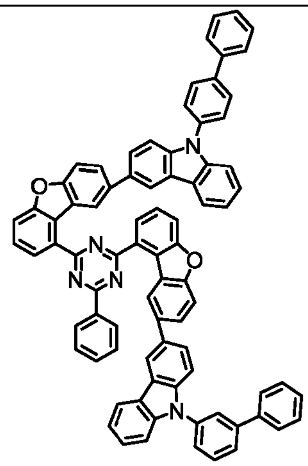 | 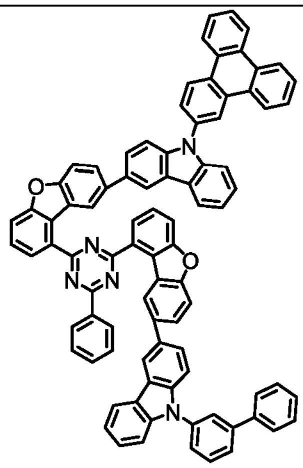 | 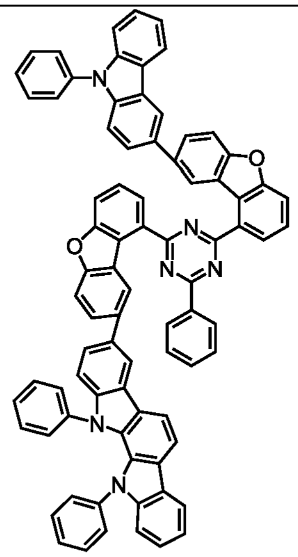 |
| 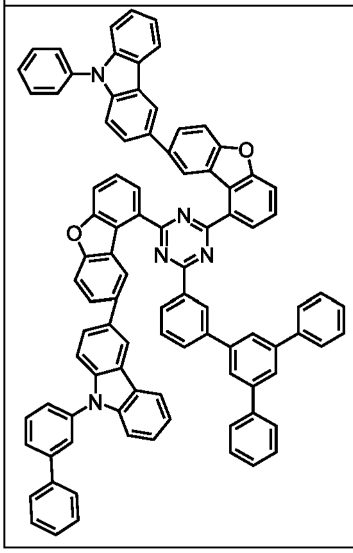 | 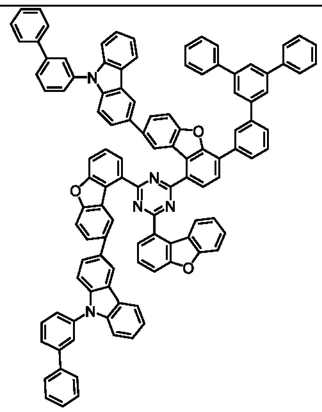 | 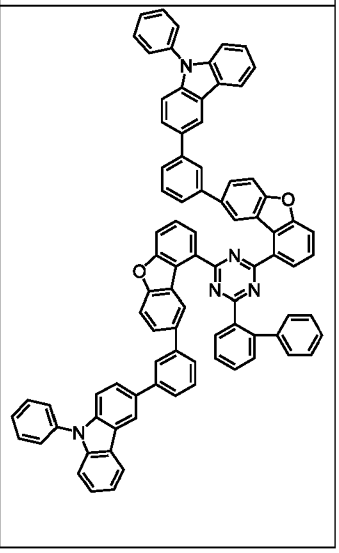 |

| | | |
|---|---|---|
| 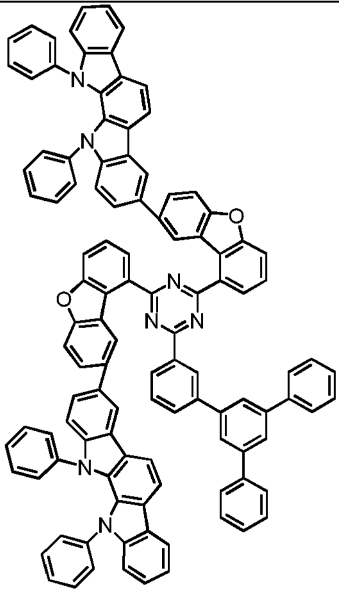 | | 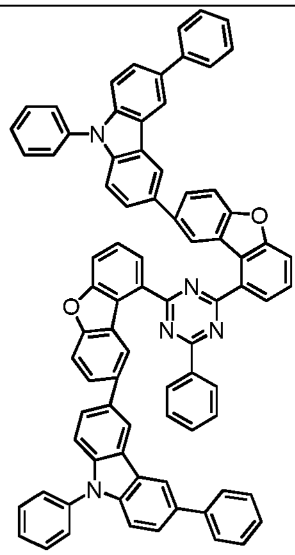 |
| 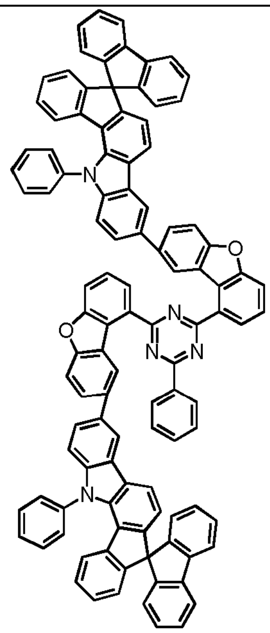 | 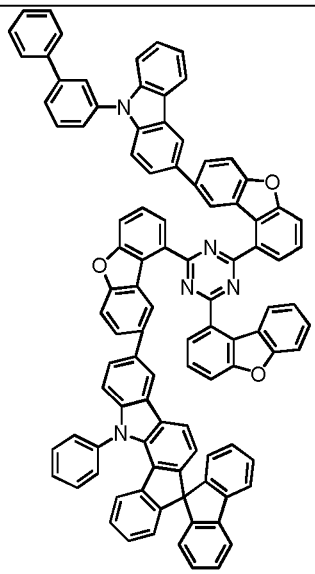 | 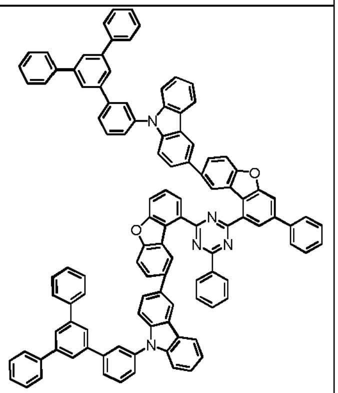 |
| 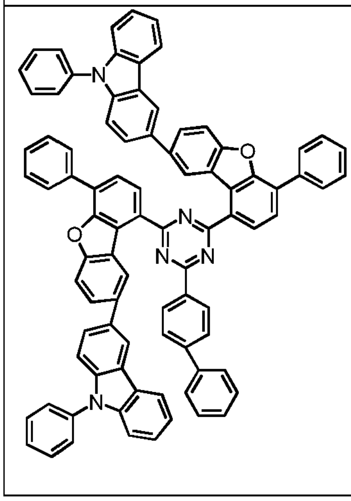 | 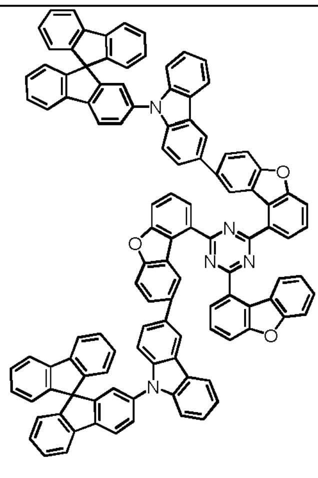 | 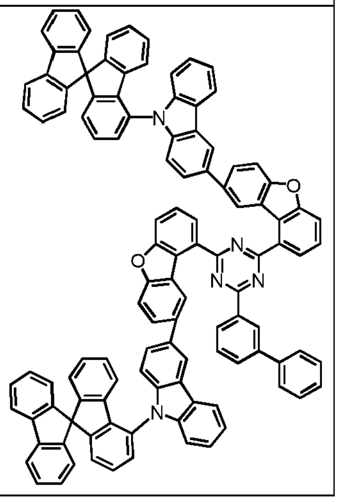 |

| | | |
|---|---|---|
| 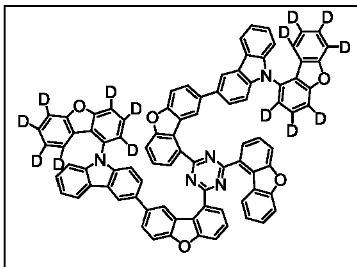 | 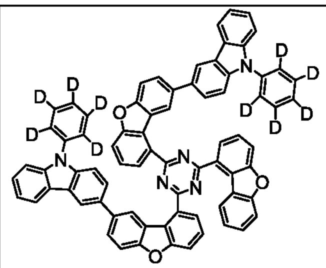 | 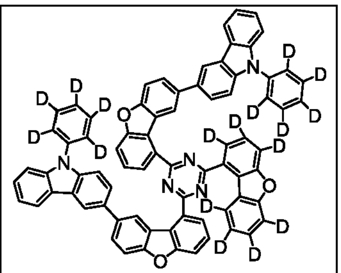 |
| 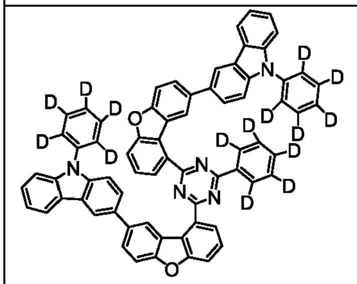 | 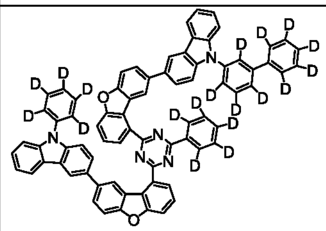 | 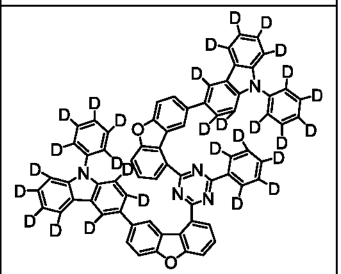 |
| 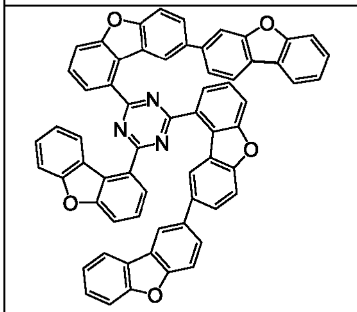 | 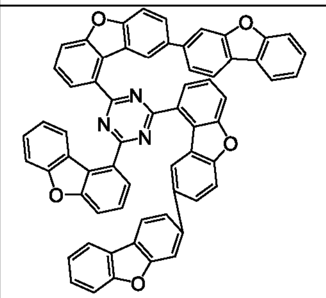 | 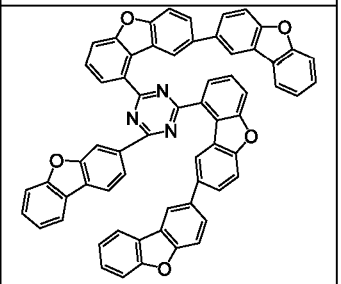 |
| 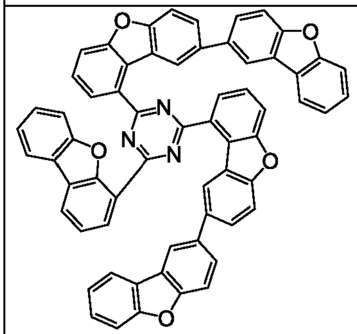 | 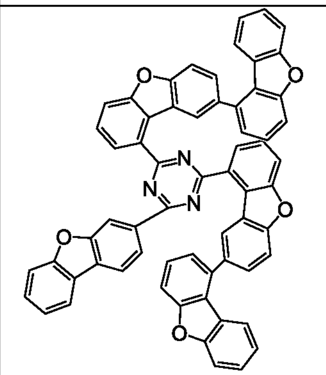 | 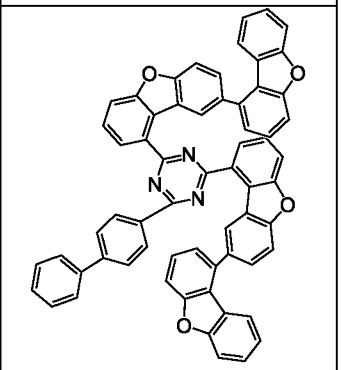 |
| 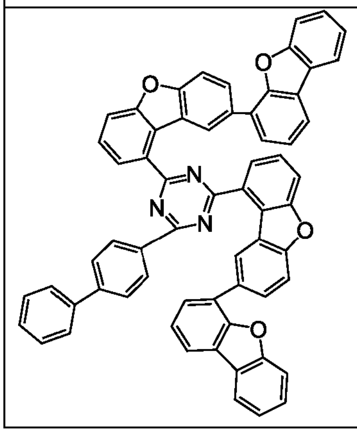 | 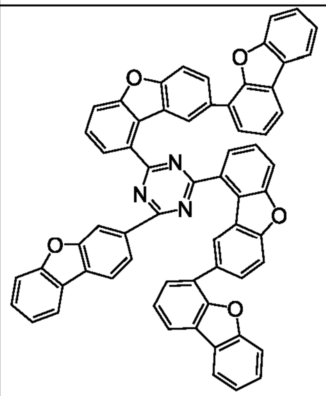 | 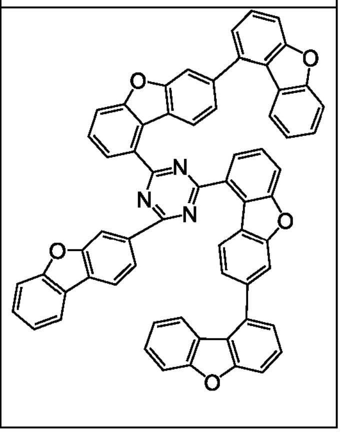 |

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt, wobei die verwendeten Symbole und Indizes die oben genannten Bedeutungen aufweisen. Dabei können die erfindungsgemäßen Verbindungen ausgehend von einem mit $Ar^1$ funktionalisierten Dichlorpyrimidin oder -triazin durch Umsetzung mit einer 1-Dibenzofuran-boronsäure bzw. deren Ester in einer Suzuki-Kupplung dargestellt werden. Die Reaktion kann wie in Schema 1 gezeigt in zwei Schritten durchgeführt werden oder bei Verwendung identisch substiutierter 1-Dibenzofuran-boronsäuren bzw. -boronsäureester auch in einem Schritt. Die Suzuki-Kupplung kann in aprotischen Lösungsmitteln (z. B. Toluol, Dioxan, THF, DMF, DMSO) oder unter Zusatz von protischen Lösungsmitteln (z. B. Alkohole, Wasser) durchgeführt werden. Als Katalysator werden üblicherweise Pd-Katalysatoren oder Pd-Verbindungen (Palladiumacetat, Pd-dba, etc.) und Phosphine (Triphenylphosphin, Tri-o-tolylphosphin, S-Phos, X-Phos, BINAP, DPPF, Xanth-Phos, etc.) verwendet. Als Basen für die Kupplungsreaktion kommen anorganische Salze (Natrium-, Kalium- oder Caesiumcarbonat, -phosphat, -fluorid, -borat) und deren Hydrate zum Einsatz.

Schema 1

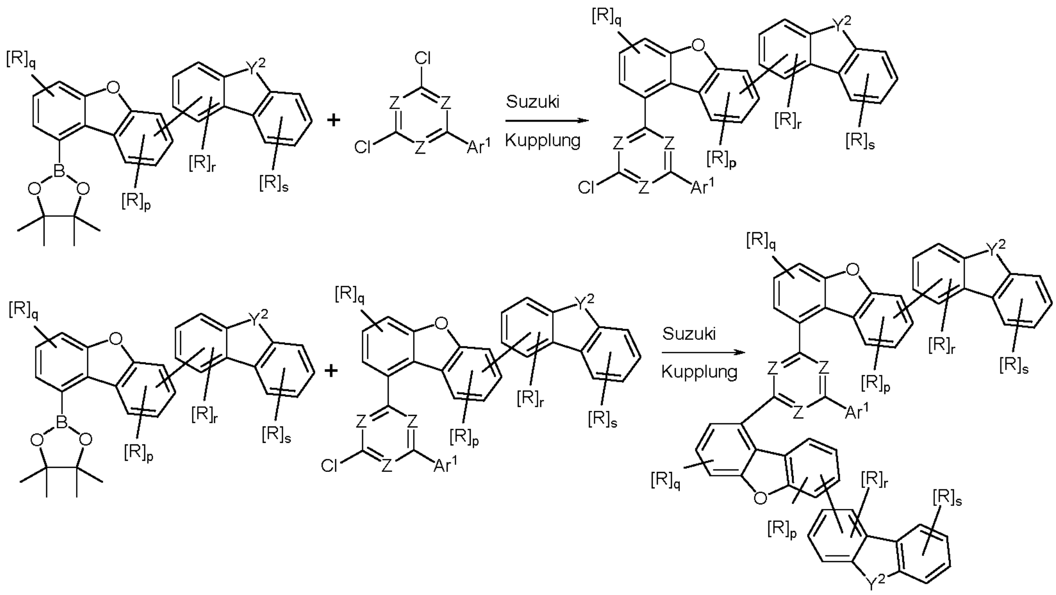


Auch wenn die erfindungsgemäßen Verbindungen auch durch Aufdampfen im Vakuum verarbeitet werden können, ist die vorrangige Aufgabe der vorliegenden Erfindung die Bereitsstellung von Verbindungen für die Verarbeitung aus flüssiger Phase, beispielsweise durch Spin-Coating oder durch Druckverfahren. Hierfür sind Formulierungen der erfindungsgemäßen Verbindungen erforderlich. Diese Formulierungen können beispielsweise Lösungen, Dispersionen oder Emulsionen sein, bevorzugt Lösungen. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat, Alkoxy-substitutierte Ethylbenzoate, insbesondere 2-Methoxy-ethylbenzoat und 4-Methoxy-ethylbenzoat, Alkyl-substituierte Ethylbenzoate, inbesondere 2-Methyl-ethylbenzoat und 4-Methylethylbenzoat, Propylencarbonat, Ethylencarbonat, Isopropylbiphenyle, insbesondere 2-Isopropylbiphenyl, 3-Isopropylbiphenyl und 2,2'-Diisopropylbiphenyl, Amylbenzoat, Isoamylbenzoat, 1-Isopropylnaphthalin, 2-Isopropylnaphthalin, 1-Isopropylnaphthalin, Diisopropylnaphthalin, Benzylbenzoat oder Mischungen dieser Lösemittel.

Ein weiterer Gegenstand der vorliegenden Erfindung ist daher eine Formulierung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres

Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung. Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind. Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht eingesetzt werden.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769, WO 2019/020538, WO 2018/178001, WO 2019/115423 oder WO 2019/158453 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

| | | |
|---|---|---|
| 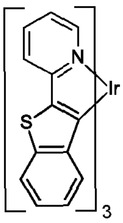 | 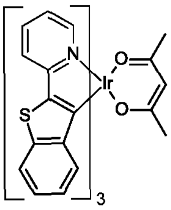 | 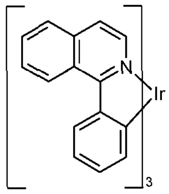 |
| 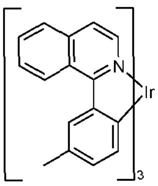 | 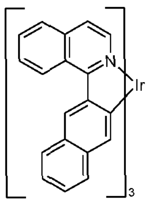 | 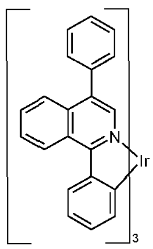 |
| 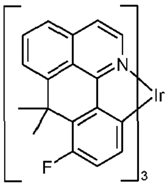 | 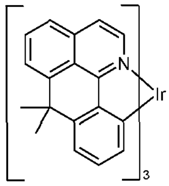 | 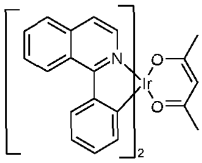 |
| 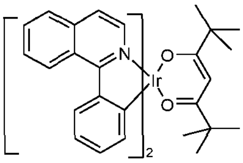 | 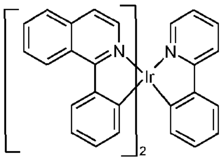 | 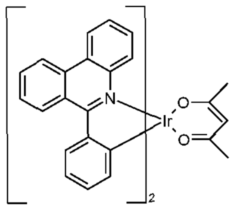 |
| 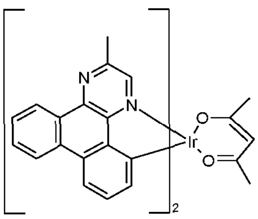 | 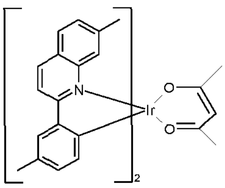 | 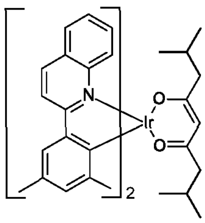 |
| 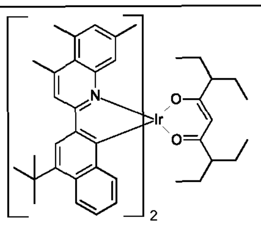 | 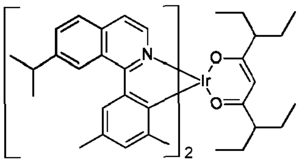 | 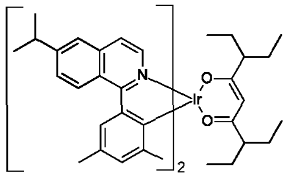 |
| 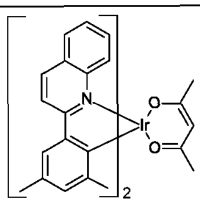 | 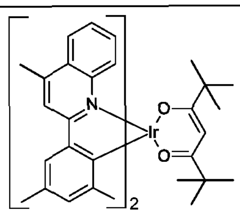 | 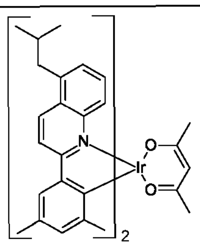 |

| | | |
|---|---|---|
| 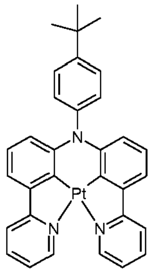 | 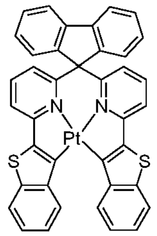 | 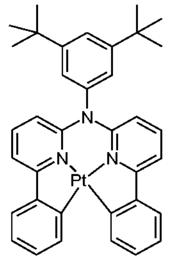 |
| 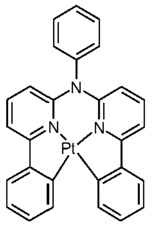 | 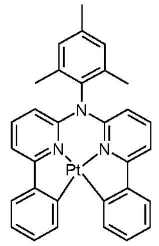 | 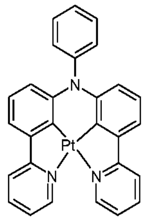 |
| 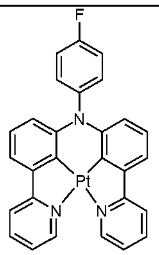 | 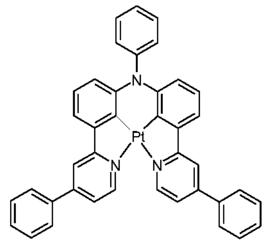 | 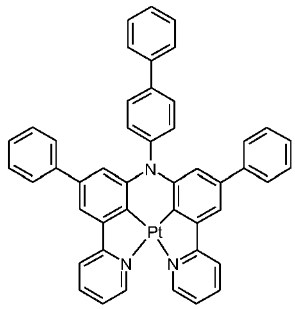 |
| 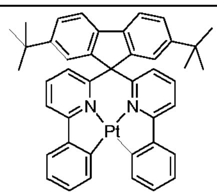 | 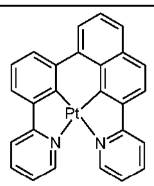 | 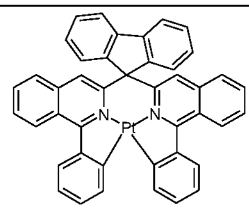 |
| 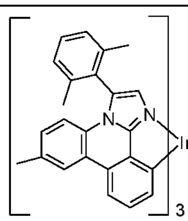 | 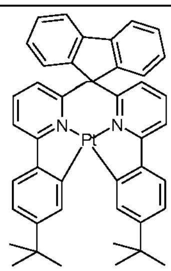 | 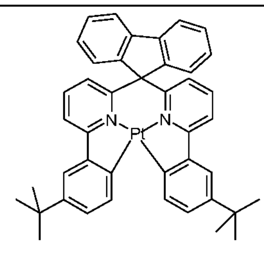 |
| 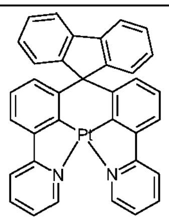 | 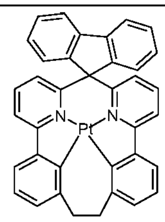 | 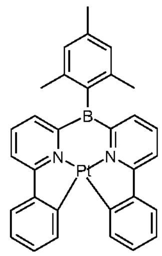 |

| | | |
|---|---|---|
| 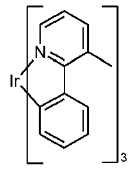 | 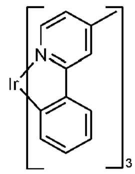 | 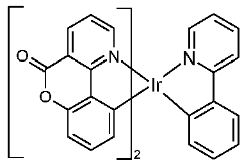 |
| 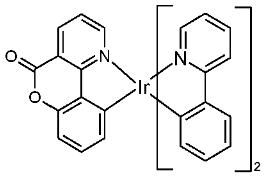 | 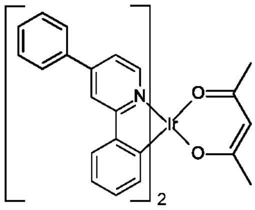 | 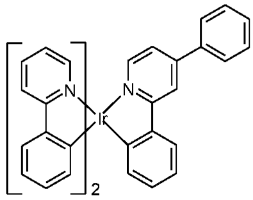 |
| 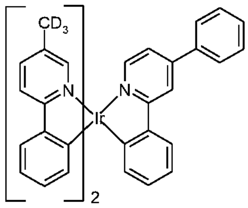 | 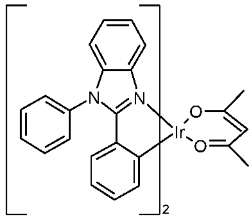 | 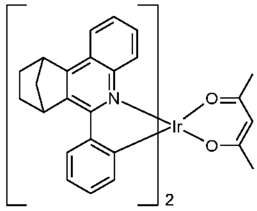 |
| 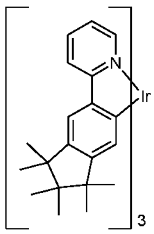 | 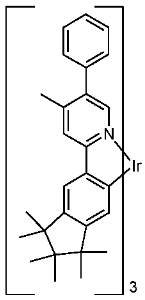 | 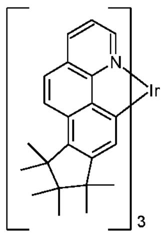 |
| 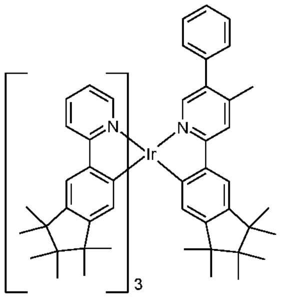 | 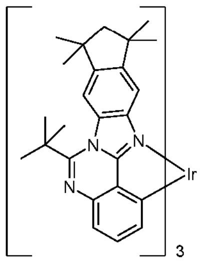 | 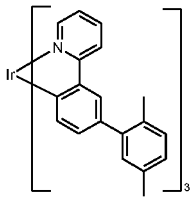 |
| 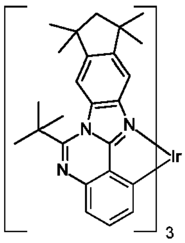 | 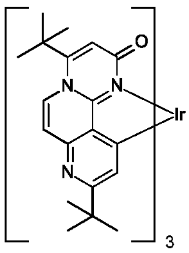 | 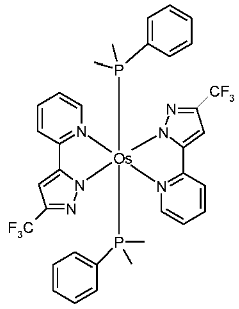 |

| | | |
|---|---|---|
| 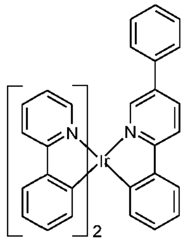 | 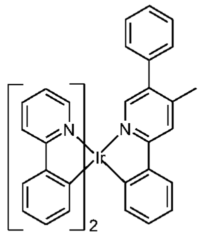 | 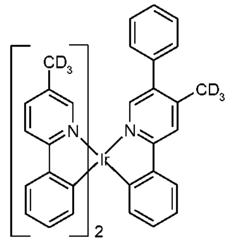 |
| 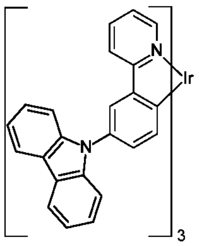 | 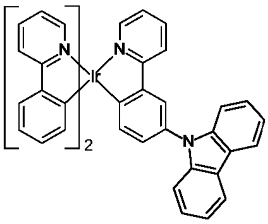 | 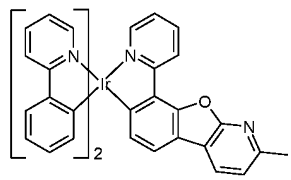 |
| 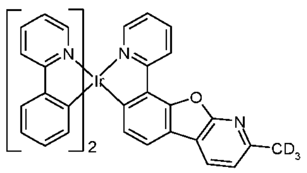 | 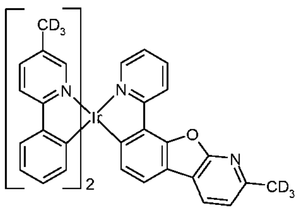 | 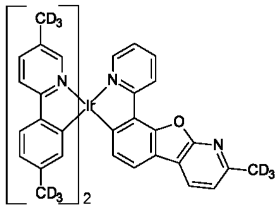 |
| 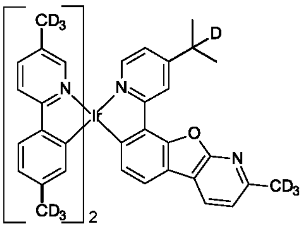 | 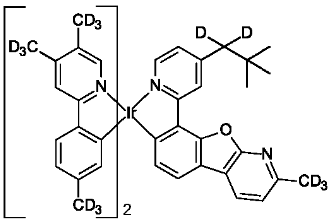 | 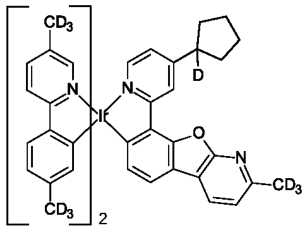 |
| 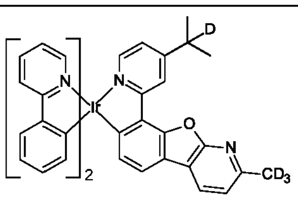 | 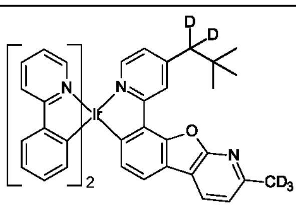 | 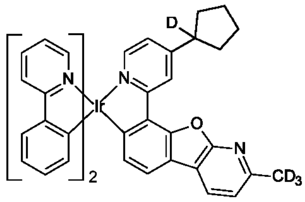 |
| 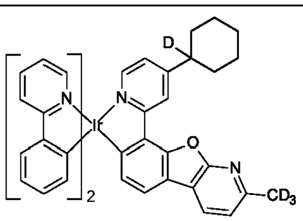 | 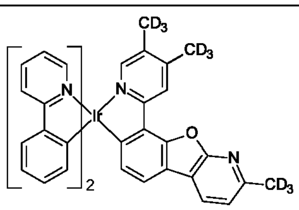 | 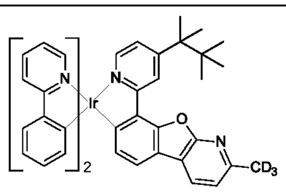 |
| 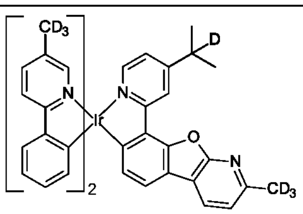 | 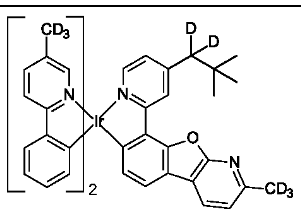 | 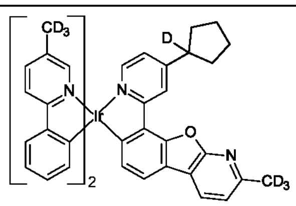 |

| | | |
|---|---|---|
| 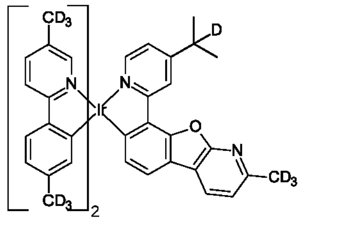 | 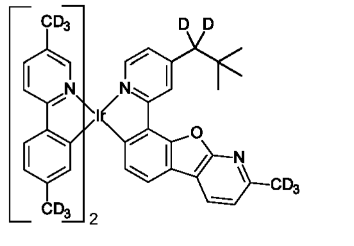 | 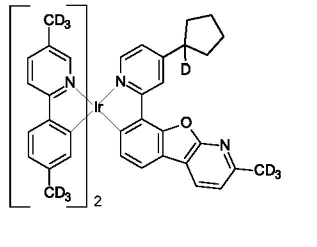 |
| 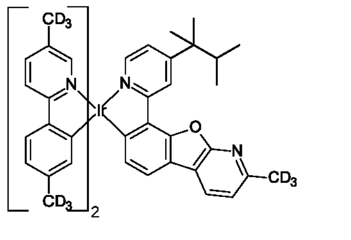 | 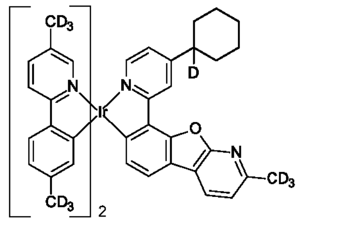 | 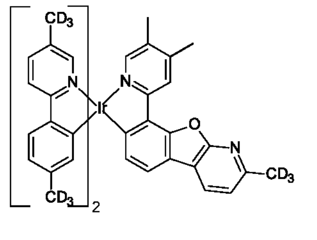 |
| 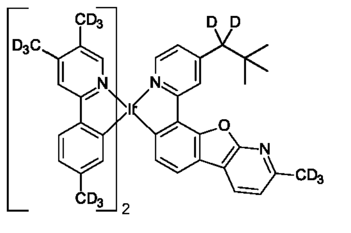 | 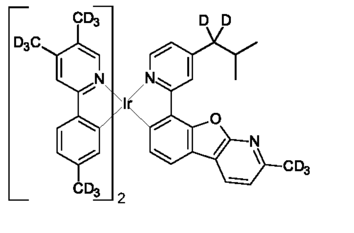 | 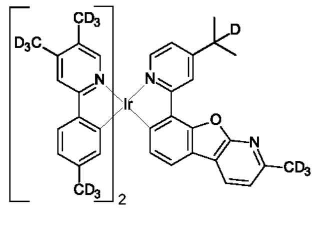 |
| 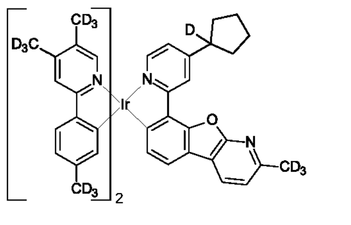 | 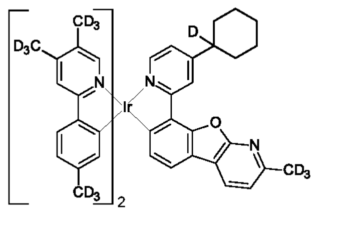 | 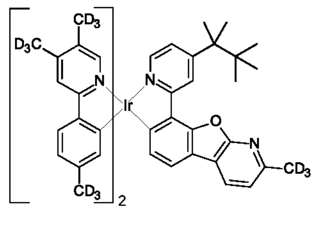 |
| 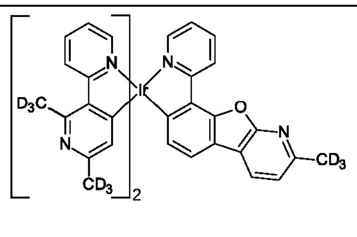 | 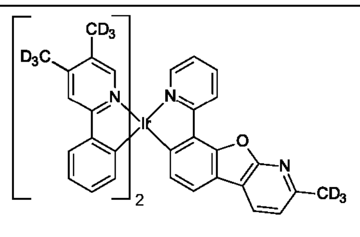 | 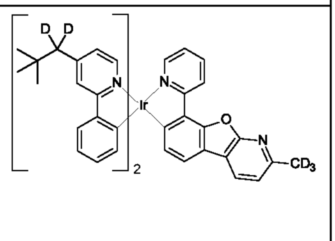 |
| 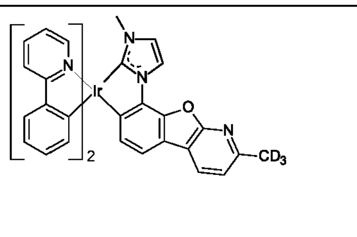 | 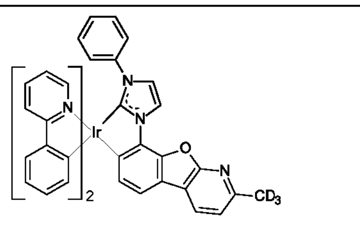 | 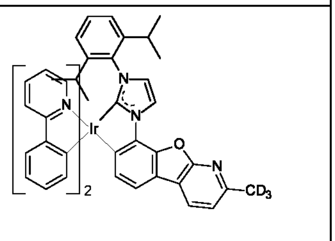 |
| 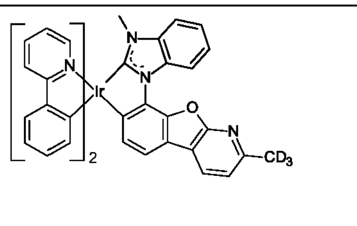 | 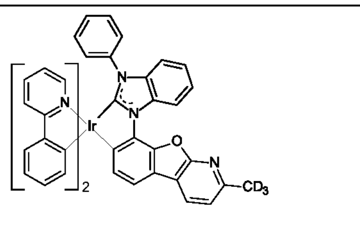 | 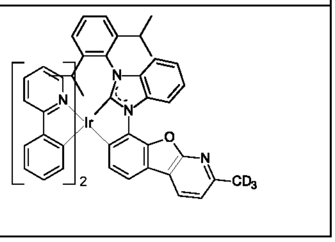 |

| | | |
|---|---|---|
| 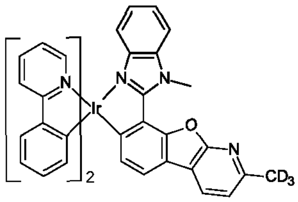 | 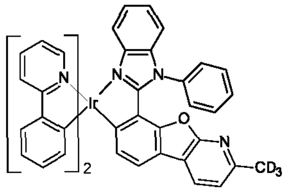 | 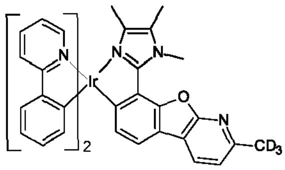 |
| 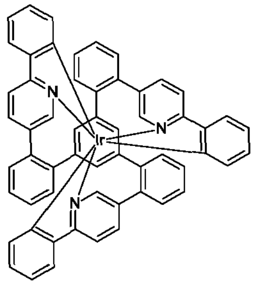 | 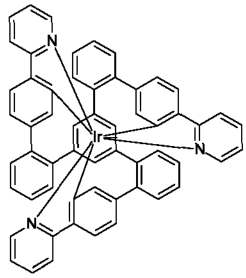 | 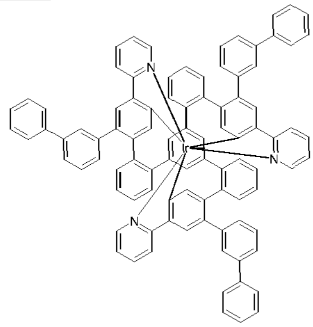 |
| 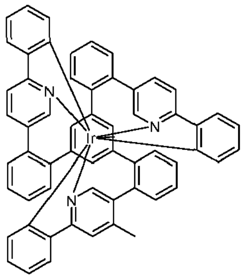 | 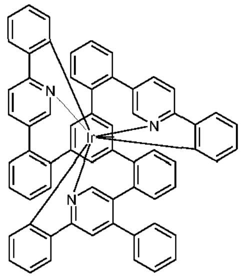 | 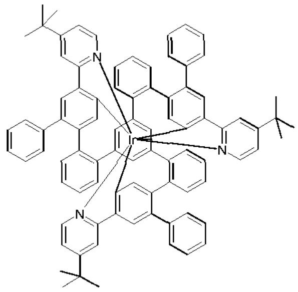 |
| 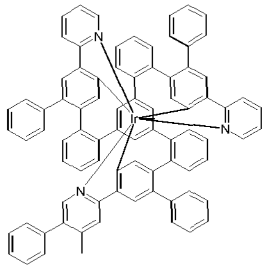 | 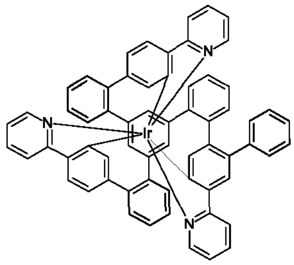 | 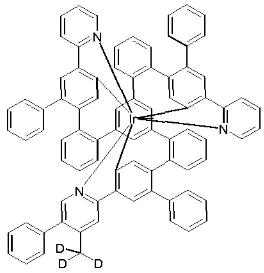 |
| 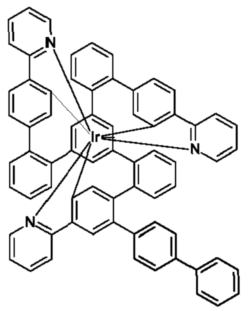 | 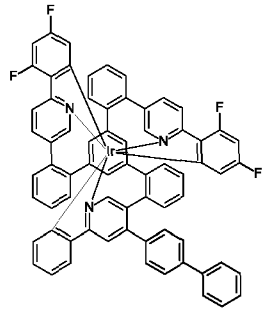 | 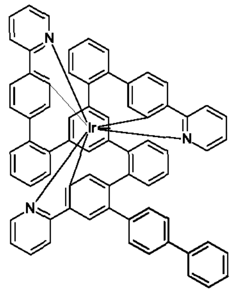 |

| | | |
|---|---|---|
| 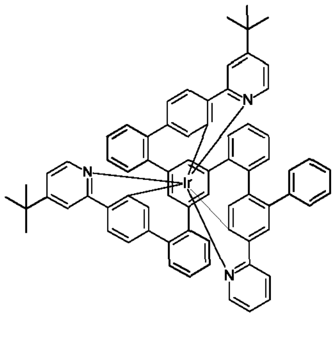 | 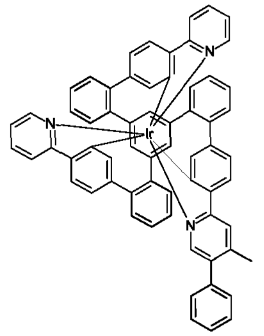 | 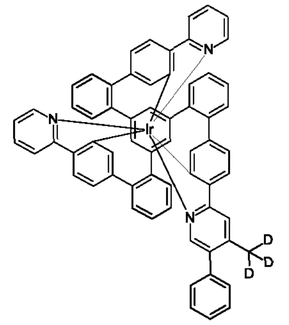 |
| 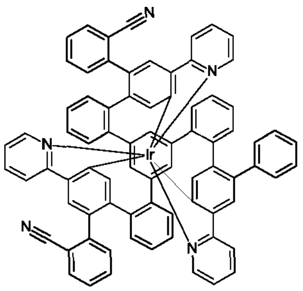 | 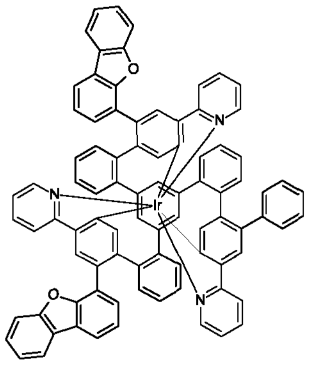 | 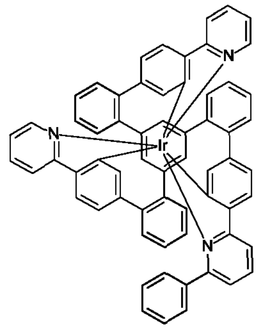 |
| 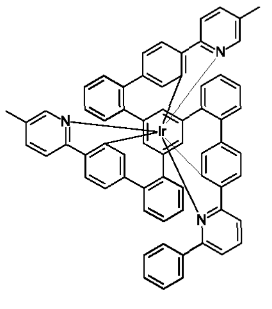 | 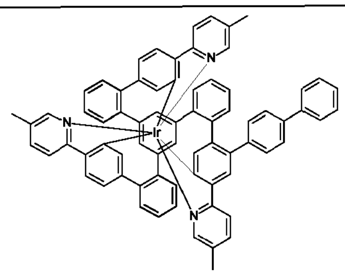 | 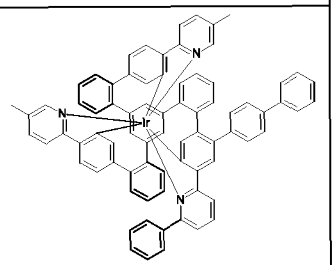 |
| 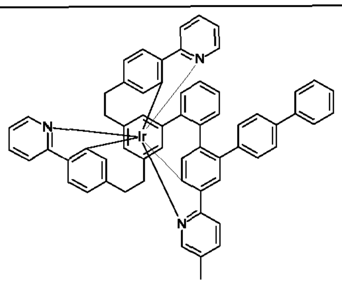 | 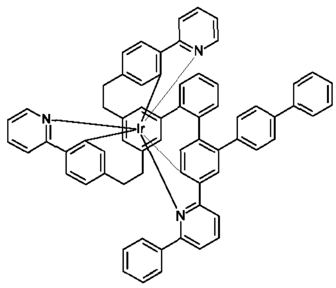 | 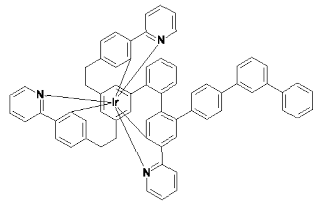 |
| 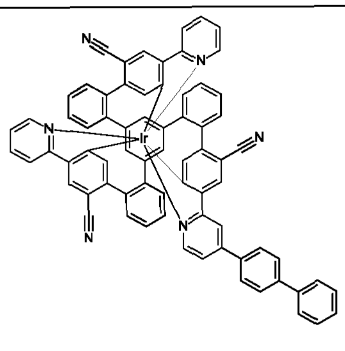 | 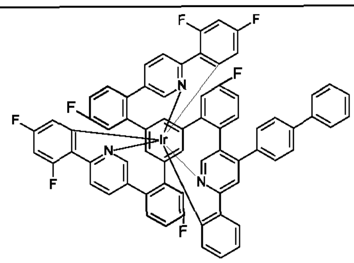 | 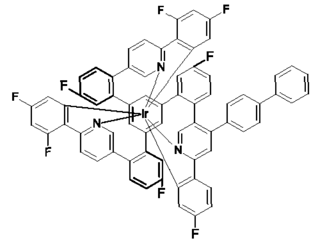 |

| | | |
|---|---|---|
| 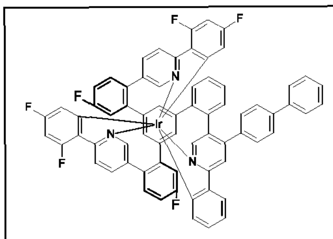 | 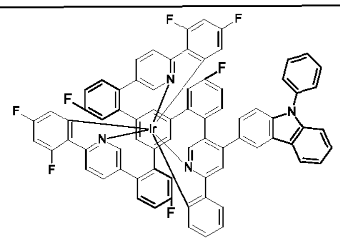 | 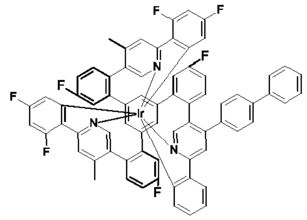 |
| 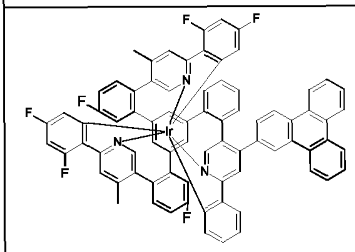 | 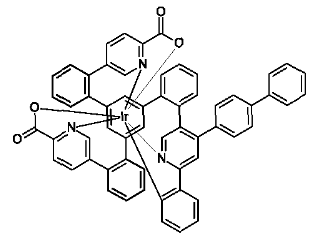 | 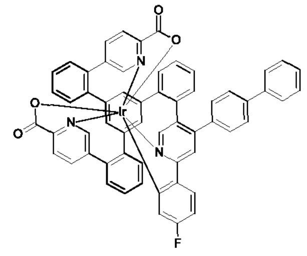 |
| 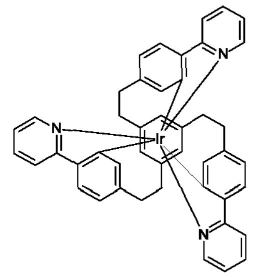 | 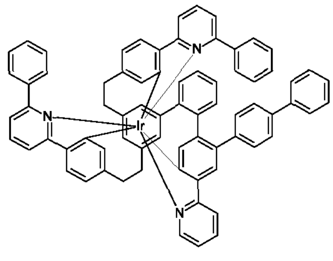 | 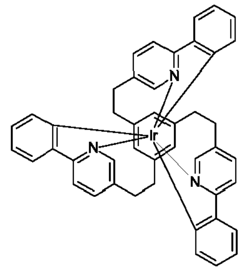 |
| 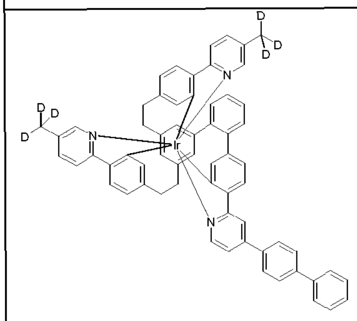 | 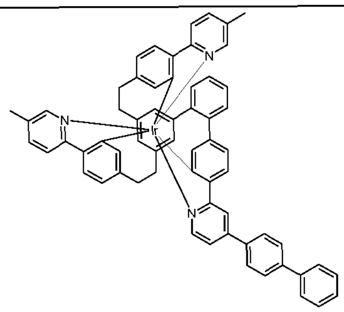 | 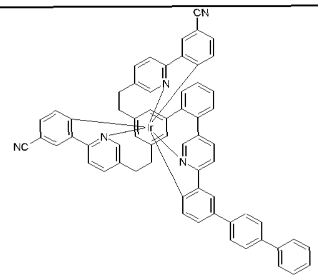 |
| 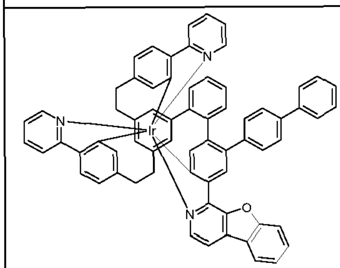 | 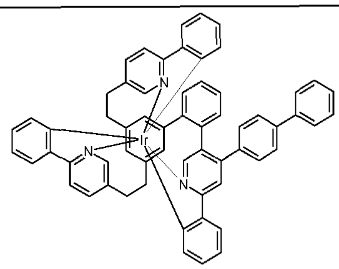 | 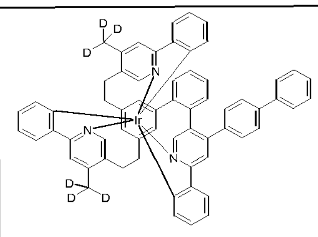 |
| 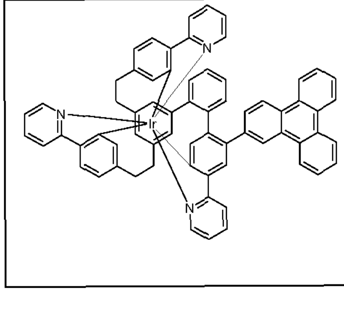 | 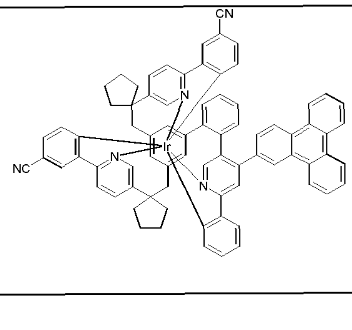 | 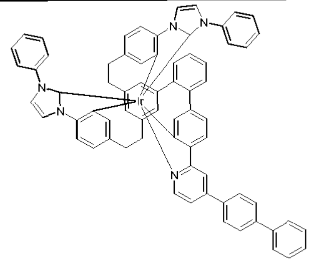 |

| | | |
|---|---|---|
| 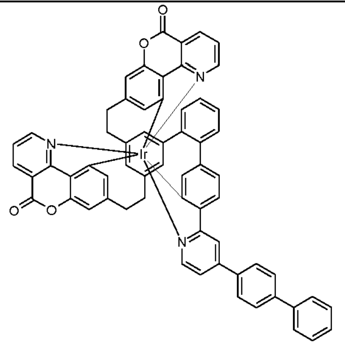 | 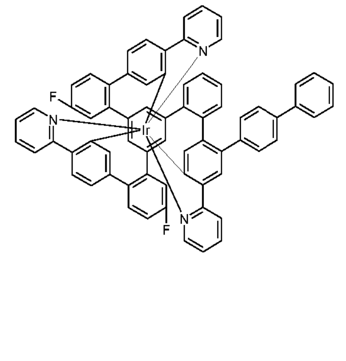 | 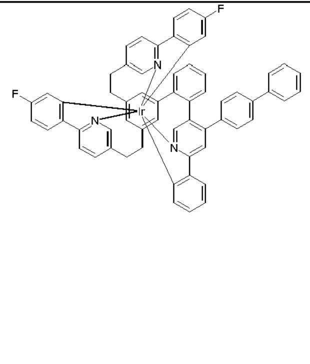 |
| 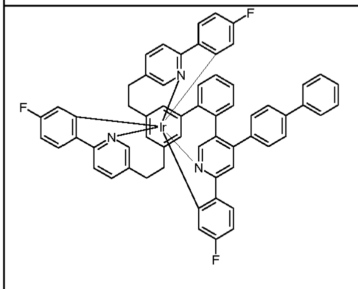 | 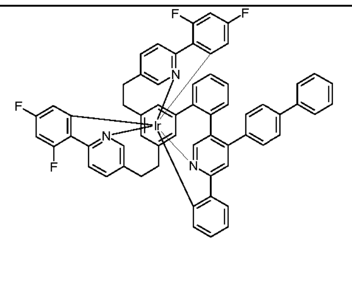 | 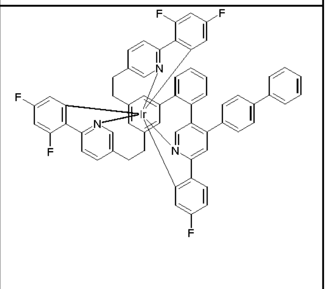 |
| 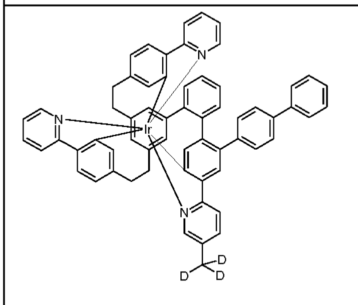 | 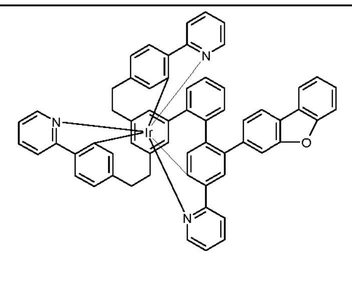 | 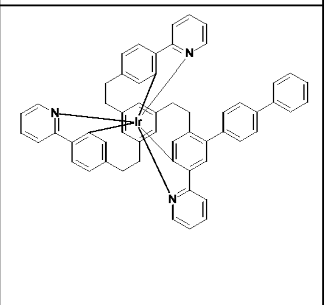 |
| 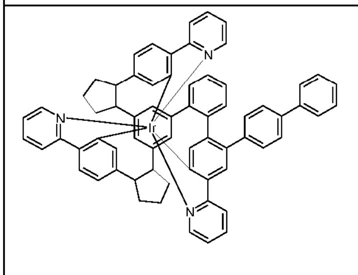 | 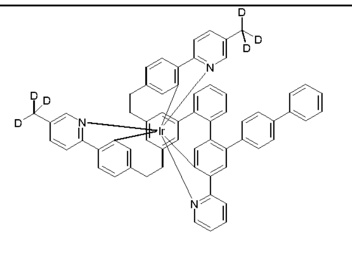 | 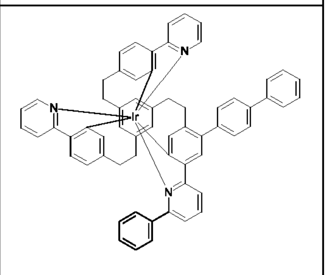 |
| 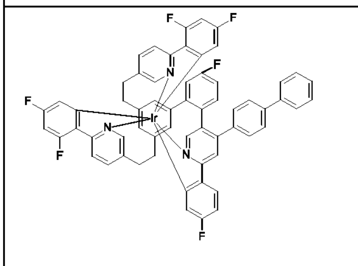 | 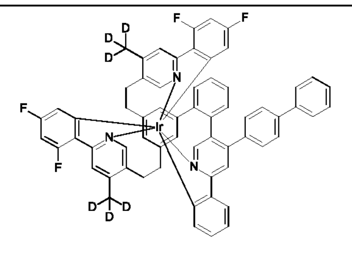 | 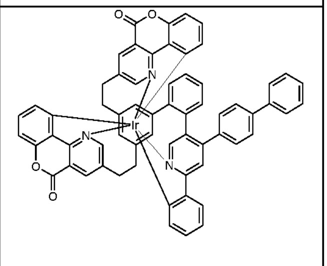 |
| 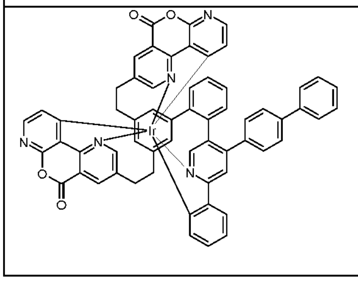 | 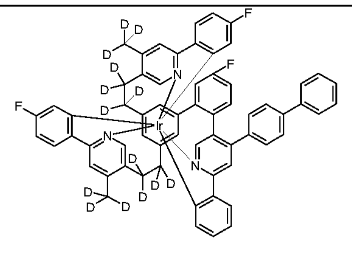 | 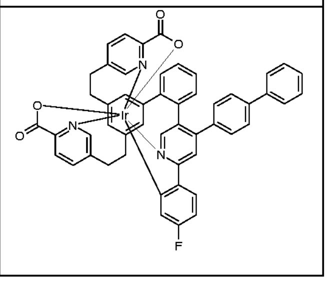 |

| | | |
|---|---|---|
| 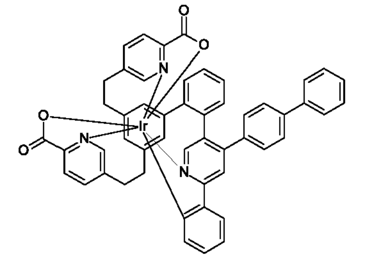 | 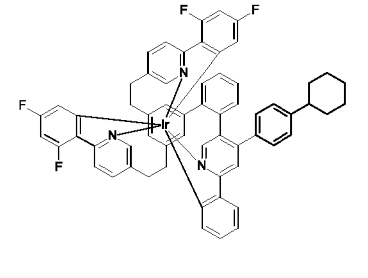 | 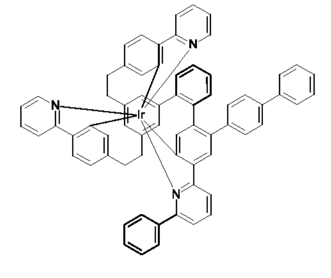 |
| 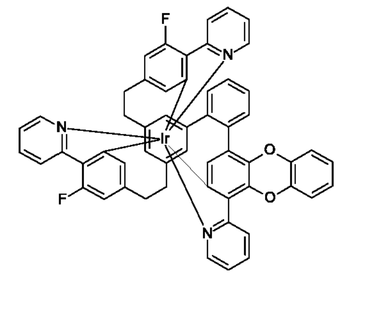 | 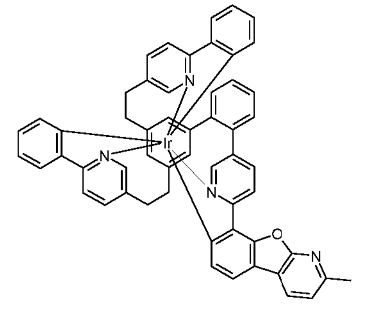 | 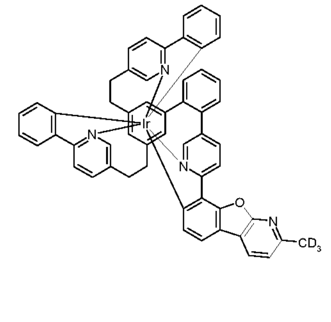 |
| 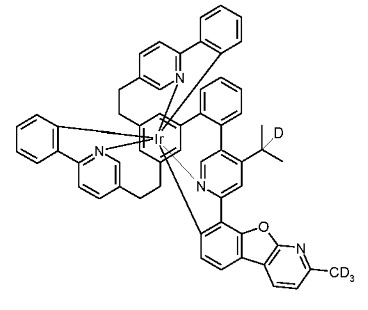 | 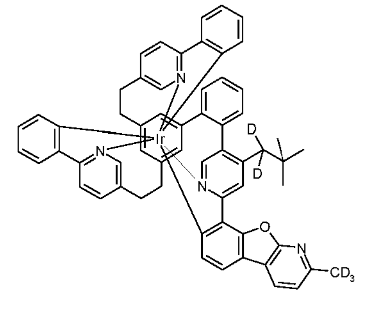 | 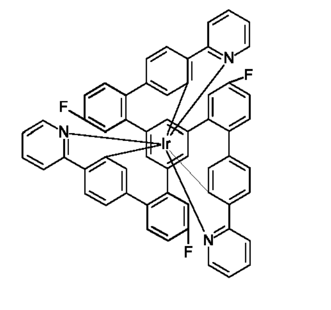 |
| 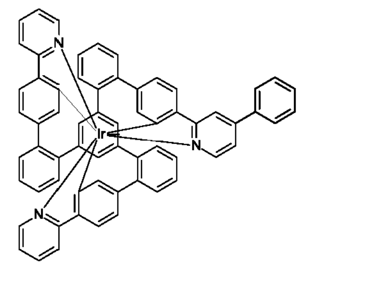 | 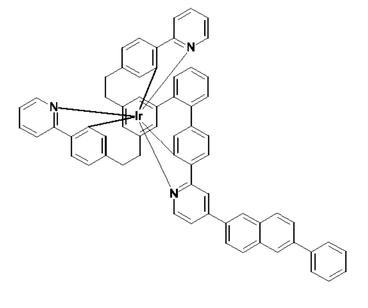 | 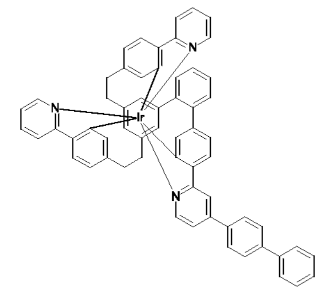 |
| 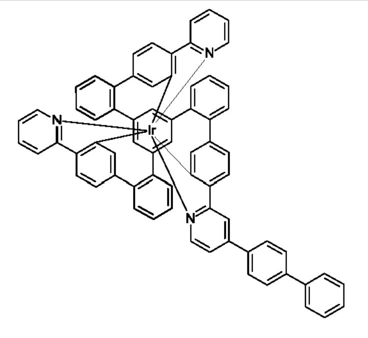 | 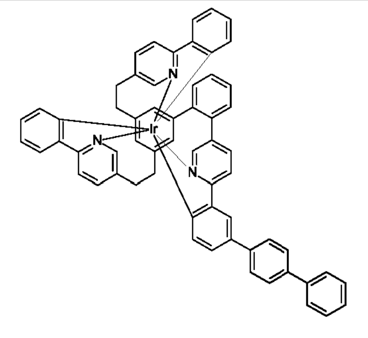 | 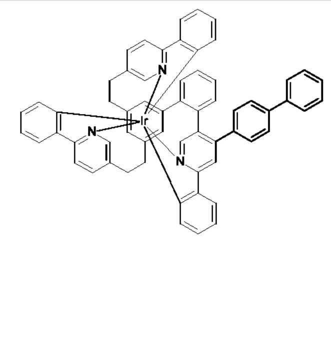 |

| | | |
|---|---|---|
| 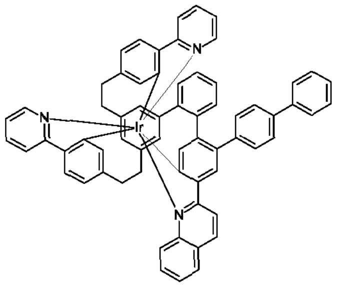 | 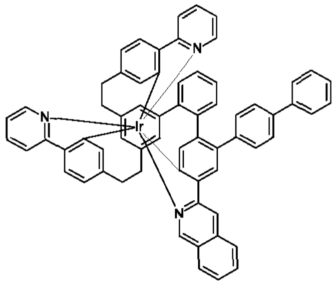 | 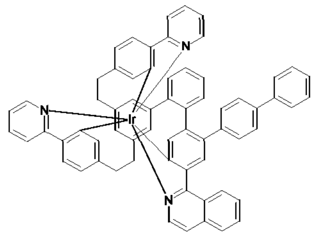 |
| 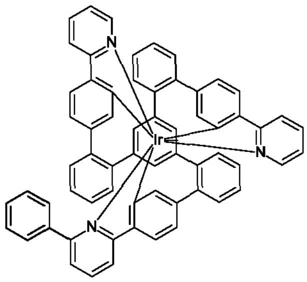 | 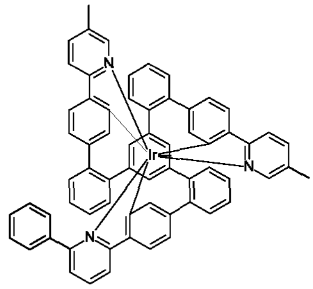 | 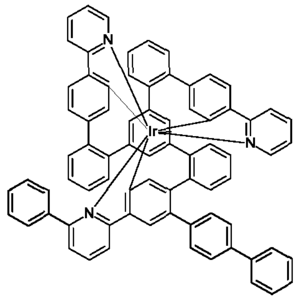 |
| 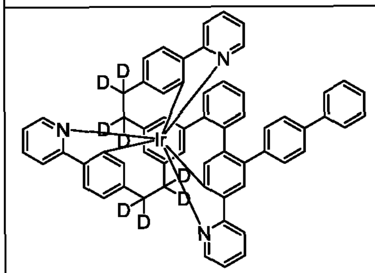 | 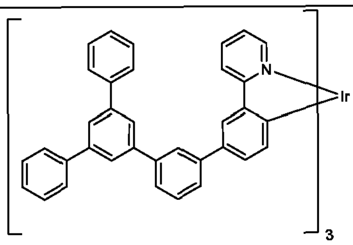 | 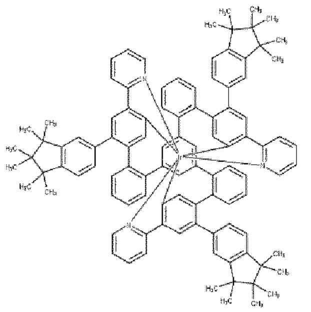 |
| 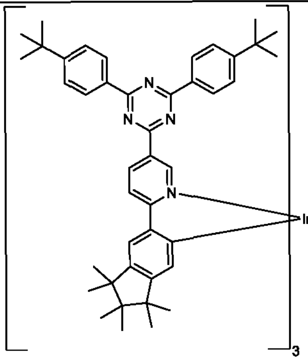 | 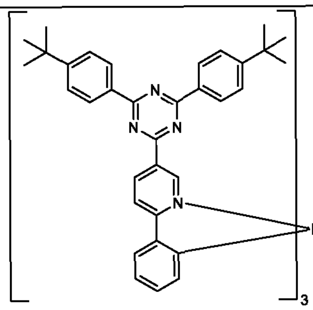 | 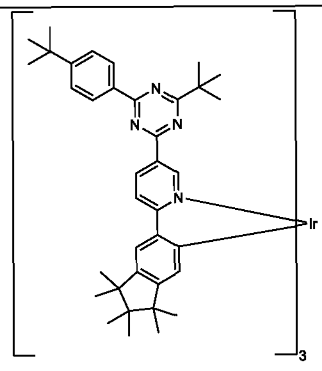 |
| 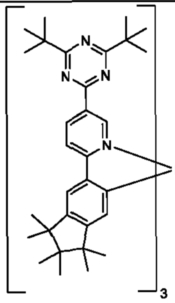 | | |

| | |
|---|---|
| 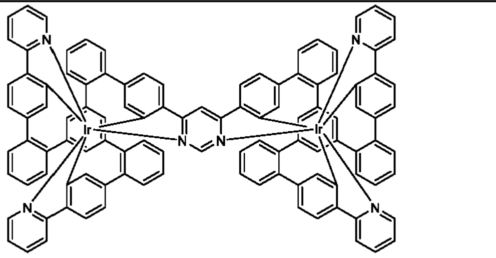 | 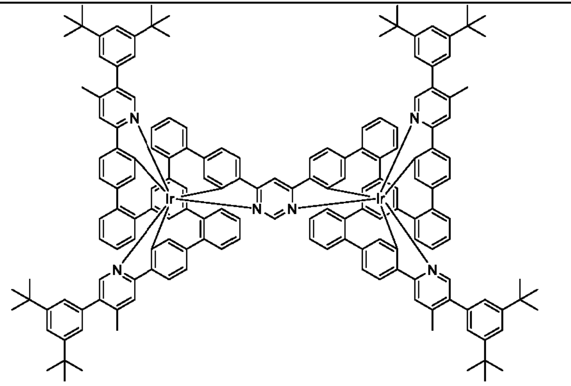 |
| 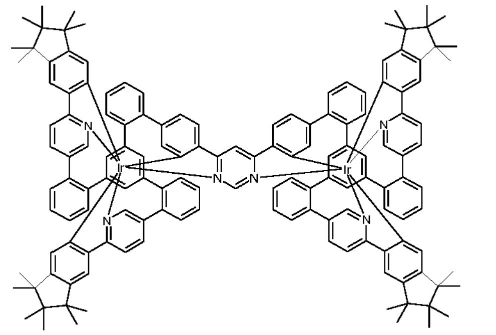 | 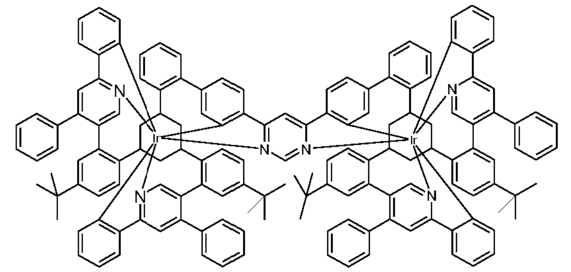 |

Wenn die organische Elektrolumineszenzvorrichtung aus Lösung hergestellt wird, enthält die Mischung aus dem Host insgesamt, der die erfindungsgemäße Verbindung enthält, und der emittierenden Verbindung zwischen 99 und 1 Gew.-%, bevorzugt zwischen 97 und 50 Gew.-%, besonders bevorzugt zwischen 96 und 60 Gew.-% und insbesondere bevorzugt zwischen 95 und 75 Gew.-% des Hosts, der die erfindungsgemäße Verbindung enthält. Entsprechend enthält die Mischung zwischen 1 und 99 Gew.-%, vorzugsweise zwischen 3 und 50 Gew.-%, besonders bevorzugt zwischen 5 und 40 Gew..-%, insbesondere zwischen 5 und 25 Gew.-% des Emitters bezogen auf die Gesamtmischung aus Emitter und allen Matrixmaterialien.

Dabei kann das Hostmaterial, wie nachfolgend noch genauer ausgeführt, auch eine Mischung aus zwei oder mehreren Materialien sein, von denen mindestens eines eine erfindungsgemäße Verbindung ist. Der Anteil des erfindungsgemäßen Hostmaterials, also der Verbindung gemäß Formel (1) bzw. der bevorzugten Ausführungsformen, liegt dabei zwischen 99 und 1 Gew.-%, bevorzugt zwischen 95 und 10 Gew.-%, besonders bevorzugt zwischen 80 und 20 Gew.-% und ganz besonders bevorzugt zwischen 70 und 25 Gew.-% bezogen auf alle Komponenten der emittierenden Schicht.

Wenn die organische Elektrolumineszenzvorrichtung durch Aufdampfen hergestellt wird, enthält die Mischung aus der erfindungsgemäßen Verbindung und der emittierenden Verbindung zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol-

bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565, oder Dibenzothiophenderivaten.

Ebenso kann ein weiterer phosphoreszierender Emitter, welcher kürzerwellig als der eigentliche Emitter emittiert, als Co-Host in der Mischung vorhanden sein. So ist es beispielsweise möglich, wenn eine rot emitterende OLED gebaut werden soll, zusätzlich zu dem rot phosphoreszierenden Emitter einen grün phosphoreszierenden Emitter einzusetzen.

Weiterhin kann eine Verbindung, die nicht oder nicht in wesentlichem Umfang am Ladungstransport teilnimmt, wie beispielsweise in WO 2010/108579, WO 2016/184540 oder der noch nicht offen gelegten Anmeldung EP 19152285.3 beschrieben, als zusätzliches, so genannten Wide Bandgap Matrixmaterial eingesetzt werden. In einer bevorzugten Ausführungsform der Erfindung ist das Wide Bandgap Material ein Kohlenwasserstoff, enthält also keine Heteroatome.

Ein Wide Bandgap Matrixmaterial im Sinne der vorliegendne Erfindung ist ein Material, das sich in der verwendeten Mischung der emittierenden Schicht dadurch auszeichnet, dass sein HOMO mindestens 0.2 eV kleiner ist als das höchste HOMO aller anderen in der Mischung vertretenen Materialien (Emitter und Hostmaterialien) und dass sein LUMO mindestens 0.2 eV größer ist als das niedrigste LUMO aller anderen in der Mischung vertretenen Materialien (Emitter und Hostmaterialien).

Die angegebenen Energiewerte beziehen sich dabei auf isolierte Verbindungen, und werden, wie im Folgenden dargelegt, ermittelt.

Die HOMO (highest occupied molecular orbital) und LUMO (lowest unoccupied molecular orbital) Energien sowie das Triplettniveau der Materialien werden über quantenchemische Rechnungen bestimmt. Hierzu wird vorliegend das Programmpaket "Gaussian09, Revision D.01" (Gaussian Inc.) verwendet. Es können aber auch andere Programmpakete verwendet werden, sofern darin dieselben Methoden implementiert wurden. Zur Berechnung organischer Substanzen ohne Metalle wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für elektronischen Grundzustand und Triplett-Niveau. Hierbei wird die TDDFT-Methode (Time Dependent Density Functional Theory) B3PW91 mit dem Basissatz 6-31G(d) (Gaussian-Eingabezeile "# B3PW91/6-31G(d) td=(50-50,nstates=4)") verwendet (Ladung 0, Multiplizität 1). Für metallorganische Verbindungen wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt analog zu den organischen Substanzen, wie oben beschrieben, mit dem Unterschied, dass für das Metallatom der Basissatz "LanL2DZ" und für die Liganden der Basissatz "6-31G(d)" verwendet wird (Gaussian-Eingabezeile "#B3PW91/gen pseudo=lanl2 td=(50-50,nstates=4)"). Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten (HEh bzw. LEh). Daraus wird der anhand von Cyclovoltammetriemessungen kalibrierte HOMO- und LUMO-Wert in Elektronenvolt wie folgt bestimmt:

$$\mathrm{LUMO(eV)} = (1.0658 * \mathrm{LEh} * 27.212) - 0.5049$$

$$\mathrm{HOMO(eV)} = (0.8308 * \mathrm{HEh} * 27.212) - 1.1180$$

Diese Werte sind im Sinne dieser Anmeldung als HOMO bzw. als LUMO der Materialien anzusehen.

Beispiele für geeignete Wide Bandgap Materialien sind die in der folgenden Tabelle aufgeführten Strukturen.

| | |
|---|---|
| 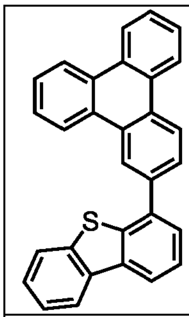 | 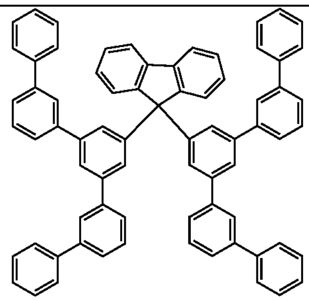 |
| 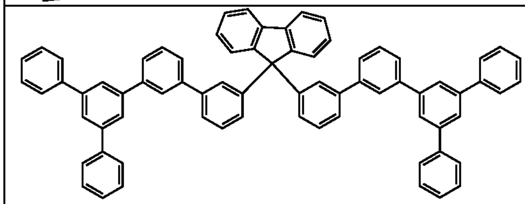 | 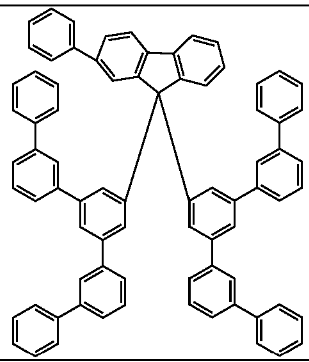 |
| 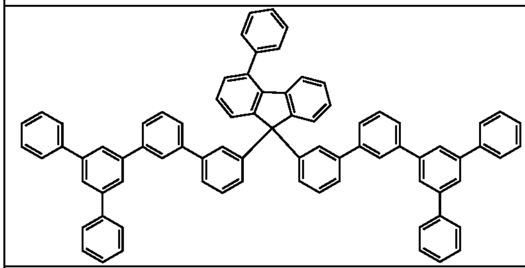 | 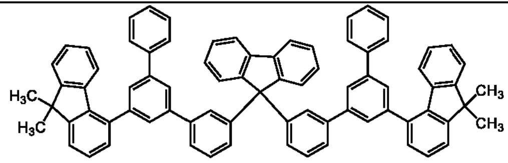 |
| 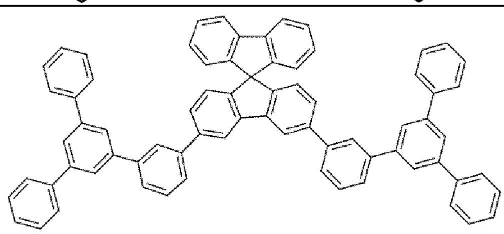 | 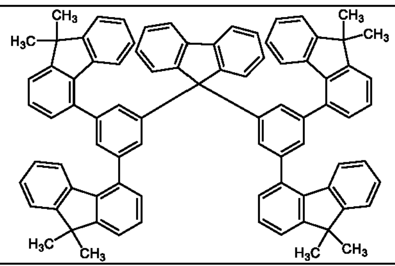 |
| 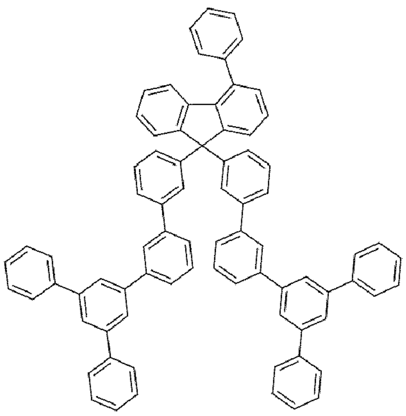 | 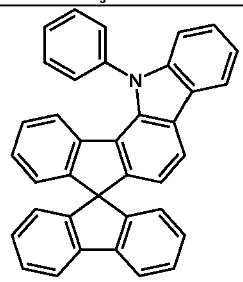 |

| | |
|---|---|
| 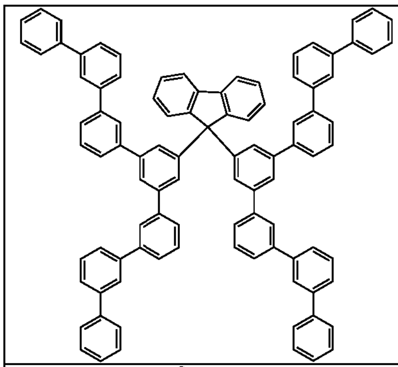 | 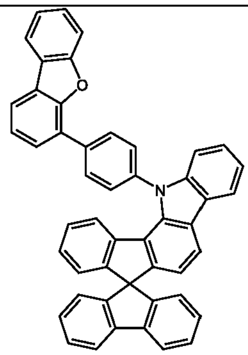 |
| 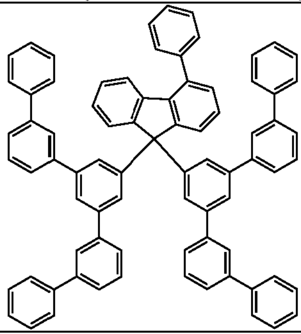 | 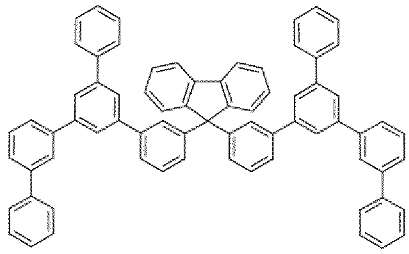 |
| 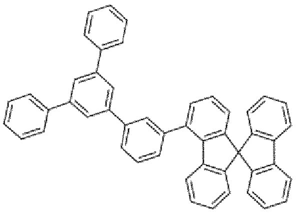 | |

Weiterhin ist es möglich, die erfindungsgemäßen Verbindungen in einer Lochblockier- und/oder Elektronentransportschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren aufgebracht werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Es ist aber auch möglich, dass der Anfangsdruck noch geringer oder höher ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation aufgebracht werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Es hat sich gezeigt, dass die erfindungsgemäßen Verbindungen gerade für die Verarbeitung aus Lösung besonders geeignet sind.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen eignen sich sehr gut für die Verarbeitung aus Lösung. Sie weisen dabei

gleichzeitig eine sehr gute Löslichkeit in gängigen organischen Lösemitteln und eine hohe Glasübergangstemperatur auf. Die Verwendung der erfindungsgemäßen Verbindungen als Matrixmaterial für phosphoreszierende Emitter in aus Lösung hergestellten organischen Elektrolumineszenzvorrichtungen führt zu einer signifikanten Verbesserung der Lebensdauer der organischen Elektrolumineszenzvorrichtungen im Vergleich zu Verbindungen gemäß dem Stand der Technik, insbesondere im Vergleich zu Verbindungen, die nur eine Dibenzofuran-Carbazol-Einheit am Triazin enthalten oder zu Verbindungen, bei denen das Triazin zwar mit zwei oder mehr 1-Dibenzofurangruppen substituiert ist, aber nur eine Dibenzofurangruppe mit einer Carbazolgruppe substituiert ist. Dabei sind die weiteren Eigenschaften der organischen Elektrolumineszenzvorrichtung, insbesondere die Effizienz und die Spannung, vergleichbar oder besser.

Die Erfindung wird nun durch die nachfolgenden Beispiele näher erläutert, ohne sie dadurch einschränken zu wollen.

## Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Stufe 1: BB-1a

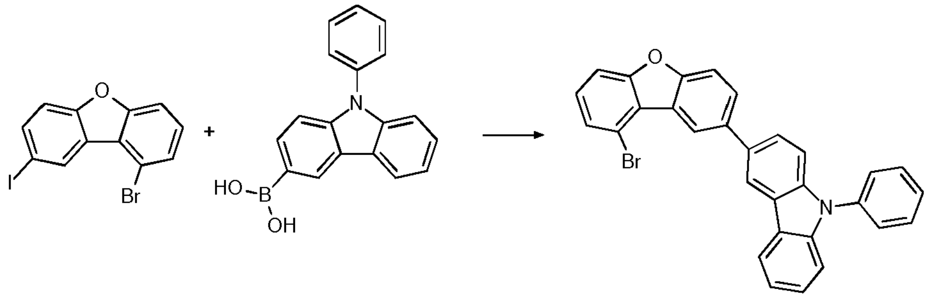

100 g (26 mmol) 1-Brom-8-iod-dibenzofuran (CAS: 1822311-11-4), 92.4 g (32 mmol) (9-Phenyl-9H-carbazol-3-yl)boronsäure, 85.2 g (61 mmol) Kaliumcarbonat und 15.5 g (13 mmol) Tetrakis(triphenylphosphin)-palladium(0) werden in 1 L Ethylenglycoldimethylether/Wasser (3:1) gemischt und über Nacht unter Rückfluss erhitzt. Man lässt die Mischung auf Raumtemperatur kommen, filtriert den Niederschlag ab und wäscht ihn mit Wasser und Ethanol. Ausbeute: 86 g (176 mmol, 66 %).

Die folgenden Verbindungen können analog synthetisiert werden:

| Bsp. | Edukt 1 | Edukt 2 | Produkt |
|---|---|---|---|
| BB-1b | 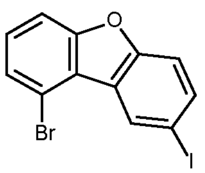 CAS 1822311-11-4 | 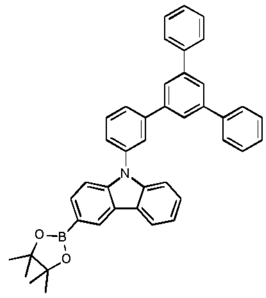 CAS 1846559-20-3 | 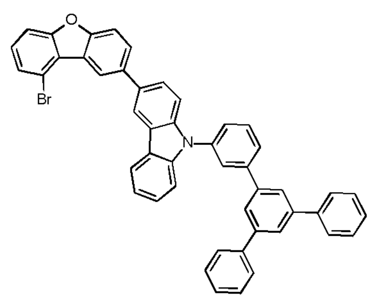 |

(fortgesetzt)

| Bsp. | Edukt 1 | Edukt 2 | Produkt |
|---|---|---|---|
| BB-1c | 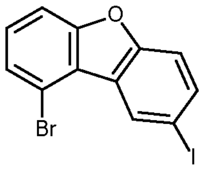 CAS 1822311-11-4 | 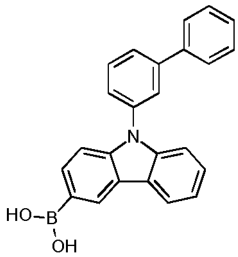 CAS 1416814-68-0 | 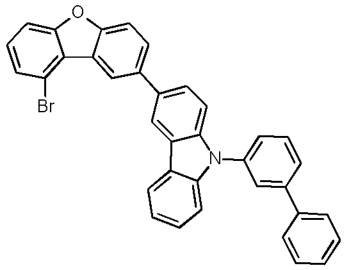 |
| BB-1d | 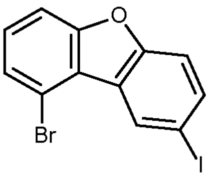 CAS 1822311-11-4 | 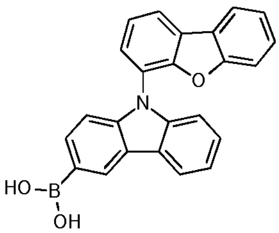 CAS 1547397-15-8 | 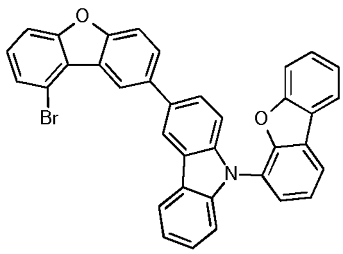 |
| BB-1e | 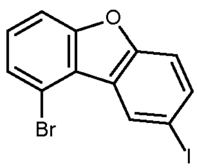 CAS 1822311-11-4 | 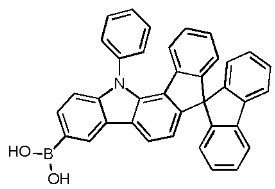 | 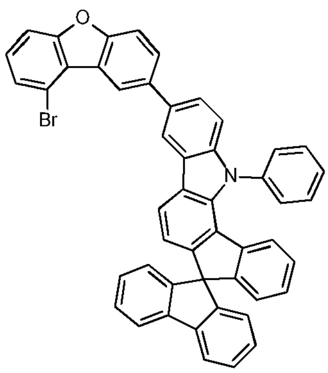 |
| BB-1f | 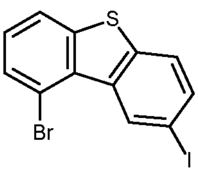 CAS 1822311-12-5 | 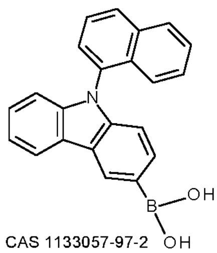 CAS 1133057-97-2 | 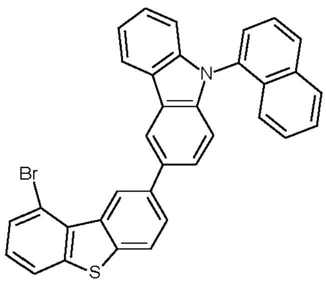 |

(fortgesetzt)

| Bsp. | Edukt 1 | Edukt 2 | Produkt |
|---|---|---|---|
| BB-1g | 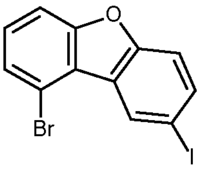 CAS 1822311-11-4 | 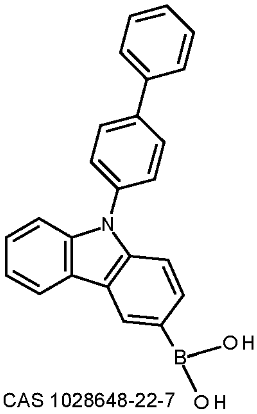 CAS 1028648-22-7 | 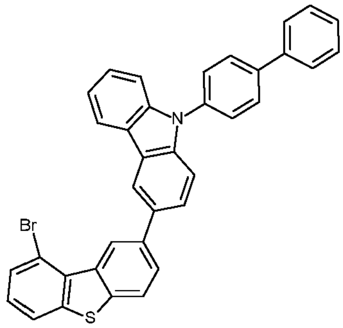 |
| BB-1h | 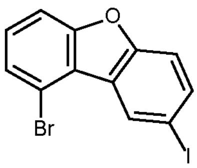 CAS 1822311-11-4 | 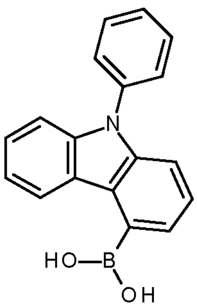 CAS 137055-65-9 | 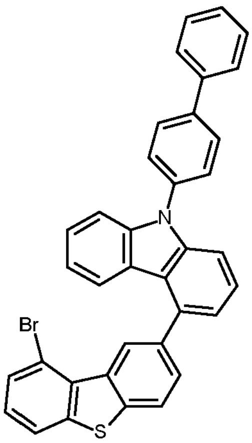 |
| BB-1i | 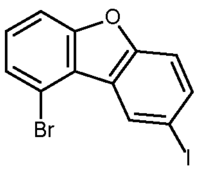 CAS 1822311-11-4 | 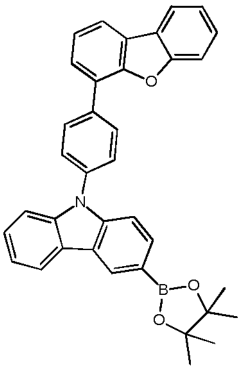 CAS 2260995-43-3 | 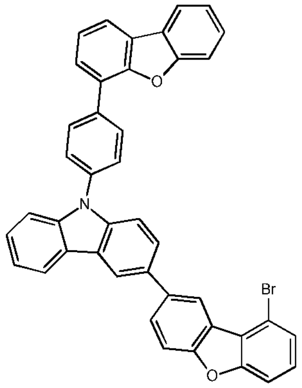 |
| BB-1j | 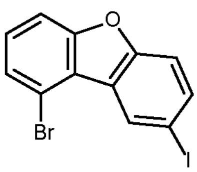 CAS 1822311-11-4 | 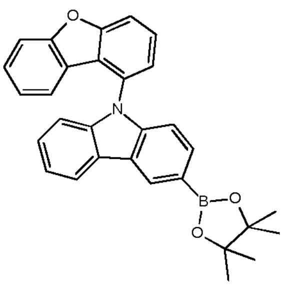 CAS 1427160-10-8 | 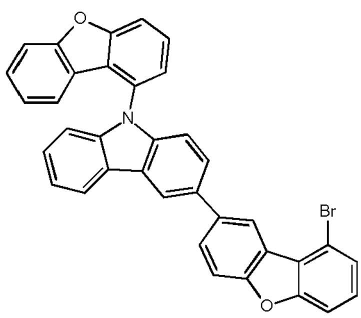 |

**Stufe 2: BB-2a**

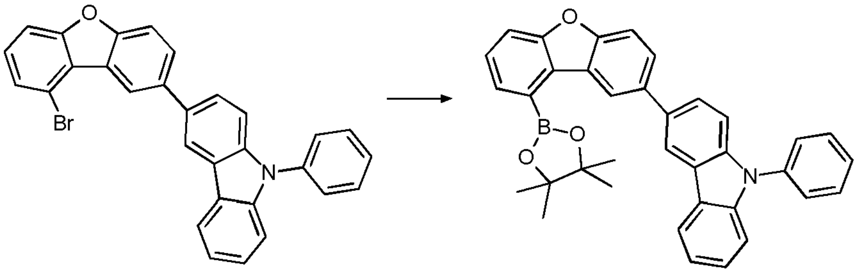

86 g (176 mmol) 3-(9-Brom-dibenzofuran-2-yl)-9-phenyl-9H-carbazol, 82.1 g (320 mmol) 4,4,5,5,4',4',5',5'-Octamethyl-[2,2']bi[[1,3,2]dioxaborolanyl], 52.4 g (530 mmol) Kaliumacetat and 2.8 g (12 mmol) Palladiumacetat werden in 1 L DMF gemischt und bei bei 100 °C gerührt. Nach 24 h lässt man die Reaktionsmischung auf Raumtemperatur abkühlen und engt die Mischung unter verringertem Druck auf ein Drittel ein. Man gibt Wasser zu und filtriert den ausgefallenen Feststoff ab, wäscht mit Wasser, Ethanol und Heptan und reinigt weiter durch Filtration über Silica mit THF als Eluent. Das Produkt wird durch Entfernen der Lösemittel unter verringertem Druck erhalten. Ausbeute: 58.2 g (109 mmol, 62 %).

Die folgenden Verbindungen können analog synthetisiert werden:

| Bsp. | Edukt | Produkt |
|---|---|---|
| BB-2b | 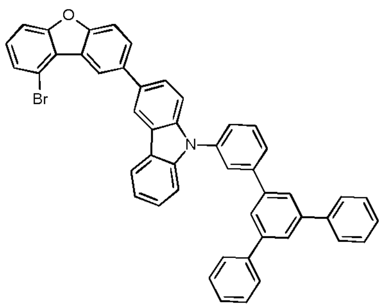 | 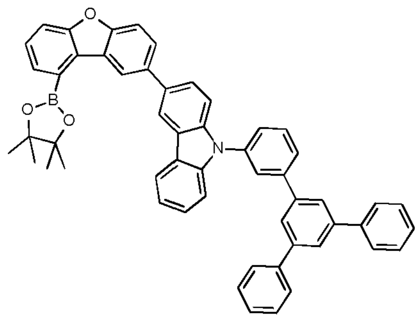 |
| BB-2c | 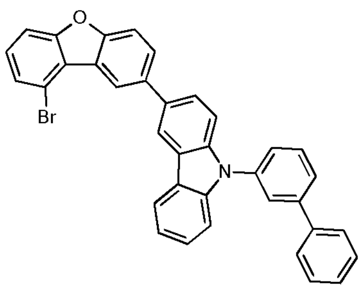 | 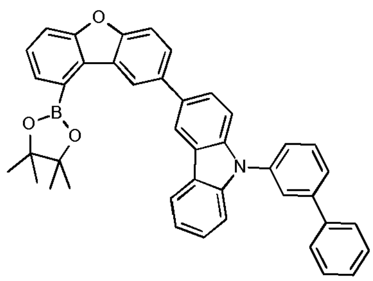 |
| BB-2d | 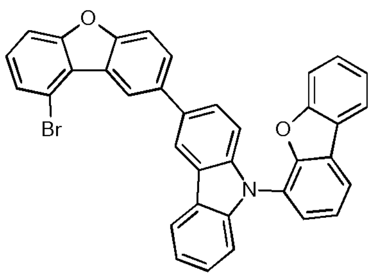 | 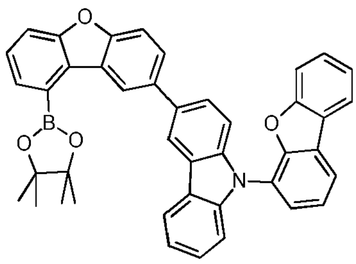 |

(fortgesetzt)

| Bsp. | Edukt | Produkt |
|---|---|---|
| BB-2e | 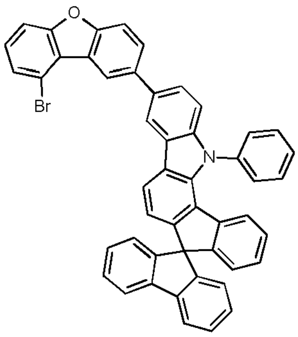 | 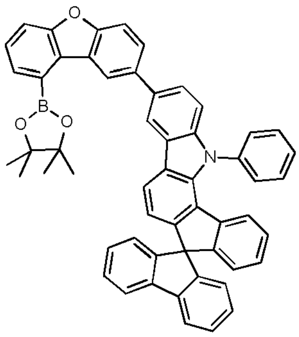 |
| BB-2f | 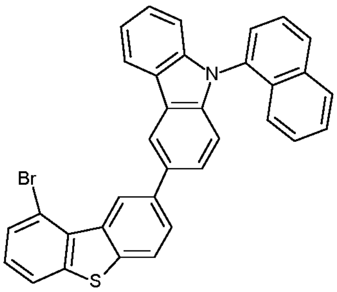 | 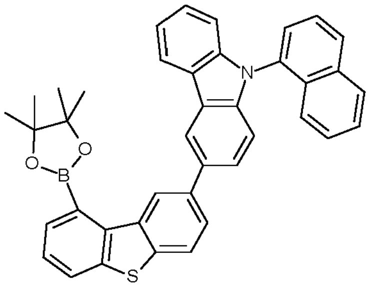 |
| BB-2g | 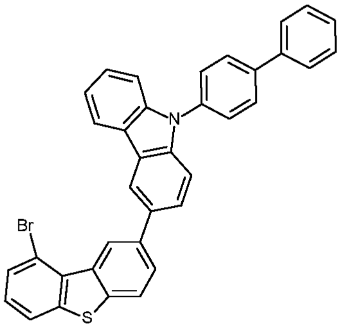 | 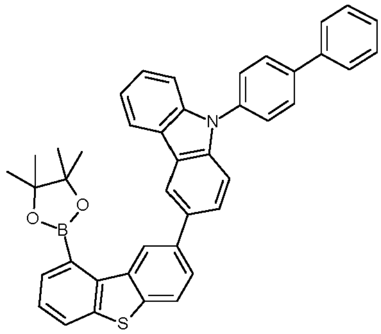 |
| BB-2h | 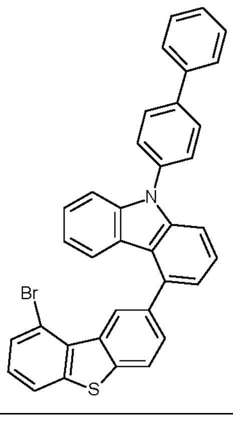 | 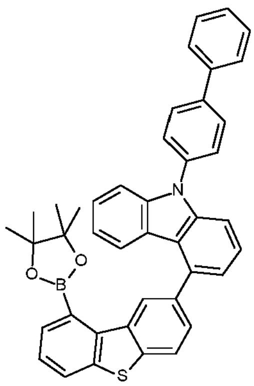 |

(fortgesetzt)

| Bsp. | Edukt | Produkt |
|---|---|---|
| BB-2i | 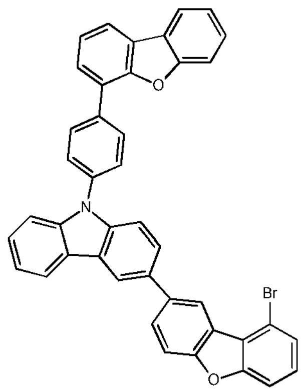 | 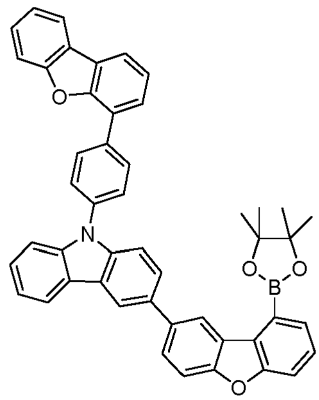 |
| BB-2j | 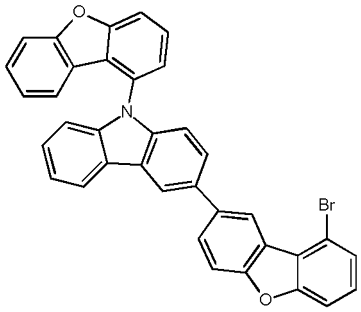 | 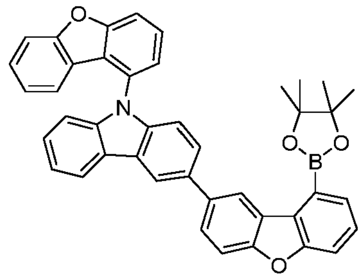 |

**Stufe 3: BB-3a**

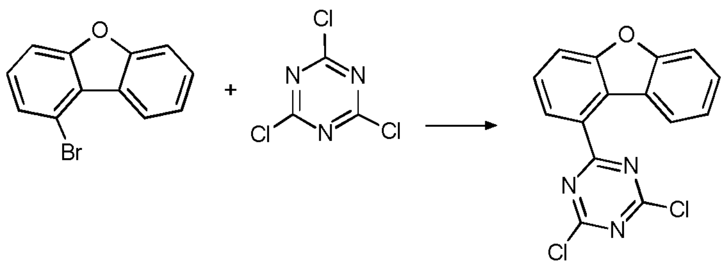

21.1 g (85.4 mmol) 1-Brom-dibenzofuran in 100 ml THF wird tropfenweise zu 2.27 g (93.5 mmol) Magnesium (unter Zusatz von Iod zum Starten der Grignard Reaktion) gegeben, und die Mischung wird 2 h unter Rückfluss erhitzt. Man gibt 350 mL THF hinzu und lässt die Reaktionsmischung auf Raumtemperatur kommen. Das Grignard-Reagens wird tropfenweise zu 15 g (81 mmol) 2,4,6-Trichlor-[1,3,5]triazin, gelöst in 150 mL THF, bei -10 °C gegeben. Man lässt die Mischung bei Raumtemperatur über Nacht rühfen. Dann wird die Mischung auf 0 °C gekühlt, und es werden tropfenweise 8 ml Salzsäure (1 M) zugegeben. Die Mischung wird 1 h gerührt, dann werden 400 ml Wasser zugegeben. Die organische Phase wird abgetrennt und mit Wasser gewaschen. Die wässrige Phase wird mit Ethylacetat extrahiert. Die organischen Phasen werden vereint und unter verringertem Druck eingeengt. Das Rohprodukt wird durch Flash-Chromatographie (Heptan/Dichlormethan 10:1) aufgereinigt. Ausbeute: 24.0 g (75.9 mmol; 89 %).

**Stufe 4 (symmetrisch): Verbindung 1**

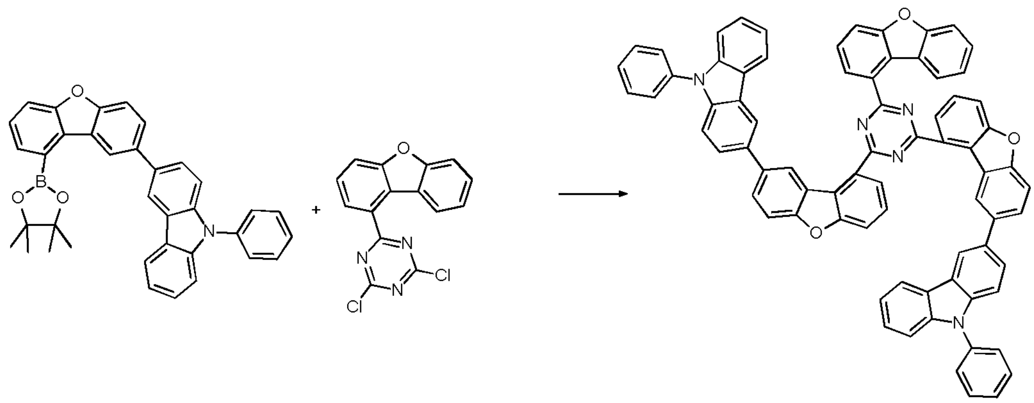

24 g (75.9 mmol) 2,4-Dichlor-6-dibenzofuran-1-yl-[1,3,5]triazin, 80.3 g (150 mmol) 9-Phenyl-3-[9-(4,4,5,5-tetramethyl-[1,3,2]dioxaborolan-2-yl)-dibenzofuran-2-yl]-9H-carbazol, 2.2 g (1.9 mmol) Tetrakis(triphenylphosphin)palladium(0) und 15.7 g (113.9 mmol) Kaliumcarbonat werden in 500 mL Wasser/THF (1:3) gemischt und über Nacht bei 70 °C gerührt. Anschließend wird die Reaktionsmischung auf Raumtemperatur kommen gelassen, gibt Ethylacetat zu und trennt die Phasen. Die organische Phase wird mit Wasser gewaschen, und die wässrige Phase mit Ethylacetat extrahiert. Die vereinten organischen Phasen werden unter verringertem Druck eingeengt und mit Flash-Chromatographie (Heptan/THF 10:1) gereinigt. Das Produkt wird durch Umkristallisation aus Heptan/ Toluol aufgereinigt. Die weitere Aufreinigung erfolgt mittels Sublimation (430 °C, $10^{-4}$ mbar). Ausbeute: 22.6 g (21.28 mmol; 28%).

Die folgenden Verbindungen können analog synthetisiert werden:

| Bsp. | Edukt 1 | Edukt 2 | Produkt |
|---|---|---|---|
| 2 | BB-2b | BB-3a | 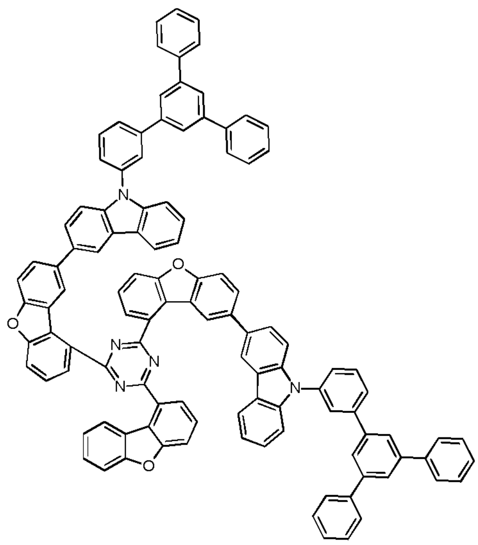 |
| 3 | BB-2c | BB-3a | 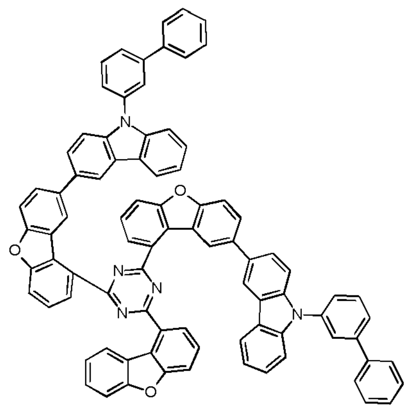 |

(fortgesetzt)

| Bsp. | Edukt 1 | Edukt 2 | Produkt |
|---|---|---|---|
| 4 | BB-2d | BB-3a | 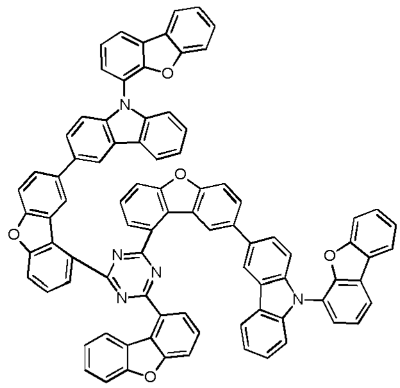 |
| 5 | BB-2e | BB-3a | 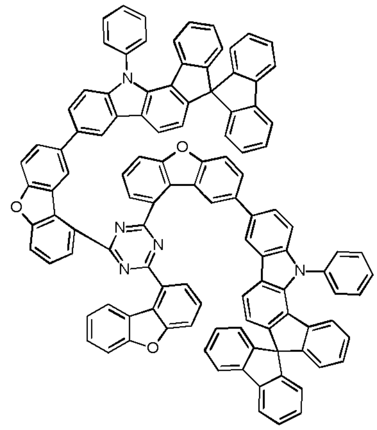 |
| 6 | BB-2a | 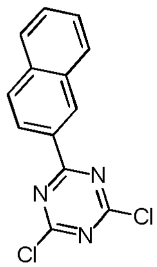 CAS 112719-97-8 | 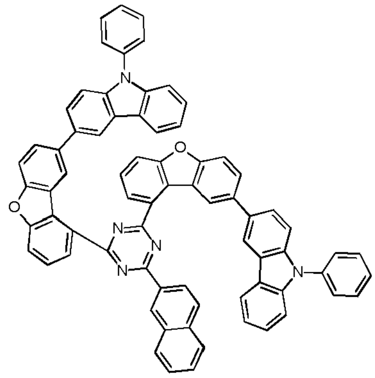 |

(fortgesetzt)

| Bsp. | Edukt 1 | Edukt 2 | Produkt |
|---|---|---|---|
| 7 | BB-2b | 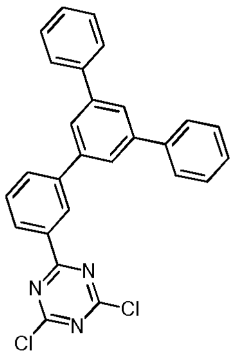 CAS 1845707-19-8 | 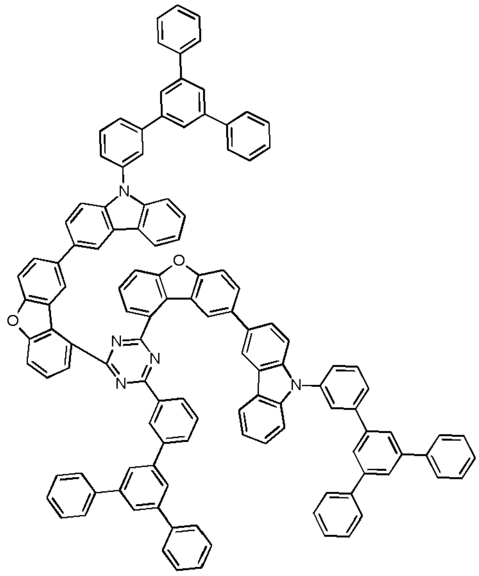 |
| 8 | BB-2c | 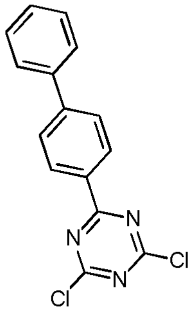 CAS 10202-45-6 | 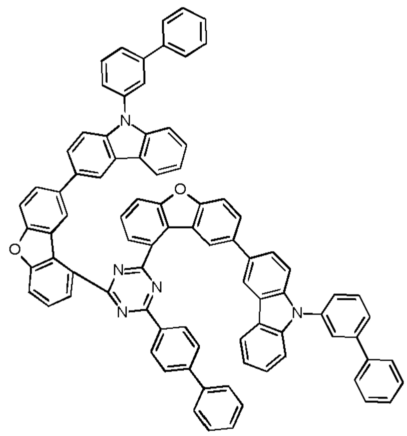 |
| 9 | BB-2b | 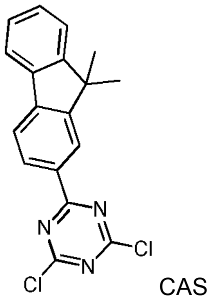 CAS 1644054-61-4 | 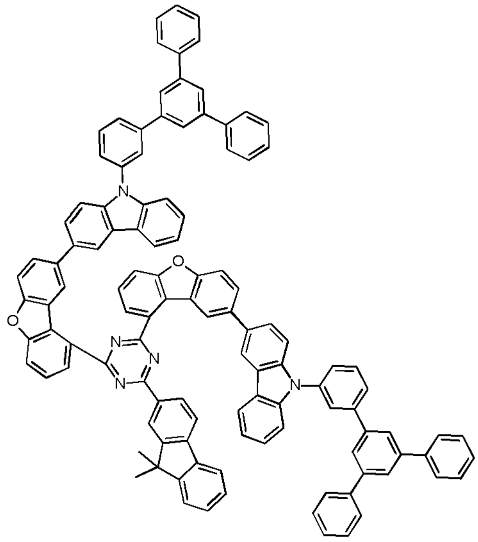 |

(fortgesetzt)

| Bsp. | Edukt 1 | Edukt 2 | Produkt |
|---|---|---|---|
| 10 | BB-2a | 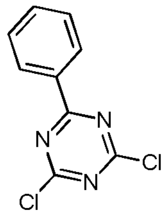 CAS 1700-02-3 | 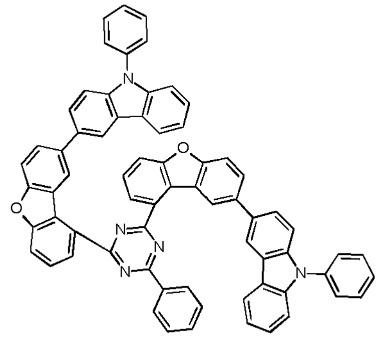 |
| 11 | BB-2f | 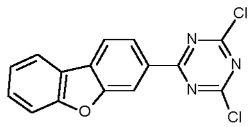 CAS 2102042-41-9 | 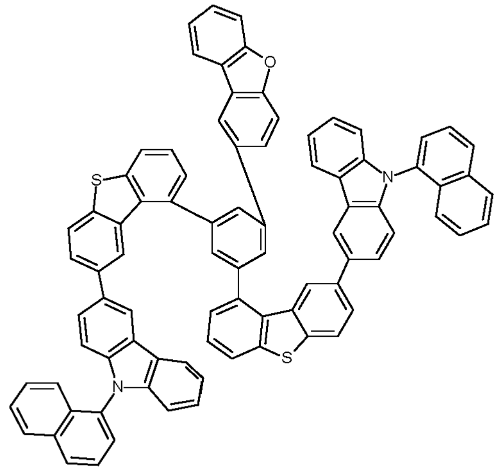 |
| 12 | BB-2g | 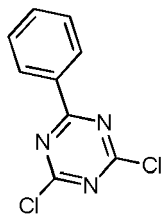 | 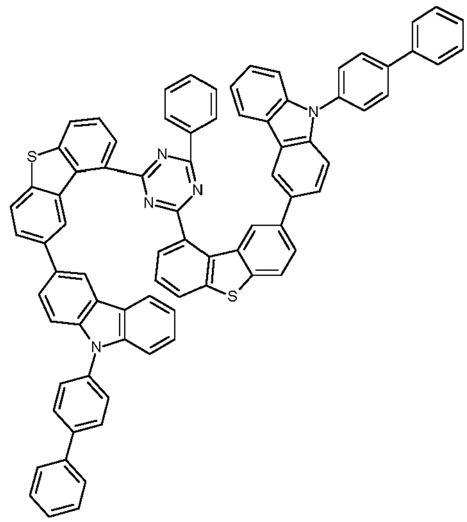 |

(fortgesetzt)

| Bsp. | Edukt 1 | Edukt 2 | Produkt |
|---|---|---|---|
| 13 | BB-2h | 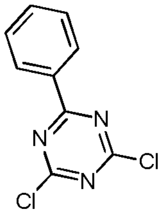 | 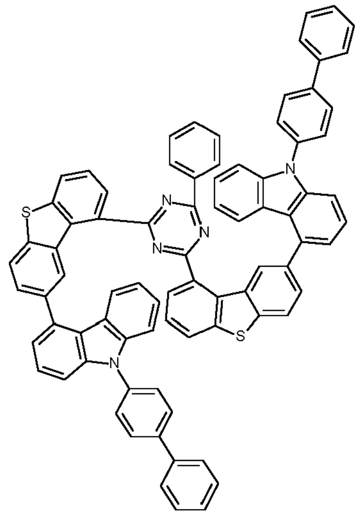 |
| 14 | BB-2i | BB-3a | 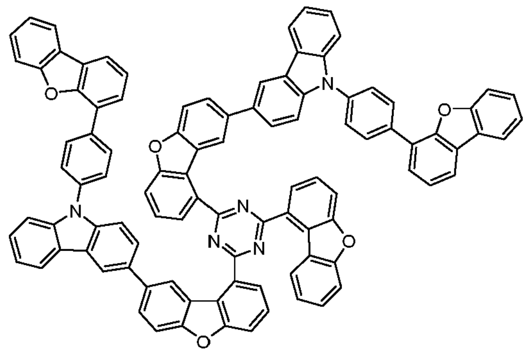 |
| 15 | BB-2j | BB-3a | 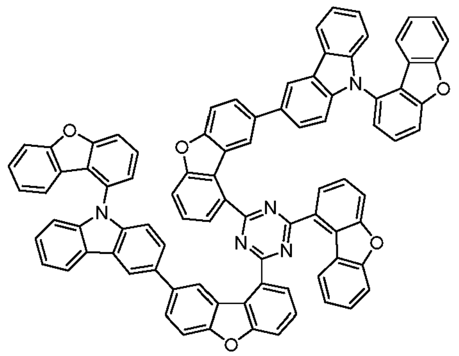 |

**Stufe 5 (asymmetrisch): BB-4a**

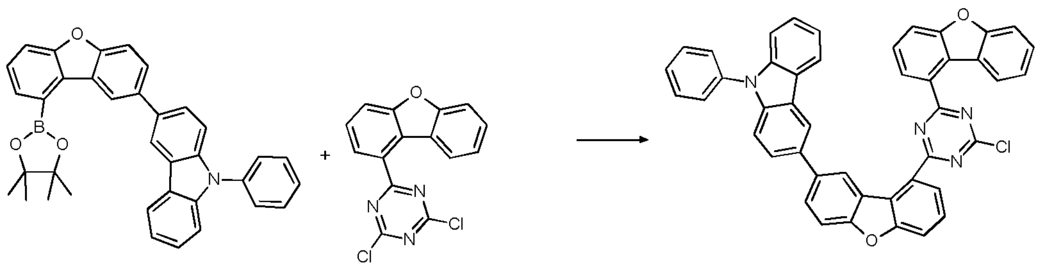

24 g (75.9 mmol) 2,4-Dichlor-6-dibenzofuran-1-yl-[1,3,5]triazin, 36.6 g (68.3 mmol) 9-Phenyl-3-[9-(4,4,5,5-tetramethyl-[1,3,2]dioxaborolan-2-yl)-dibenzofuran-2-yl]-9H-carbazol, 2.2 g (1.9 mmol) Tetrakis(triphenylphosphin)palladium(0) und 15.7 g (113.9 mmol) Kaliumcarbonat werden in 500 mL Wasser/THF (1:3) gemischt und über Nacht bei 70 °C gerührt. Man lässt die Mischung auf Raumtemperatur kommen, gibt Ethylacetat zu und trennt die Phasen. Die organische Phase wird mit Wasser gewaschen und die wässrige Phase mit Ethylacetat extrahiert. Die vereinten organischen Phasen werden unter verringertem Druck eingeengt und durch Flash-Chromatographie (Heptan/THF 10:1) aufgereinigt. Ausbeute: 30 g (43.5 mmol; 58%).

Die folgenden Verbindungen können analog synthetisiert werden:

| Bsp. | Edukt 1 | Edukt 2 | Produkt |
|---|---|---|---|
| BB-4b | BB-2a | 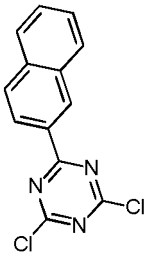 CAS 112719-97-8 | 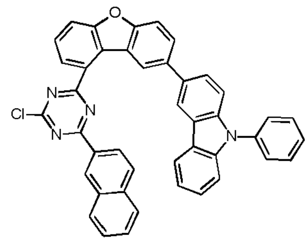 |
| BB-4c | BB-2a | 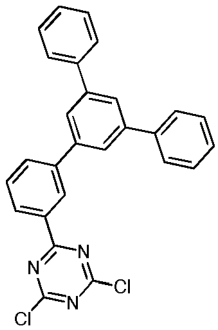 CAS 1845707-19-8 | 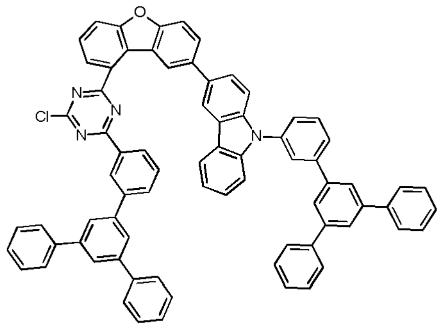 |
| BB-4d | BB-2a | 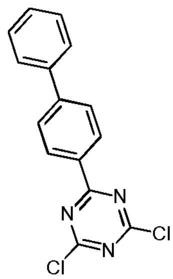 CAS 10202-45-6 | 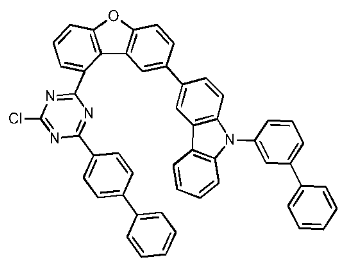 |

**Stufe 6 (asymmetrisch): Verbindung 1.1**

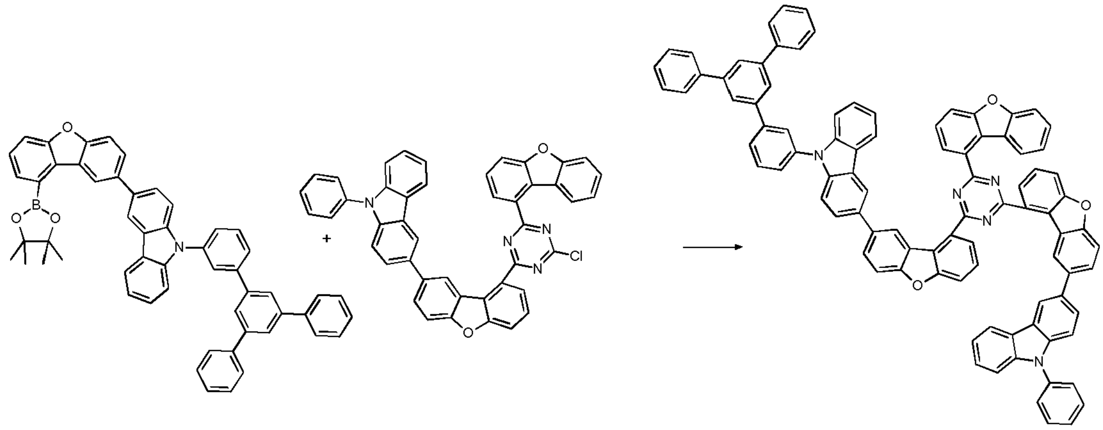

29 g (42.1 mmol) 3-[9-(4-Chlor-6-dibenzofuran-1-yl-[1,3,5]triazin-2-yl)-dibenzofuran-2-yl]-9-phenyl-9H-carbazol, 33.6 g (44 mmol) BB-2b, 1.2 g (1.1 mmol) Tetrakis(triphenylphosphin)palladium(0) und 8.7 g (63.1 mmol) Kaliumcarbonat werden in 500 mL Wasser/THF (1:3) gemischt und über Nacht bei 70 °C gerührt. Man lässt die Reaktionsmischung auf Raumtemperatur kommen, gibt Ethylacetat zu und trennt die Phasen. Die organische Phase wird mit Wasser gewaschen, und die wässrige Phase mit Ethylacetat extrahiert. Die vereinten organischen Phasen werden unter verringertem Druck eingeengt. Das Produkt wird durch Flash-Chromatographie (Heptan/THF 10:1) und dann durch Umkristallisation (Heptan/Toluol) aufgereinigt. Ausbeute: 20.6 g (16.0mmol; 38%).

Analog können die folgenden Verbindungen synthetisiert werden:

| Bsp. | Edukt 1 | Edukt 2 | Produkt |
|---|---|---|---|
| 1.2 | BB-4b | BB-2b | 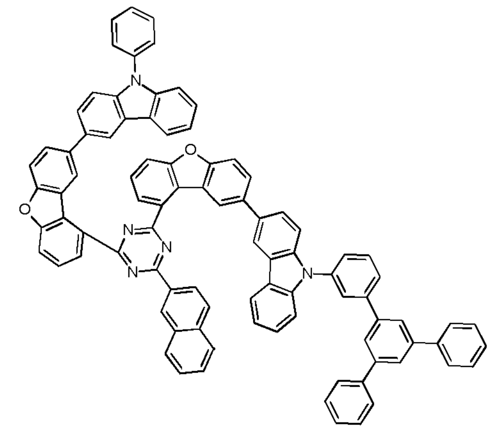 |
| 1.3 | BB-4c | BB-2c | 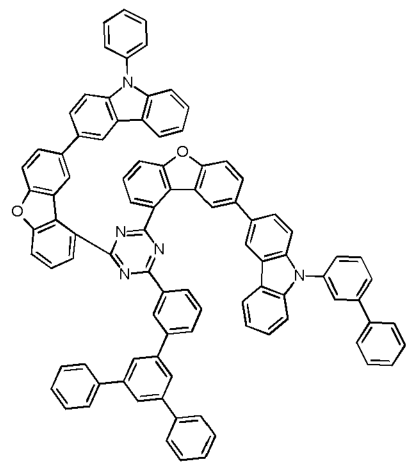 |

(fortgesetzt)

| Bsp. | Edukt 1 | Edukt 2 | Produkt |
|---|---|---|---|
| 1.4 | BB-4d | BB-2d | 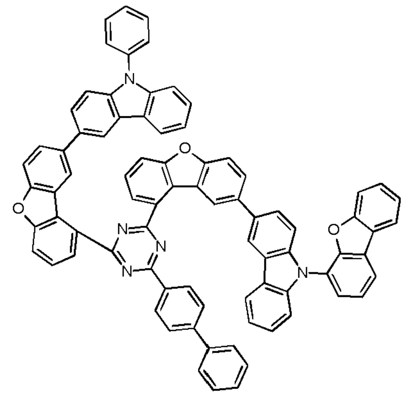 |
| 1.5 | BB-4a | BB-2e | 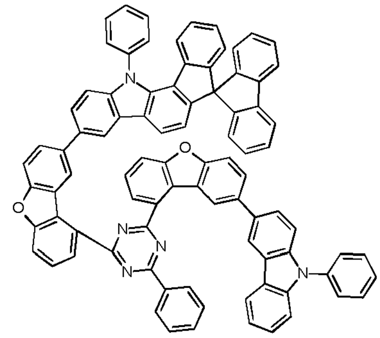 |
| 1.6 | BB-4a | BB-2b | 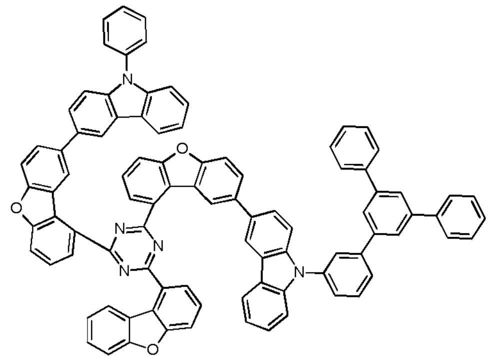 |
| 1.7 | BB-4a | BB-2c | 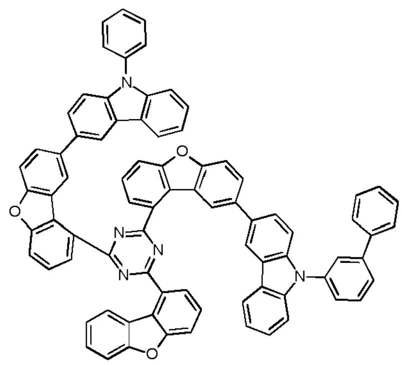 |

**Devicebeispiele aus Lösung prozessiert: Herstellung der OLEDs**

Die Herstellung von aufgedampften OLEDs ist in der Literatur vielfach beschrieben, z. B. in WO 2004/058911. Die Herstellung lösungsbasierter OLEDs ist z. B. in WO 2004/037887 und WO 2010/097155 wiedergegeben. Bei den

folgenden Beispielen werden beide Herstellungsverfahren kombiniert, so dass bis einschließlich der Emissionsschicht aus Lösung prozessiert wird und die darauffolgenden Schichten (Lochblockierschicht/Elektronentransportschicht) im Vakuum aufgedampft werden. Die vorbeschriebenen allgemeinen Verfahren werden dafür wie folgt auf die hier beschriebenen Gegebenheiten (Schichtdickenvariation, Materialien) angepasst und kombiniert.

Der Aufbau der verwendeten OLEDs ist wie folgt:

- Substrat,
- ITO (50 nm),
- Buffer (20 nm),
- Lochtransportschicht (HTL, 20 nm),
- Emissionsschicht (EML, 50 nm),
- Elektronentransportschicht (ETL, 20 nm),
- Elektroneninjektionsschicht (EIL, 3 nm)
- Al Kathode (100 nm).

Als Substrat werden Glasplättchen, die mit strukturiertem ITO (Indium-Zinn-Oxid) der Dicke 50 nm beschichtet sind, verwendet. Zur besseren Prozessierung werden diese mit dem Buffer PEDOT:PSS (Poly(3,4-ethylendioxy-2,5-thiophen) : Polystyrolsulfonat) von Heraeus Precious Metals GmbH & Co. KG beschichtet. Das Aufschleudern erfolgt an Luft aus Wasser. Die Schicht wird anschließend für 10 Minuten bei 180 °C ausgeheizt. Auf die so beschichteten Glasplättchen werden die Lochtransport- sowie die Emissionsschicht aufgebracht. Bei der Lochtransportschicht handelt es sich um das Polymer der in Tabelle 1 gezeigten Struktur, das gemäß WO 2010/097155 synthetisiert werden kann. Das Polymer wird in Toluol gelöst, so dass die Lösung typischerweise einen Feststoffgehalt von ca. 5 g/l besitzt, wenn, wie hier, die für ein Device typische Schichtdicke von 20 nm mittels Spincoating erzielt werden soll. Die Schichten werden in einer Inertgasatmosphäre, im vorliegenden Fall Stickstoff, aufgeschleudert und 30 min bei 220 °C ausgeheizt.

Die Emissionsschicht setzt sich immer aus mindestens einem Matrixmaterial (Hostmaterial, Wirtsmaterial) und einem emittierenden Dotierstoff (Dotand, Emitter) zusammen. Eine Angabe wie H1 (92%) : D1 (8%) bedeutet hierbei, dass das Material H1 in einem Gewichtsanteil von 92% und der Dotand D1 in einem Gewichtsanteil von 8% in der Emissionsschicht vorliegt. Die Mischung für die Emissionsschicht wird in Toluol gelöst. Der typische Feststoffgehalt solcher Lösungen liegt bei ca. 14 g/l, wenn, wie hier, die für ein Device typische Schichtdicke von 50 nm mittels Spincoating erzielt werden soll. Die Schichten werden in einer Inertgasatmosphäre, im vorliegenden Fall Stickstoff, aufgeschleudert und 10 Minuten ausgeheizt. Die verwendeten Materialien sind in Tabelle 1 gezeigt. Die jeweilige Zusammensetzung der Emissionsschicht, die EML Schichtdicke, sowie deren Ausheiztemperatur sind in Tabelle 2 dargelegt.

Die Materialien für die Elektronentransportschicht sowie für die Kathode werden in einer Vakuumkammer thermisch aufgedampft. Dabei kann z. B. die Elektronentransportschicht aus mehr als einem Material bestehen, die einander durch Co-Verdampfung in einem bestimmten Volumenanteil beigemischt werden. Eine Angabe wie ETM:EIM (50%:50%) bedeutet hierbei, dass die Materialien ETM und EIM in einem Volumenanteil von je 50% in der Schicht vorliegen. Im vorliegenden Fall besteht die Elektronentransportschicht aus dem Material ETM mit einer Schichtdicke von 20 nm. Die Elektroneninjektionsschicht wird aus 3 nm des Materials EIM gebildet. Beide Materialien sind in Tabelle 1 abgebildet. Den Abschluss bildet eine Kathodenschicht aus Aluminium mit einer Schichtdicke von 100 nm.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 $cd/m^2$ aufgenommen und daraus die CIE 1931 x und y Farbkoordinaten berechnet. Die Angabe EQE @ 1000 $cd/m^2$ bezeichnet die externe Quanteneffizienz bei einer Betriebsleuchtdichte von 1000 $cd/m^2$. Die Lebensdauer LT95 @ 4000 $cd/m^2$ ist die Zeit, die vergeht, bis die Starthelligkeit von 4000 $cd/m^2$ um 5% auf 3800 $cd/m^2$ gesunken ist. Die Daten für die verschiedenen OLEDs sind in Tabelle 3 zusammengefasst.

| Tabelle 1: Strukturen der verwendeten Materialien | |
|---|---|
| 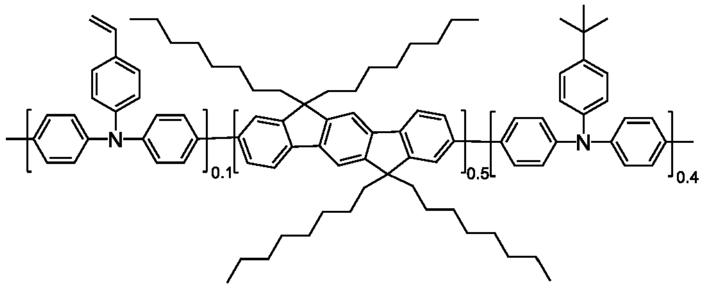 | |
| HTL-A | |
| 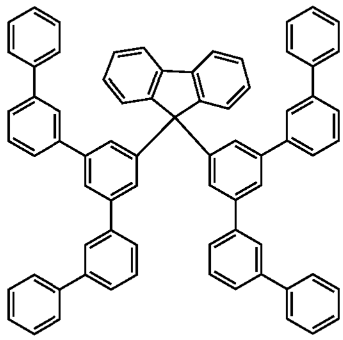 | 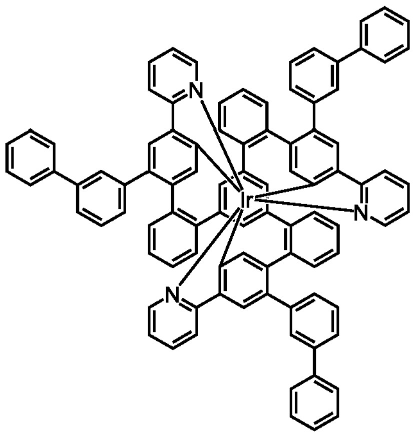 |
| Host 2 | TEG |
| 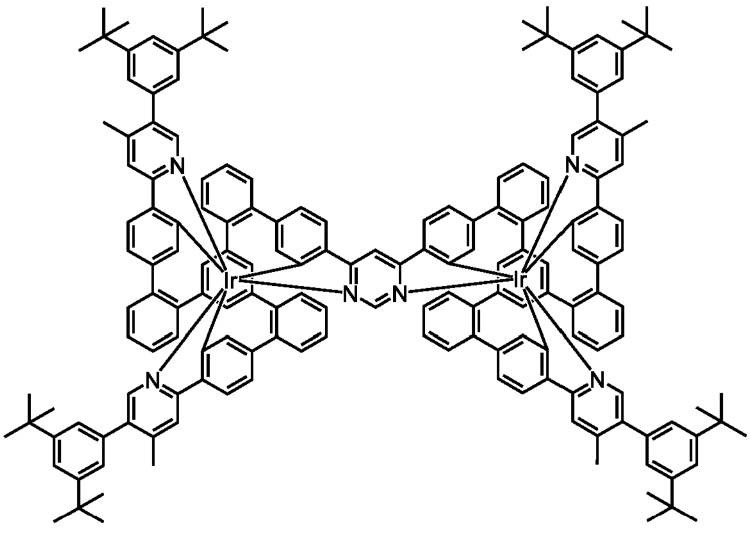 | |
| TER | |
| 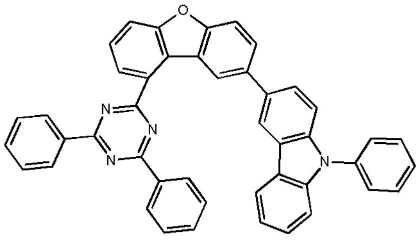 | 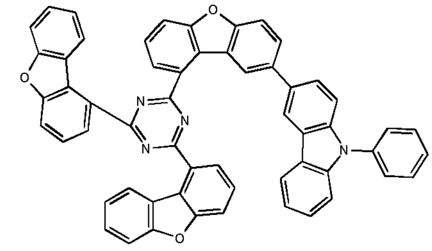 |

(fortgesetzt)

| Tabelle 1: Strukturen der verwendeten Materialien | |
|---|---|
| SdT1 | SdT2 |
| 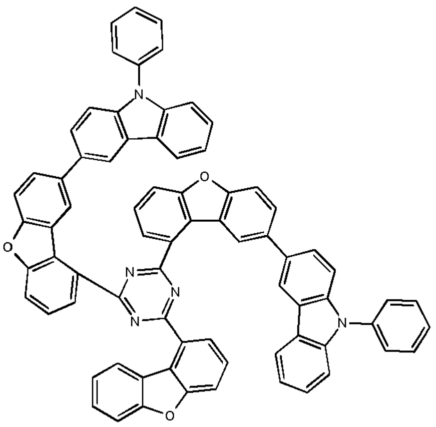 | 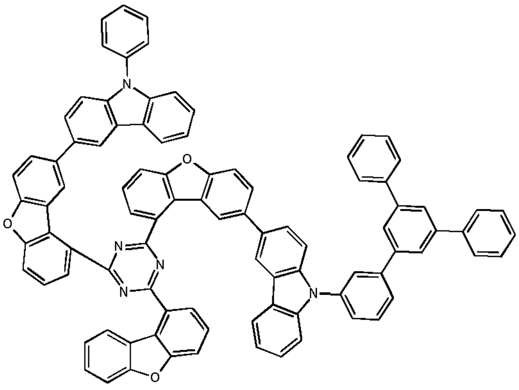 |
| EG1 | EG2 |
| 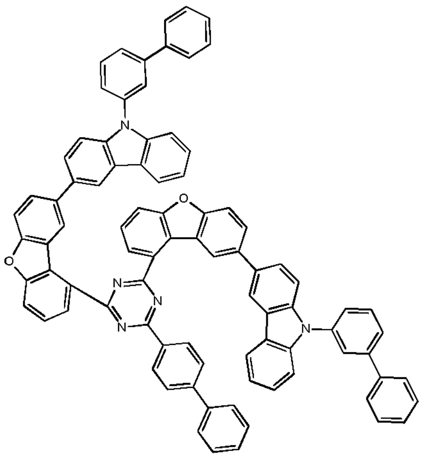 | 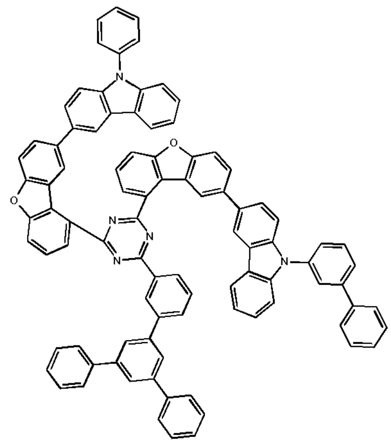 |
| EG3 | EG4 |
| 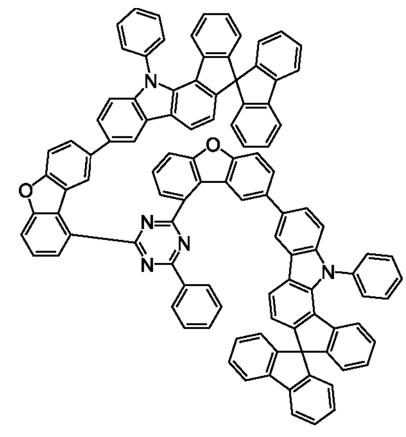 | 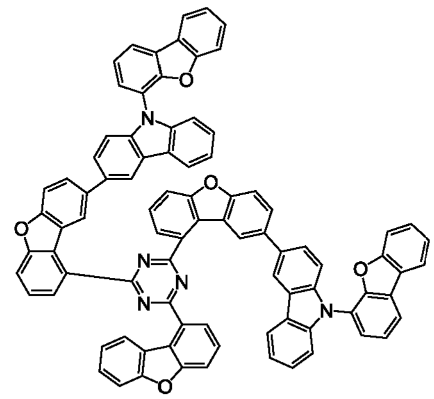 |
| EG5 | EG6 |

(fortgesetzt)

| **Tabelle 1: Strukturen der verwendeten Materialien** | |
|---|---|
| 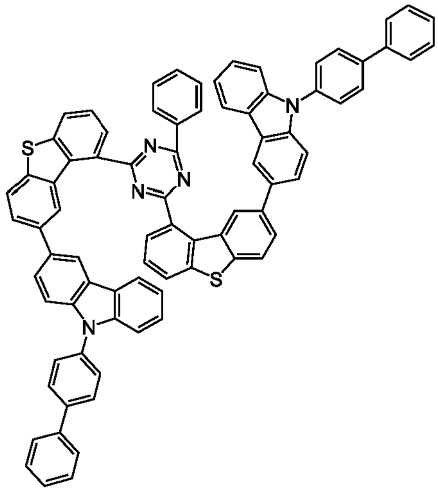 | 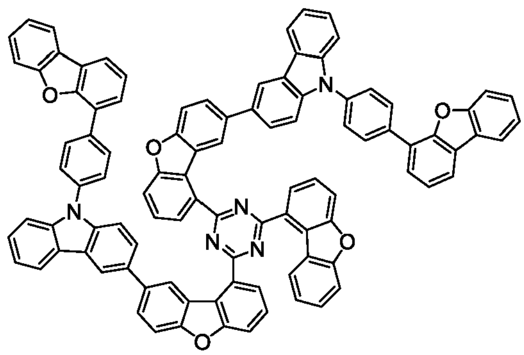 |
| EG7 | EG8 |
| 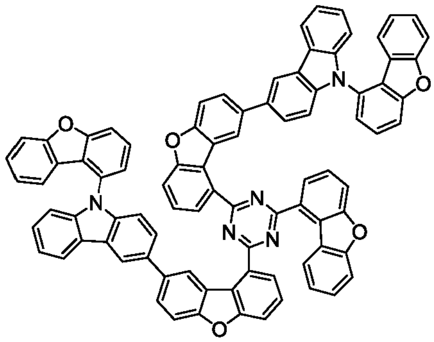 | |
| EG9 | |
| 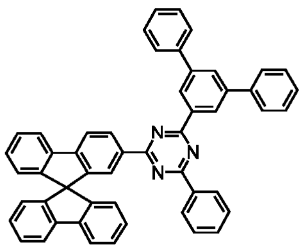 | 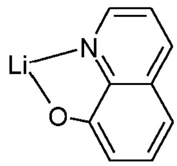 |
| ETM | EIM |

| Tabelle 2: Detaillierter Device-Aufbau der löslich prozessierten OLEDs | | | | | | |
|---|---|---|---|---|---|---|
| Bsp. | HIL Dicke [nm] | HTL Dicke [nm] | EML Dicke [nm] | EML Ausheiztemperatur [°C] | ETL Dicke [nm] | EIL Dicke [nm] |
| V1 | PEDOT: PSS 80nm | HTL-A 20nm | SdT1:Host2:TEG:TER (40%:35%:17%:8%) 50nm | 140 | ETM 20nm | EIM 3nm |
| V2 | PEDOT: PSS 80nm | HTL-A 20nm | SdT2:Host2:TEG:TER (40%:35%:17%:8%) 50nm | 160 | ETM 20nm | EIM 3nm |

(fortgesetzt)

| Tabelle 2: Detaillierter Device-Aufbau der löslich prozessierten OLEDs | | | | | | |
|---|---|---|---|---|---|---|
| Bsp. | HIL Dicke [nm] | HTL Dicke [nm] | EML Dicke [nm] | EML Ausheiztemperatur [°C] | ETL Dicke [nm] | EIL Dicke [nm] |
| E3 | PEDOT: PSS 80nm | HTL-A 20nm | EG1:Host2:TEG:TER (40%:35%:17%:8%) 50nm | 160 | ETM 20nm | EIM 3nm |
| E4 | PEDOT: PSS 80nm | HTL-A 20nm | EG2:Host2:TEG:TER (40%:35%:17%:8%) 50nm | 160 | ETM 20nm | EIM 3nm |
| E5 | PEDOT: PSS 80nm | HTL-A 20nm | EG3:Host2:TEG:TER (40%:35%:17%:8%) 50nm | 160 | ETM 20nm | EIM 3nm |
| E6 | PEDOT: PSS 80nm | HTL-A 20nm | EG4:Host2:TEG:TER (40%:35%:17%:8%) 50nm | 160 | ETM 20nm | EIM 3nm |
| E7 | PEDOT: PSS 80nm | HTL-A 20nm | EG5:Host2:TEG:TER (40%:35%:17%:8%) 50nm | 160 | ETM 20nm | EIM 3nm |
| E8 | PEDOT: PSS 80nm | HTL-A 20nm | EG6:Host2:TEG:TER (40%:35%:17%:8%) 50nm | 160 | ETM 20nm | EIM 3nm |
| E9 | PEDOT: PSS 80nm | HTL-A 20nm | EG7:Host2:TEG:TER (40%:35%:17%:8%) 50nm | 160 | ETM 20nm | EIM 3nm |
| E10 | PEDOT: PSS 80nm | HTL-A 20nm | EG8:Host2:TEG:TER (40%:35%:17%:8%) 50nm | 160 | ETM 20nm | EIM 3nm |
| E11 | PEDOT: PSS 80nm | HTL-A 20nm | EG9:Host2:TEG:TER (40%:35%:17%:8%) 50nm | 160 | ETM 20nm | EIM 3nm |

| Tabelle 3: Daten der löslich prozessierten OLEDs | | | | |
|---|---|---|---|---|
| Bsp. | CIE x/y @1000 cd/m$^2$ | | EQE @1000cd/m$^2$ (%) | LT95 @4000cd/m$^2$ (hrs) |
| V1 | 0.67 | 0.33 | 12.3 | 75 |
| V2 | 0.67 | 0.33 | 12.8 | 70 |
| E3 | 0.67 | 0.33 | 13.4 | 180 |
| E4 | 0.67 | 0.33 | 13.1 | 200 |
| E5 | 0.67 | 0.33 | 13.3 | 170 |
| E6 | 0.67 | 0.33 | 13.2 | 190 |
| E7 | 0.67 | 0.33 | 13.3 | 220 |
| E8 | 0.67 | 0.33 | 13.4 | 180 |
| E9 | 0.67 | 0.33 | 13.0 | 140 |
| E10 | 0.67 | 0.33 | 13.5 | 190 |

(fortgesetzt)

| Tabelle 3: Daten der löslich prozessierten OLEDs | | | | |
|---|---|---|---|---|
| Bsp. | CIE x/y @1000 cd/m$^2$ | | EQE @1000cd/m$^2$ (%) | LT95 @4000cd/m$^2$ (hrs) |
| E11 | 0.67 | 0.33 | 13.2 | 210 |

Die Ergebnisse in Tabelle 3 zeigen, dass neben einer leicht verbesserten externen Quanteneffizienz eine deutliche Verbesserung der Lebensdauer erzielt werden kann. Die gefundenen Strukturen eignen sich somit als Host für Prozessierung aus Lösung und führen zu hervorragenden Leistungsdaten.

## Patentansprüche

1. Verbindung gemäß Formel (1),

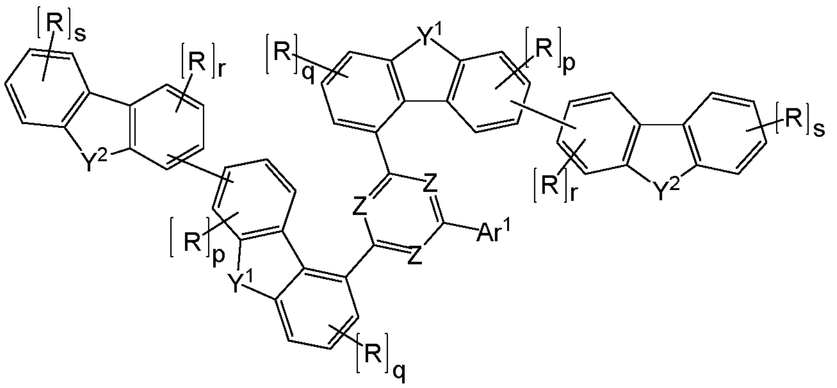

Formel (1)

wobei für die verwendeten Symbole und Indizes gilt:

$Y^1$ ist bei jedem Auftreten gleich oder verschieden O oder S;
$Y^2$ ist bei jedem Auftreten gleich oder verschieden $NAr^2$, O, S oder $CR_2$;
Z ist bei jedem Auftreten gleich oder verschieden CR oder N mit der Maßgabe, dass mindestens zwei Z für N stehen;
$Ar^1$, $Ar^2$ ist bei jedem Auftreten gleich oder verschieden ein aromatisches oder heteroaromatisches Ringsystem mit 5 bis 40 aromatischen Ringatomen, welches mit einem oder mehreren Resten R substiutiert sein kann;
R ist bei jedem Auftreten gleich oder verschieden H, D, F, Cl, Br, I, $N(Ar')_2$, $N(R^1)_2$, OAr', SAr', CN, $NO_2$, $OR^1$, $SR^1$, $COOR^1$, $C(=O)N(R^1)_2$, $Si(R^1)_3$, $B(OR^1)_2$, $C(=O)R^1$, $P(=O)(R^1)_2$, $S(=O)R^1$, $S(=O)_2R^1$, $OSO_2R^1$, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^1$ substituiert sein kann, wobei eine oder mehrere nicht benachbarte $CH_2$-Gruppen durch $Si(R^1)_2$, C=O, $NR^1$, O, S oder $CONR^1$ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste $R^1$ substituiert sein kann; dabei können zwei Reste R auch miteinander ein Ringsystem bilden;
Ar' ist bei jedem Auftreten gleich oder verschieden ein aromatisches oder heteroaromatisches Ringsystem mit 5 bis 40 aromatischen Ringatomen, das durch einen oder mehrere Reste $R^1$ substituiert sein kann; dabei können zwei Reste Ar', welche an dasselbe N-Atom binden, auch durch eine Einfachbindung oder eine Brücke, ausgewählt aus $N(R^1)$, $C(R^1)_2$, O oder S, miteinander verbrückt sein;
$R^1$ ist bei jedem Auftreten gleich oder verschieden H, D, F, Cl, Br, I, $N(R^2)_2$, CN, $NO_2$, $OR^2$, $SR^2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^2$ substituiert sein kann, wobei eine oder mehrere nicht benachbarte $CH_2$-Gruppen durch $Si(R^2)_2$,

C=O, $NR^2$, O, S oder $CONR^2$ ersetzt sein können und wobei ein oder mehrere H-Atome in der Alkyl-, Alkenyl- oder Alkinylgruppe durch D, F, Cl, Br, I oder CN ersetzt sein können, oder ein aromatisches oder heteroaromatisches Ringsystem mit 5 bis 40 aromatischen Ringatomen, das jeweils durch einen oder mehrere Reste $R^2$ substituiert sein kann; dabei können zwei oder mehrere Reste $R^1$ miteinander ein aliphatisches Ringsystem bilden;

$R^2$ ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch F ersetzt sein können;

p, q, r ist bei jedem Auftreten gleich oder verschieden 0, 1, 2 oder 3;

s ist bei jedem Auftreten gleich oder verschieden 0, 1, 2, 3 oder 4.

**2.** Verbindung nach Anspruch 1 gemäß Formel (2),

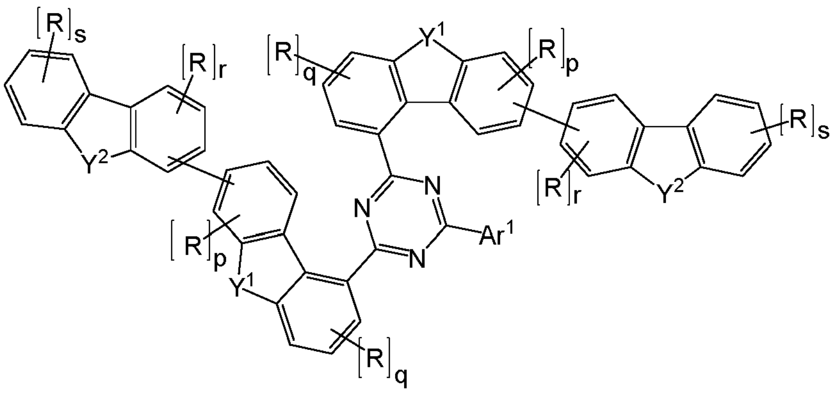

Formel (2)

wobei die Symbole und Indizes die in Anspruch 1 genannten Bedeutungen aufweisen.

**3.** Verbindung nach Anspruch 1 oder 2 gemäß Formel (3),

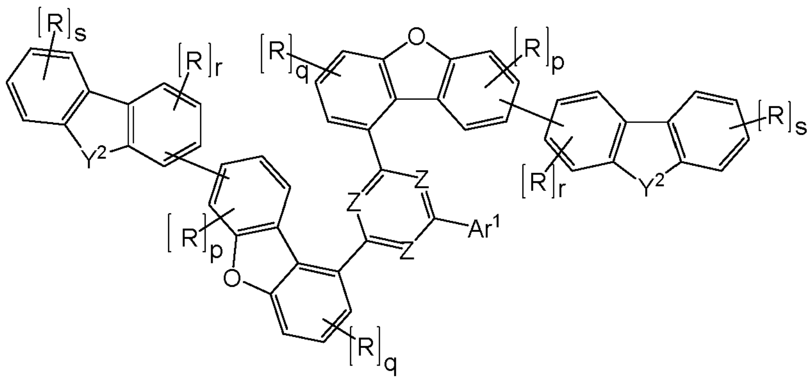

Formel (3)

wobei die Symbole und Indizes die in Anspruch 1 genannten Bedeutungen aufweisen.

**4.** Verbindung nach einem oder mehreren der Ansprüche 1 bis 3 gemäß Formel (4),

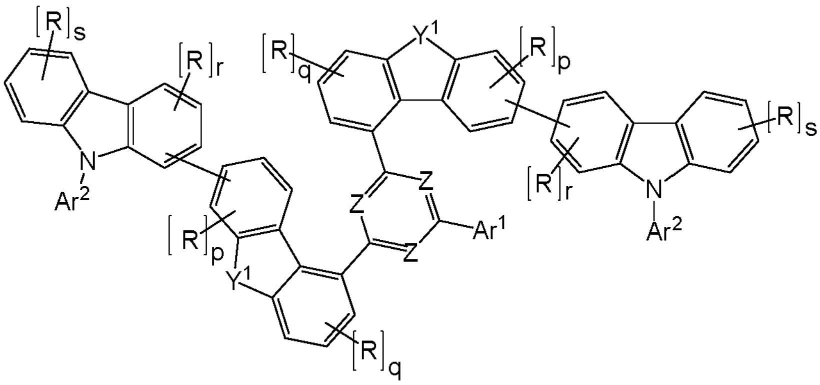

Formel (4)

wobei die Symbole und Indizes die in Anspruch 1 genannten Bedeutungen aufweisen.

**5.** Verbindung nach einem oder mehreren der Ansprüche 1 bis 4, **dadurch gekennzeichnet, dass** $Y^2$ für $NAr^2$ steht und die Carbazolgruppe mindestens zwei benachbarte Reste R aufweist, die miteinander ein Ringsystem bilden, so dass eine Struktur gemäß einer der Formeln (CARB-1) bis (CARB-6) entsteht,

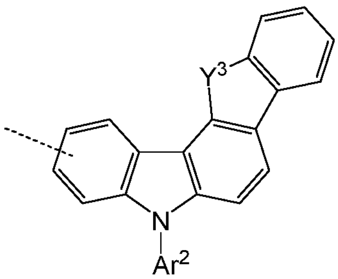

(CARB-1)

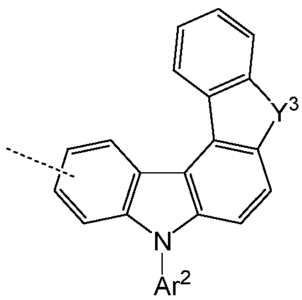

(CARB-2)

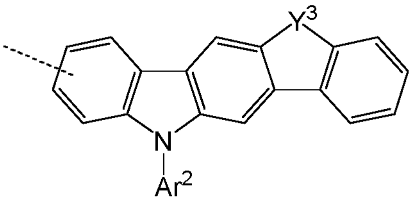

(CARB-3)

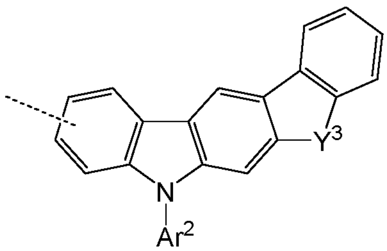

(CARB-4)

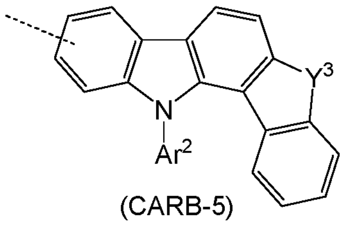

(CARB-5)

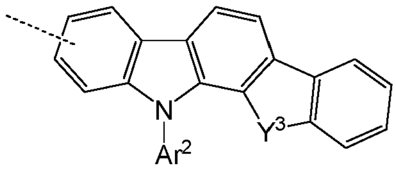

(CARB-6)

wobei $Ar^2$ und $R^1$ die in Anspruch 1 genannten Bedeutungen aufweisen, die Strukturen am Carbazol durch einen oder mehrere Reste R und an der ankondensierten Struktur durch einen oder mehrere Reste $R^1$ substituiert sein können, $Y^3$ für $C(R^1)_2$, $NR^1$, O oder S steht und die Strukturen über die gestrichelte Bindung mit dem Dibenzofuran bzw. Dibenzothiophen verknüpft sind.

**6.** Verbindung nach einem oder mehreren der Ansprüche 1 bis 5 gemäß Formel (5),

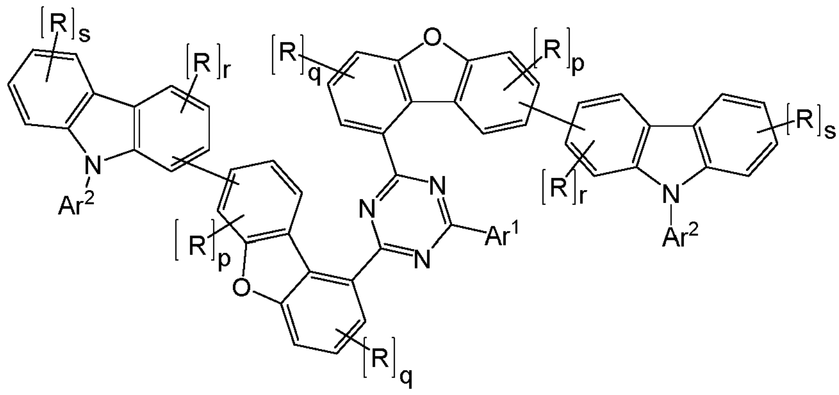

Formel (5)

wobei die Symbole und Indizes die in Anspruch 1 genannten Bedeutungen aufweisen.

**7.** Verbindung nach einem oder mehreren der Ansprüche 1 bis 6 gemäß einer der Formeln (1a) bis (5a),

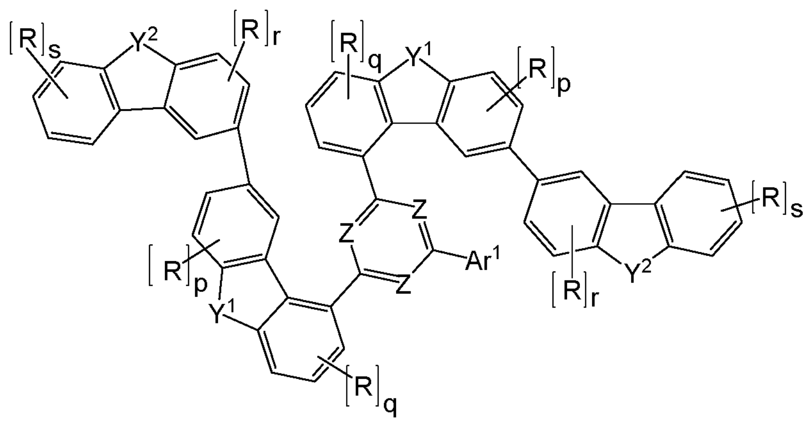

Formel (1a)

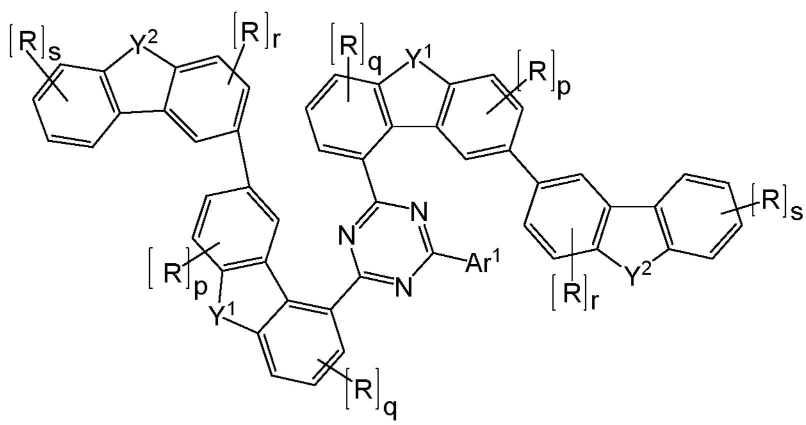

Formel (2a)

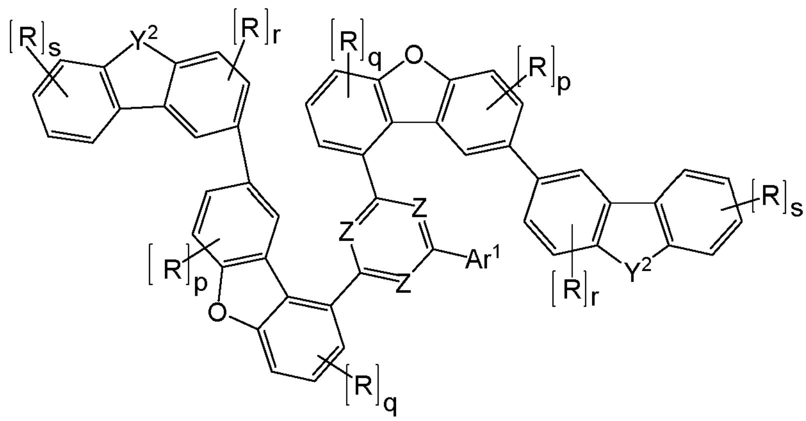

Formel (3a)

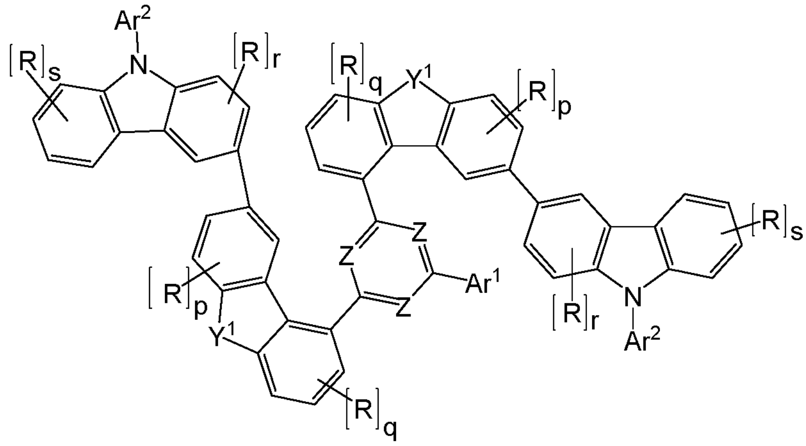

Formel (4a)

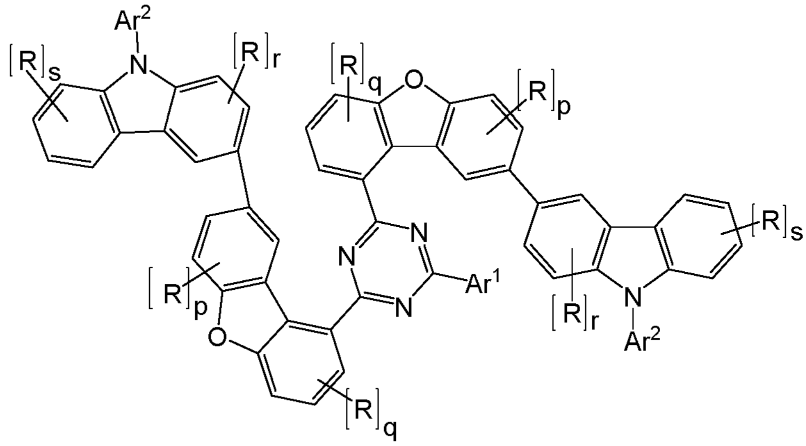

Formel (5a)

wobei die Symbole und Indizes die in Anspruch 1 genannten Bedeutungen aufweisen.

**8.** Verbindung nach einem oder mehreren der Ansprüche 1 bis 7 gemäß Formel (6a),

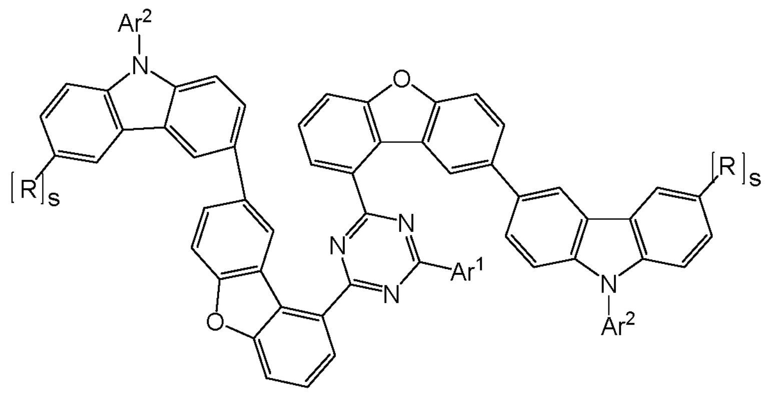

Formel (6a)

wobei die Symbole die in Anspruch 1 genannten Bedeutungen aufweisen und s gleich oder verschieden bei jedem Auftreten 0 oder 1 ist.

**9.** Verbindung nach einem oder mehreren der Ansprüche 1 bis 8, **dadurch gekennzeichnet, dass** $Ar^1$ und $Ar^2$ gleich oder verschieden bei jedem Auftreten jeweils ausgewählt ist aus der Gruppe bestehend aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Phenanthren, Triphenylen oder einer Kombination aus zwei oder drei dieser Gruppen, welche jeweils mit einem oder mehreren Resten R substituiert sein können.

**10.** Verfahren zur Herstellung einer Verbindung nach einem oder mehreren der Ansprüche 1 bis 9 durch Umsetzung eines mit $Ar^1$ funktionalisierten Dichlorpyrimidins oder -triazins mit einer 1-Dibenzofuran-boronsäure bzw. deren Ester oder einer 1-Dibenzothiophenboronsäure bzw. deren Ester in einer Suzuki-Kupplung.

**11.** Formulierung, enthaltend mindestens eine Verbindung nach einem oder mehreren der Ansprüche 1 bis 9 und mindestens eine weitere Verbindung und/oder ein Lösemittel.

**12.** Verwendung einer Verbindung nach einem oder mehreren der Ansprüche 1 bis 9 oder einer Formulierung nach Anspruch 11 in einer elektronischen Vorrichtung.

**13.** Elektronische Vorrichtung enthaltend mindestens eine Verbindung nach einem oder mehreren der Ansprüche 1 bis 9 oder eine Formulierung nach Anspruch 11.

**14.** Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 9 in einer emittierenden Schicht als Matrixmaterial für phosphoreszierende Emitter oder für Emitter, die TADF (thermally activated delayed fluorescence) zeigen, und/oder in einer Elektronentransportschicht und/oder in einer Lochblockierschicht eingesetzt wird.

**15.** Elektronische Vorrichtung nach Anspruch 14, **dadurch gekennzeichnet, dass** die Verbindung nach einem oder mehreren der Ansprüche 1 bis 9 in einer emittierenden Schicht als Matrixmaterial für einen phosphoreszierenden Emitter eingesetzt wird, wobei die Verbindung in Kombination mit einem oder mehreren weiteren Matrixmaterialien eingesetzt wird, die ausgewählt sind aus der Gruppe bestehend aus aromatischen Ketonen, aromatischen Phosphinoxiden, aromatischen Sulfoxiden, aromatischen Sulfonen, Triarylaminen, Carbazolderivaten, Indolocarbazolderivaten, Indenocarbazolderivaten, Azacarbazolderivaten, bipolaren Matrixmaterialien, Azaborolen, Boronestern, Triazinderivaten, Zinkkomplexen, Diazasilol- bzw. Tetraazasilol-Derivaten, Diazaphosphol-Derivaten, verbrückten Carbazol-Derivaten, Triphenylenderivaten, Dibenzofuranderivaten, Dibenzothiophenderivaten, Wide Bandgap Materialien und/oder phosphoreszierenden Verbindungen, welche kürzerwellig als der eigentliche Emitter emittieren.

## Claims

**1.** Compound of formula (1)

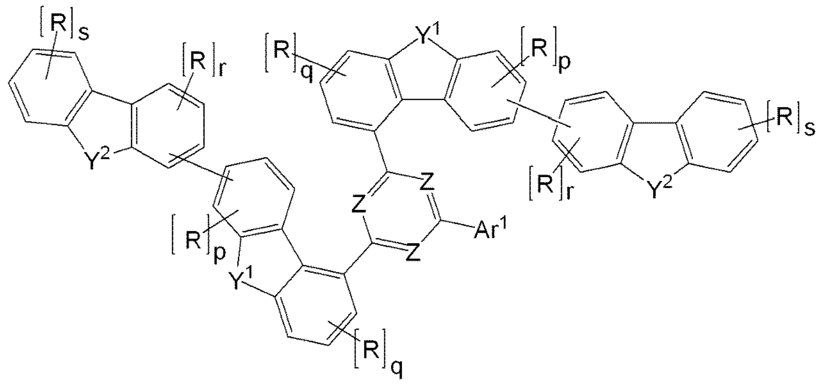

Formula (1)

where the symbols and indices used are as follows:

$Y^1$ is the same or different at each instance and is O or S;
$Y^2$ is the same or different at each instance and is $NAr^2$, O, S or $CR_2$;
Z is the same or different at each instance and is CR or N, with the proviso that at least two Z are N;
$Ar^1$, $Ar^2$ is the same or different at each instance and is an aromatic or heteroaromatic ring system which has 5 to 40 aromatic ring atoms and may be substituted by one or more R radicals;
R is the same or different at each instance and is H, D, F, Cl, Br, I, $N(Ar')_2$, $N(R^2)_2$, OAr', SAr', CN, $NO_2$, $OR^1$, $SR^1$, $COOR^1$, $C(=O)N(R^1)_2$, $Si(R^1)_3$, $B(OR^1)_2$, $C(=O)R^1$, $P(=O)(R^1)_2$, $S(=O)R^2$, $S(=O)_2R^1$, $OSO_2R^1$, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more $R^1$ radicals, where one or more nonadjacent $CH_2$ groups may be replaced by $Si(R^1)_2$, C=O, $NR^1$, O, S or $CONR^1$, or an aromatic or heteroaromatic ring system which has 5 to 60 aromatic ring atoms, preferably 5 to 40 aromatic ring atoms, and may be substituted in each case by one or more $R^1$ radicals; at the same time, two R radicals together may also form a ring system;
Ar' is the same or different at each instance and is an aromatic or heteroaromatic ring system which has 5 to 40 aromatic ring atoms and may be substituted by one or more $R^1$ radicals; at the same time, two Ar' radicals bonded to the same nitrogen atom may also be bridged to one another by a single bond or a bridge selected from $N(R^1)$, $C(R^1)_2$, O and S;
$R^1$ is the same or different at each instance and is H, D, F, Cl, Br, I, $N(R^2)_2$, CN, $NO_2$, $OR^2$, $SR^2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may each be substituted by one or more $R^2$ radicals, where one or more nonadjacent $CH_2$ groups may be replaced by $Si(R^2)_2$, C=O, $NR^2$, O, S or $CONR^2$ and where one or more hydrogen atoms in the alkyl, alkenyl or alkynyl group may be replaced by D, F, Cl, Br, I or CN, or an aromatic or heteroaromatic ring system which has 5 to 40 aromatic ring atoms and may be substituted in each case by one or more $R^2$ radicals; at the same time, two or more $R^1$ radicals together may form an aliphatic ring system;
$R^2$ is the same or different at each instance and is H, D, F, CN or an aliphatic, aromatic or heteroaromatic organic radical having 1 to 20 carbon atoms, in which one or more hydrogen atoms may also be replaced by F;
p, q, r is the same or different at each instance and is 0, 1, 2 or 3;
s is the same or different at each instance and is 0, 1, 2, 3 or 4.

**2.** Compound according to Claim 1 of formula (2)

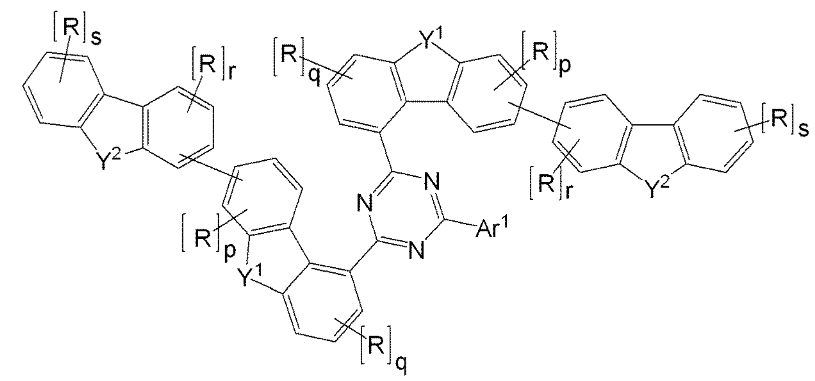

Formula (2)

where the symbols and indices have the definitions given in Claim 1.

**3.** Compound according to Claim 1 or 2 of formula (3)

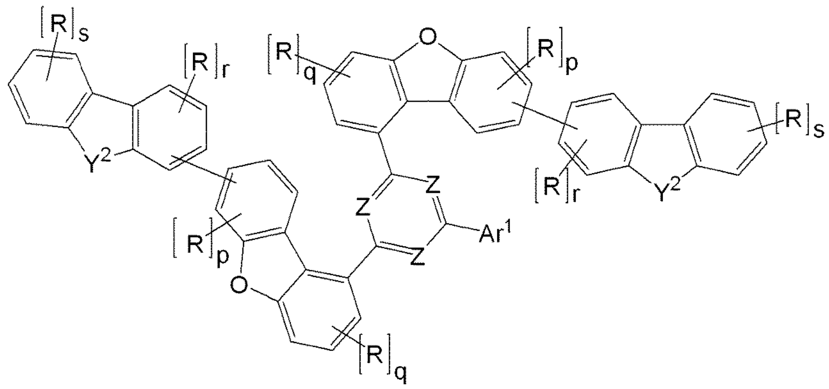

Formula (3)

where the symbols and indices have the definitions given in Claim 1.

**4.** Compound according to one or more of Claims 1 to 3 of formula (4)

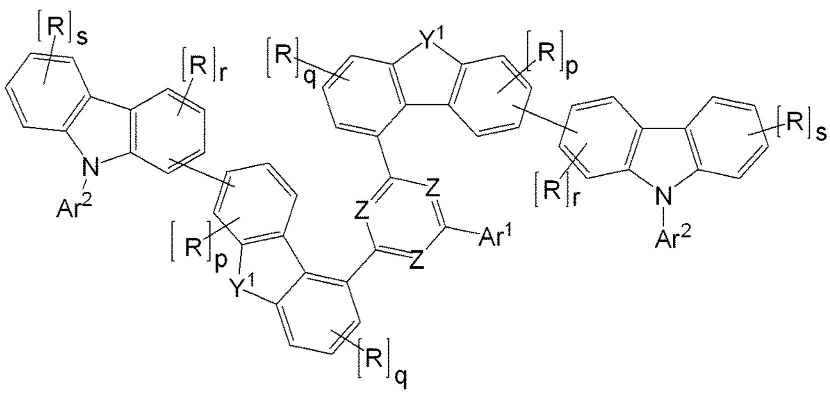

Formula (4)

where the symbols and indices have the definitions given in Claim 1.

**5.** Compound according to one or more of Claims 1 to 4, **characterized in that** $Y^2$ is $NAr^2$ and the carbazole group has at least two adjacent R radicals that form a ring system with one another, so as to form a structure of one of the formulae (CARB-1) to (CARB-6)

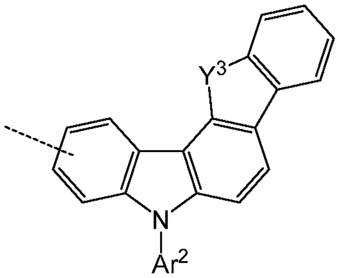

(CARB-1)

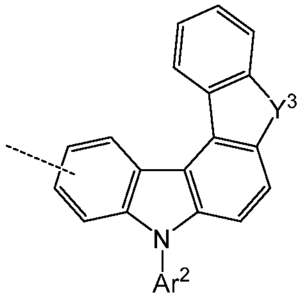

(CARB-2)

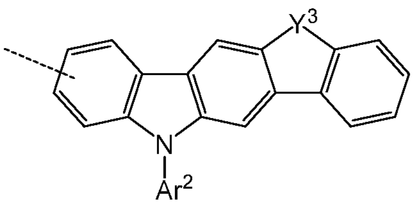

(CARB-3)

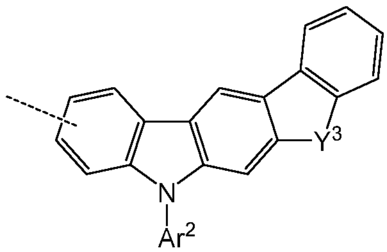

(CARB-4)

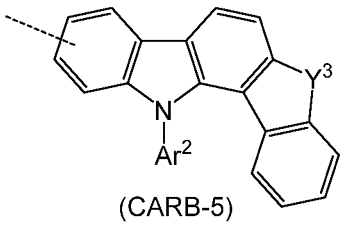

(CARB-5)

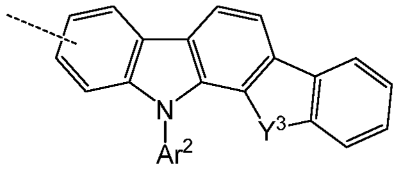

(CARB-6)

where $Ar^2$ and $R^1$ have the definitions given in Claim 1, the structures may be substituted by one or more R radicals on the carbazole and by one or more $R^1$ radicals on the fused-on structure, $Y^3$ is $C(R^1)_2$, $NR^1$, O or S, and the structures are joined to the dibenzofuran or dibenzothiophene via the dotted bond.

**6.** Compound according to one or more of Claims 1 to 5 of formula (5)

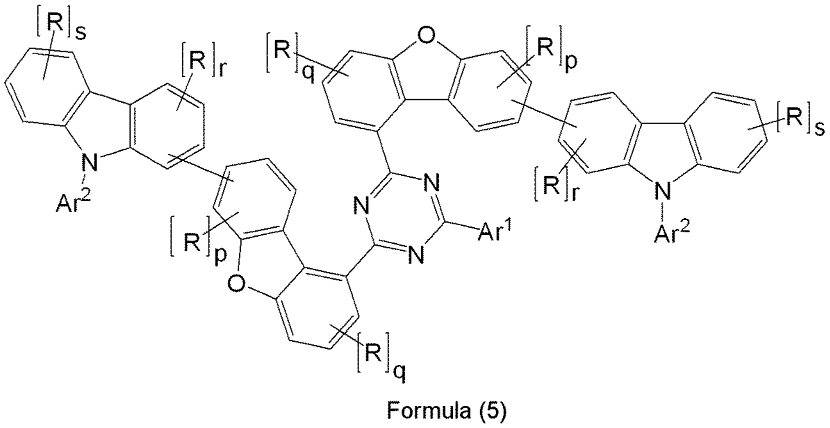

Formula (5)

where the symbols and indices have the definitions given in Claim 1.

**7.** Compound according to one or more of Claims 1 to 6 of one of the formulae (1a) to (5a)

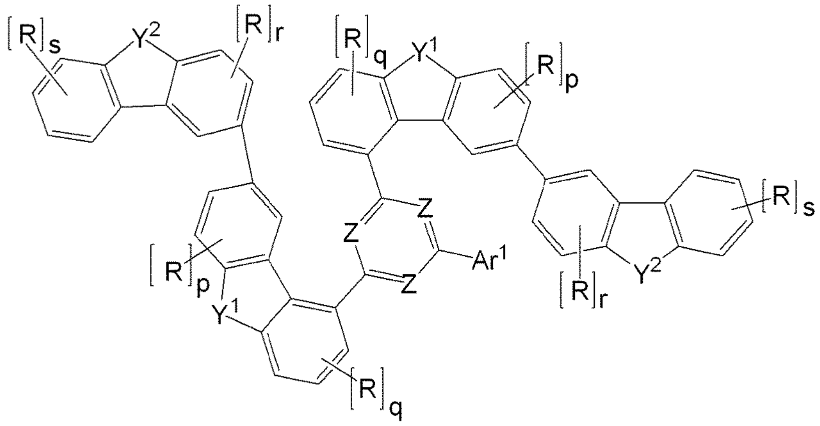

Formula (1a)

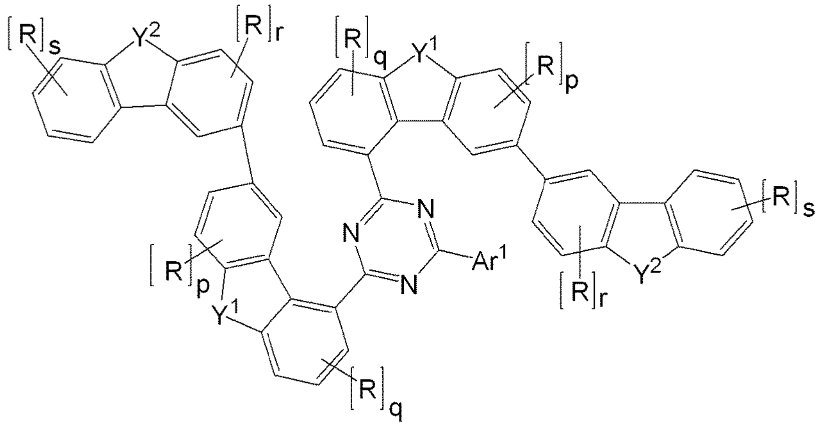

Formula (2a)

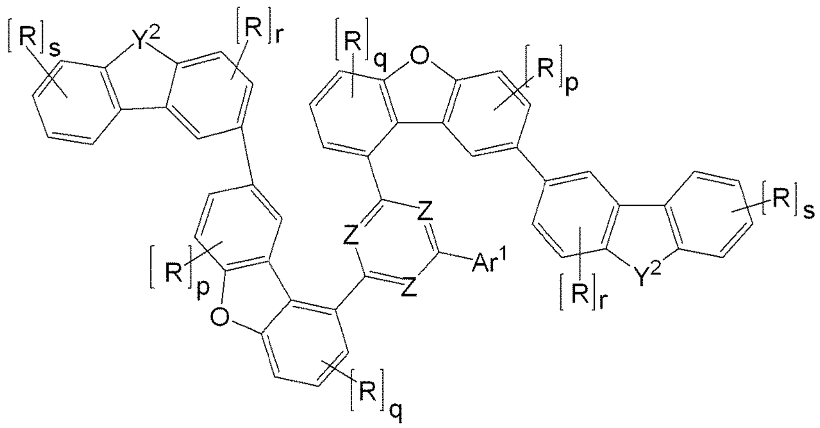

Formula (3a)

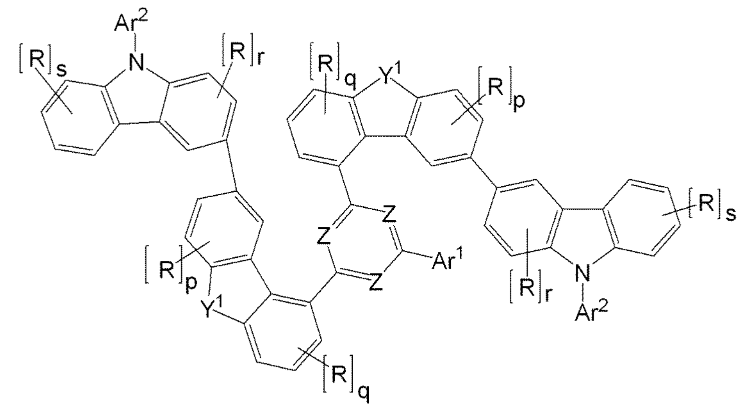

Formula (4a)

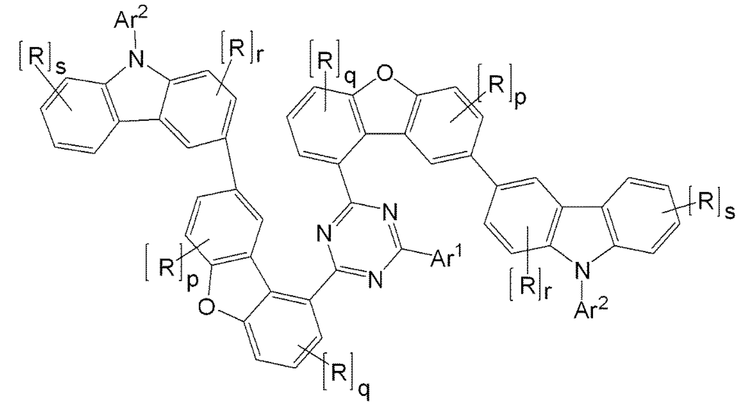

Formula (5a)

where the symbols and indices have the definitions given in Claim 1.

**8.** Compound according to one or more of Claims 1 to 7 of formula (6a)

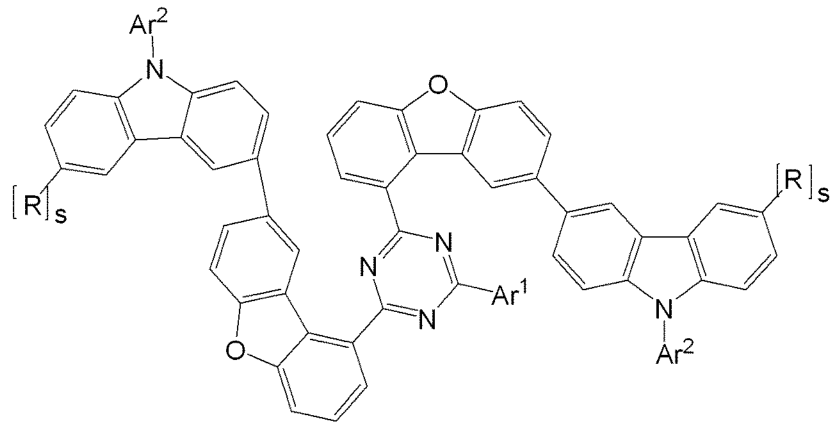

Formula (6a)

where the symbols have the definitions given in Claim 1 and s is the same or different at each instance and is 0 or 1.

**9.** Compound according to one or more of Claims 1 to 8, **characterized in that** $Ar^1$ and $Ar^2$ are the same or different at each instance and are each selected from the group consisting of phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, phenanthrene, triphenylene or a combination of two or three of these

groups, each of which may be substituted by one or more R radicals.

**10.** Process for preparing a compound according to one or more of Claims 1 to 9 by reacting an $Ar^1$-functionalized dichloropyrimidine or -triazine with a 1-dibenzofuranboronic acid or ester thereof or a 1-dibenzothiopheneboronic acid or ester thereof in a Suzuki coupling.

**11.** Formulation comprising at least one compound according to one or more of Claims 1 to 9 and at least one further compound and/or a solvent.

**12.** Use of a compound according to one or more of Claims 1 to 9 or of a formulation according to Claim 11 in an electronic device.

**13.** Electronic device comprising at least one compound according to one or more of Claims 1 to 9 or a formulation according to Claim 11.

**14.** Electronic device according to Claim 13 which is an organic electroluminescent device, **characterized in that** the compound according to one or more of Claims 1 to 9 is used in an emitting layer as matrix material for phosphorescent emitters or for emitters that exhibit TADF (thermally activated delayed fluorescence) and/or in an electron transport layer and/or in a hole blocker layer.

**15.** Electronic device according to Claim 14, **characterized in that** the compound according to one or more of Claims 1 to 9 is used in an emitting layer as matrix material for a phosphorescent emitter, wherein the compound is used in combination with one or more further matrix materials selected from the group consisting of aromatic ketones, aromatic phosphine oxides, aromatic sulfoxides, aromatic sulfones, triarylamines, carbazole derivatives, indolocarbazole derivatives, indenocarbazole derivatives, azacarbazole derivatives, bipolar matrix materials, azaboroles, boronic esters, triazine derivatives, zinc complexes, diazasilole or tetraazasilole derivatives, diazaphosphole derivatives, bridged carbazole derivatives, triphenylene derivatives, dibenzofuran derivatives, dibenzothiophene derivatives, wide bandgap materials and/or phosphorescent compounds having shorter-wave emission than the actual emitter.

## Revendications

**1.** Composé selon la formule (1)

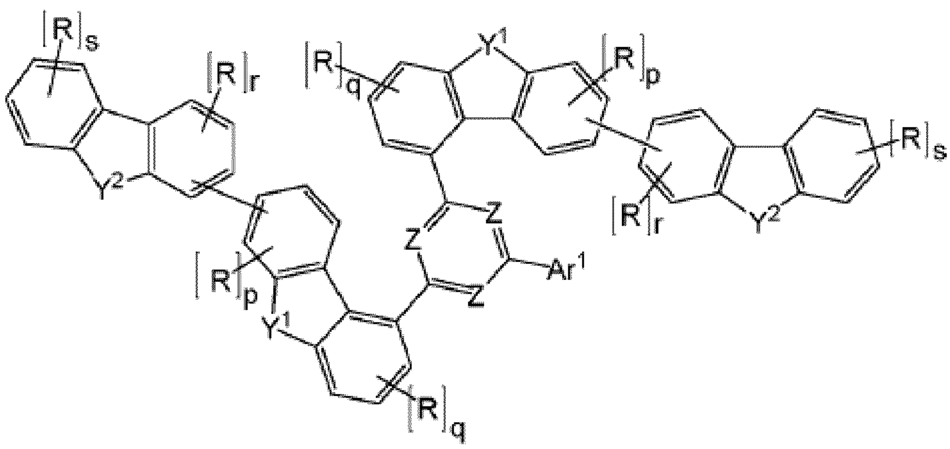

Formule (1)

s'appliquant pour les symboles et indices utilisés ce qui suit :

$Y^1$ est de manière identique ou différente à chaque occurrence O ou S ;
$Y^2$ est de manière identique ou différente à chaque occurrence $NAr^2$, O, S ou $CR_2$ ;
Z est de manière identique ou différente à chaque occurrence CR ou N à condition qu'au moins deux Z repré-

sentent N ;

$Ar^1$, $Ar^2$ sont de manière identique ou différente à chaque occurrence un système à noyau aromatique ou hétéroaromatique avec 5 à 40 atomes de noyau aromatiques, lequel peut être substitué par un ou plusieurs radicaux R ;

R est de manière identique ou différente à chaque occurrence H, D, F, Cl, Br, I, $N(Ar')_2$, $N(R^1)_2$, OAr', SAr', CN, $NO_2$, $OR^1$, $SR^1$, $COOR^1$, $C(=O)N(R^1)_2$, $Si(R^1)_3$, $B(OR^1)_2$, $C(=O)R^1$, $P(=O)(R^1)_2$, $S(=O)R^1$, $S(=O)_2R^1$, $OSO_2R^1$, un groupe alkyle à chaîne droite avec 1 à 20 atomes C ou un groupe alcényle ou alcynyle avec 2 à 20 atomes C ou un groupe alkyle ramifié ou cyclique avec 3 à 20 atomes C, le groupe alkyle, alcényle ou alcynyle pouvant chacun être substitué par un ou plusieurs radicaux $R^1$, un ou plusieurs groupes $CH_2$ non adjacents pouvant être remplacés par $Si(R^1)_2$, C=O, $NR^1$, O, S ou $CONR^1$, ou un système à noyau aromatique ou hétéroaromatique avec 5 à 60 atomes de noyau aromatiques, de manière préférée avec 5 à 40 atomes de noyau aromatiques, chacun d'eux pouvant être substitué par un ou plusieurs radicaux $R^1$ ; deux radicaux R pouvant alors également former l'un avec l'autre un système à noyau ;

Ar' est de manière identique ou différente à chaque occurrence un système à noyau aromatique ou hétéroaromatique avec 5 à 40 atomes de noyau aromatiques, lequel peut être substitué par un ou plusieurs radicaux $R^1$ ; deux radicaux Ar' , lesquels se lient au même atome N, pouvant alors être pontés l'un à l'autre également par une liaison simple ou un pont, choisi parmi $N(R^1)$, $C(R^1)_2$, O ou S ;

$R^1$ est de manière identique ou différente à chaque occurrence H, D, F, Cl, Br, I, $N(R^2)_2$, CN, $NO_2$, $OR^2$, $SR^2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle à chaîne droite avec 1 à 20 atomes C ou un groupe alcényle ou alcynyle avec 2 à 20 atomes C ou un groupe alkyle ramifié ou cyclique avec 3 à 20 atomes C, le groupe alkyle, alcényle ou alcynyle pouvant chacun être substitué par un ou plusieurs radicaux $R^2$, un ou plusieurs groupes $CH_2$ non adjacents pouvant être remplacés par $Si(R^2)_2$, C=O, $NR^2$, O, S ou $CONR^2$ et un ou plusieurs atomes H dans le groupe alkyle, alcényle ou alcynyle pouvant être remplacés par D, F, Cl, Br, I ou CN, ou un système à noyau aromatique ou hétéroaromatique avec 5 à 40 atomes de noyau aromatiques, chacun d'eux pouvant être substitué par un ou plusieurs radicaux $R^2$ ; deux ou plusieurs radicaux $R^1$ pouvant alors former l'un avec l'autre un système à noyau aliphatique ;

$R^2$ est de manière identique ou différente à chaque occurrence H, D, F, CN ou un radical organique aliphatique, aromatique ou hétéroaromatique avec 1 à 20 atomes C, dans lequel un ou plusieurs atomes H peuvent également être remplacés par F ;

p, q, r sont de manière identique ou différente à chaque occurrence 0, 1, 2 ou 3 ;

s est de manière identique ou différente à chaque occurrence 0, 1, 2, 3 ou 4.

**2.** Composé selon la revendication 1 selon la formule (2),

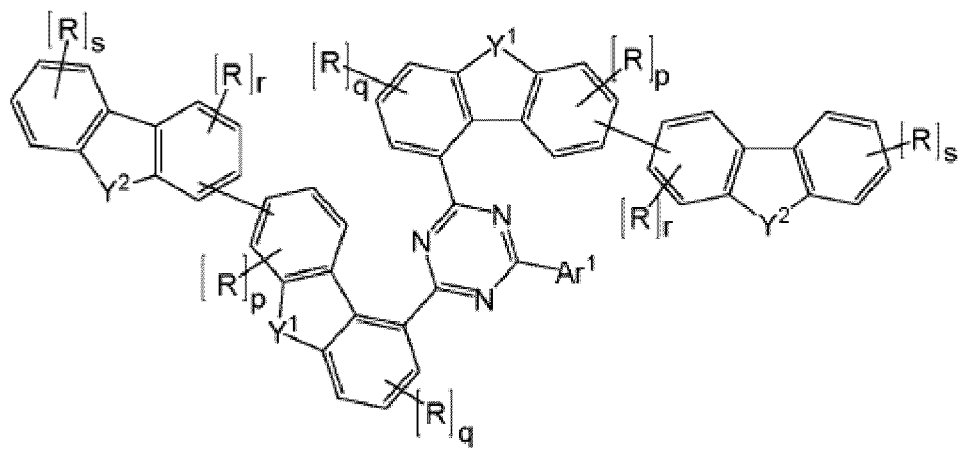

Formule (2)

les symboles et indices présentant les significations mentionnées dans la revendication 1.

**3.** Composé selon la revendication 1 ou 2 selon la formule (3),

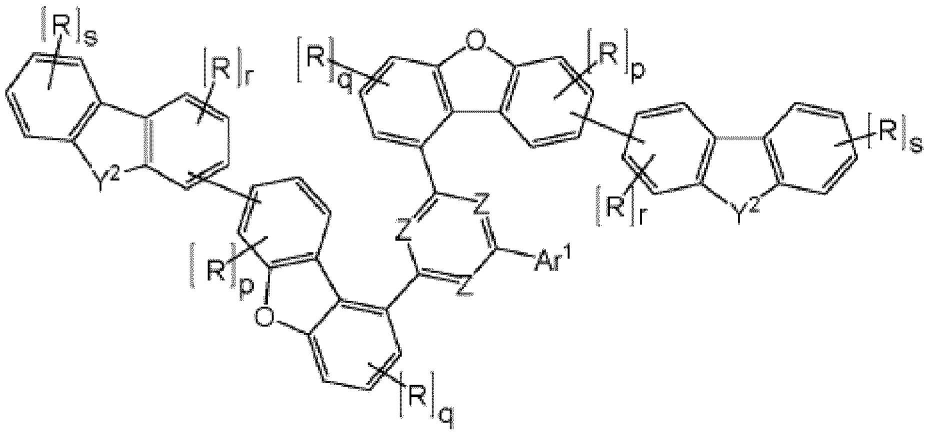

Formule (3)

les symboles et indices présentant les significations mentionnées dans la revendication 1.

**4.** Composé selon une ou plusieurs des revendications 1 à 3 selon la formule (4),

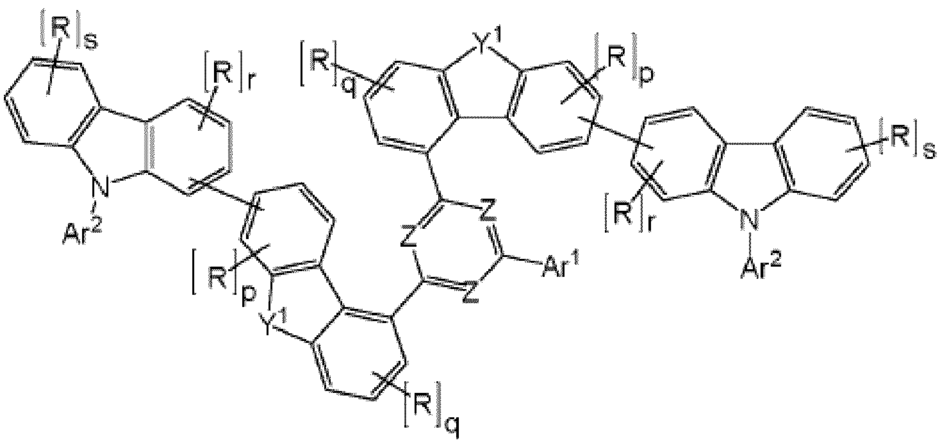

Formule (4)

les symboles et indices présentant les significations mentionnées dans la revendication 1.

**5.** Composé selon une ou plusieurs des revendications 1 à 4, **caractérisé en ce que** $Y^2$ représente $NAr^2$ et le groupe carbazole comporte au moins deux radicaux R adjacents, qui forment l'un avec l'autre un système à noyau de manière à former une structure selon l'une des formules (CARB-1) à (CARB-6),

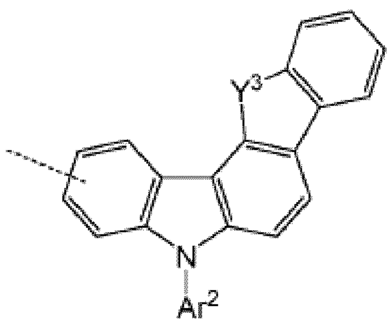

(CARB-1)

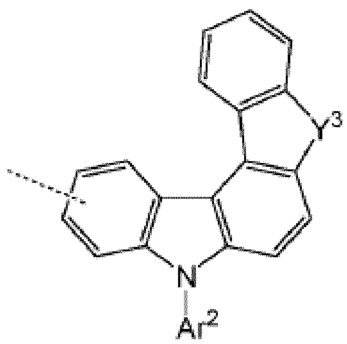

(CARB-2)

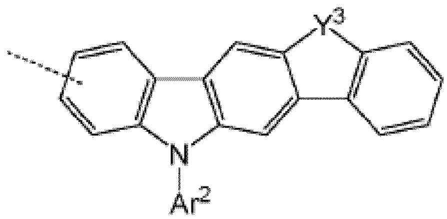

(CARB-3)

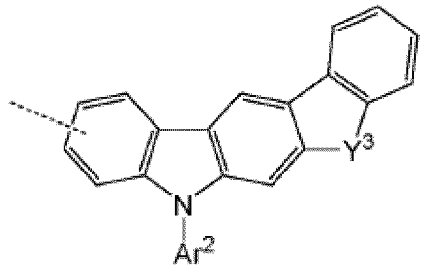

(CARB-4)

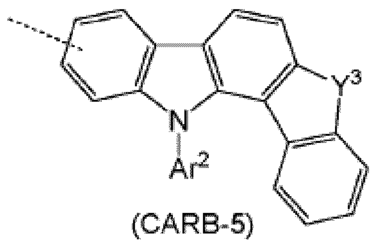

(CARB-5)

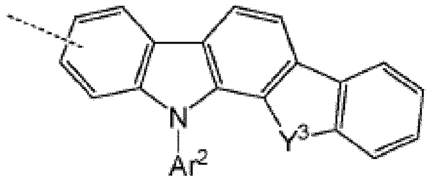

(CARB-6)

$Ar^2$ et $R^1$ présentant les significations mentionnées dans la revendication 1, les structures sur le carbazole pouvant être substituées par un ou plusieurs radicaux R et les structures sur la structure condensée pouvant être substituées par un ou plusieurs radicaux $R^1$, $Y^3$ représentant $C(R^3)_2$, $NR^1$, O ou S et les structures étant combinées au dibenzofurane ou au dibenzothiophène par l'intermédiaire de la liaison en pointillé.

**6.** Composé selon une ou plusieurs des revendications 1 à 5 selon la formule (5)

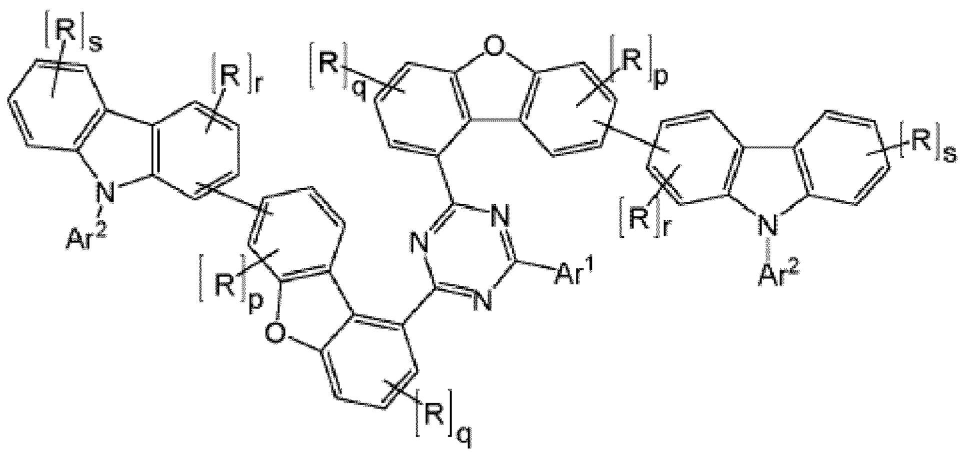

Formule (5)

les symboles et indices présentant les significations mentionnées dans la revendication 1.

**7.** Composé selon une ou plusieurs des revendications 1 à 6 selon l'une des formules (1a) à (5a),

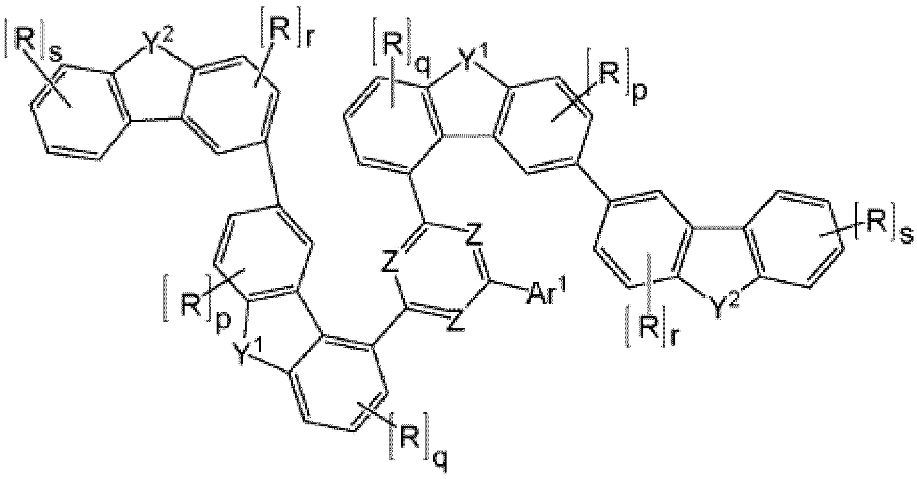

Formule (1a)

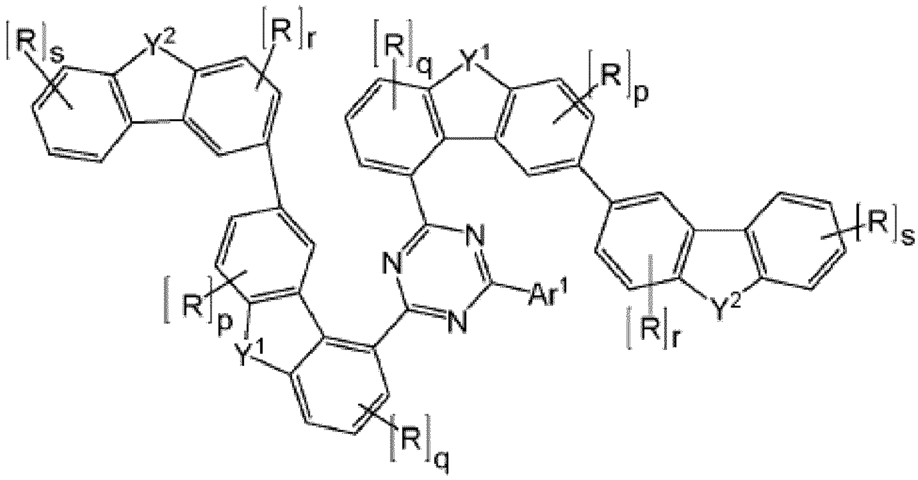

Formule (2a)

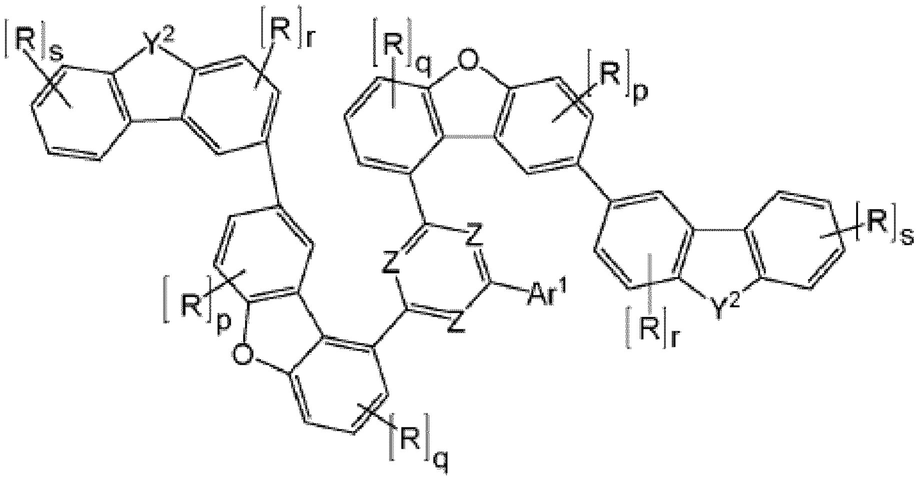

Formule (3a)

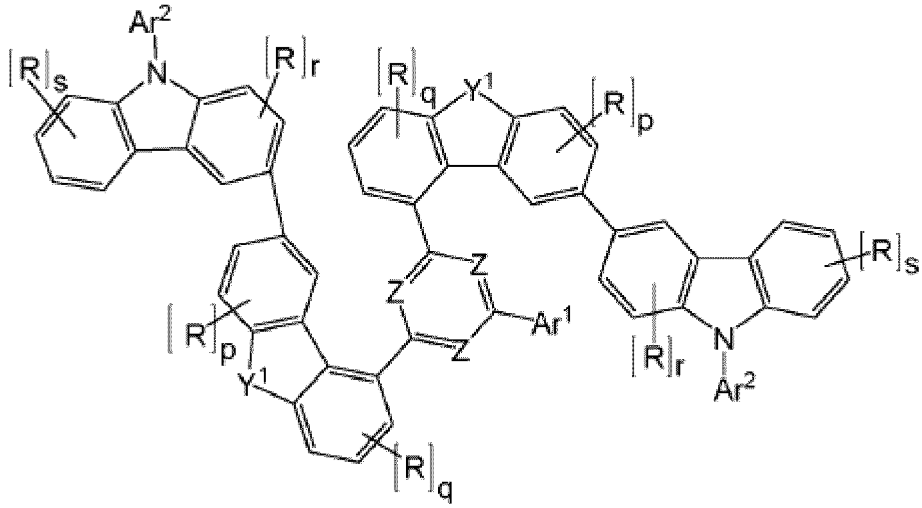

Formule (4a)

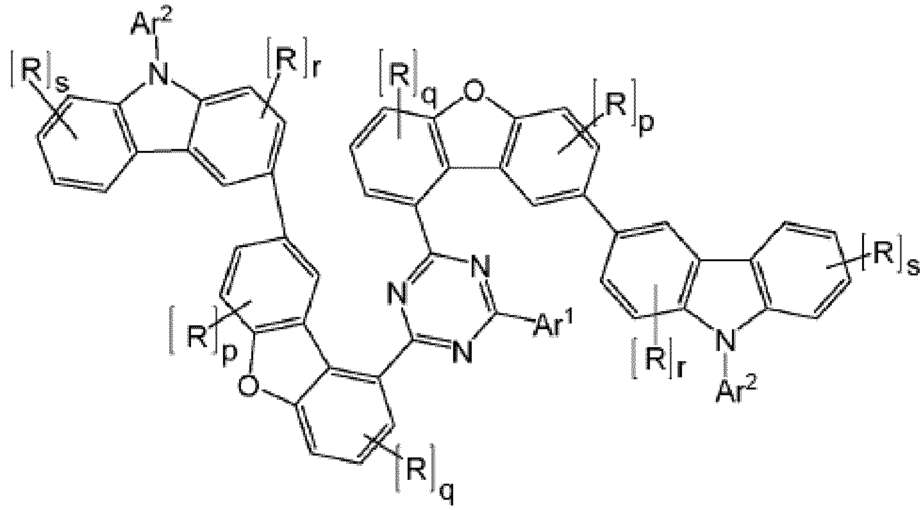

Formule (5a)

les symboles et les indices présentant les significations mentionnées dans la revendication 1.

**8.** Composé selon une ou plusieurs des revendications 1 à 7 selon la formule (6a),

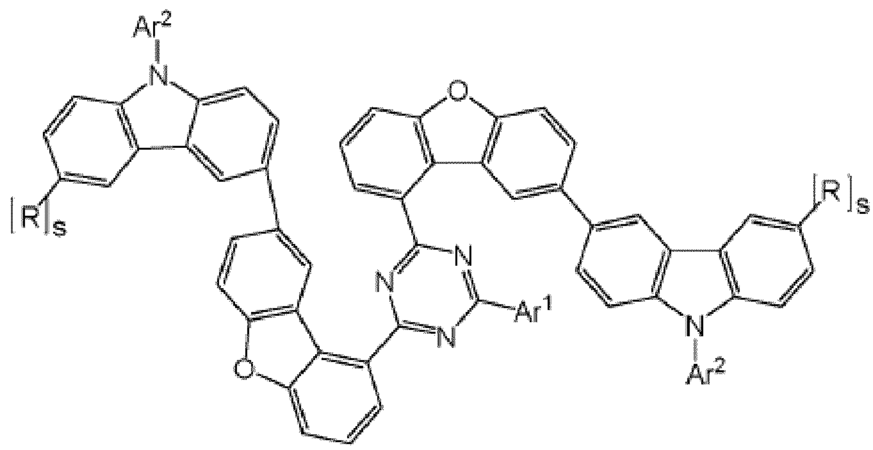

Formule (6a)

les symboles présentant les significations mentionnées dans la revendication 1 et s étant de manière identique ou différente à chaque occurrence 0 ou 1.

**9.** Composé selon une ou plusieurs des revendications 1 à 8,
**caractérisé en ce que** $Ar^1$ et $Ar^2$ sont chacun choisis de manière identique ou différente à chaque occurrence dans le groupe comprenant phényle, biphényle, terphényle, quaterphényle, fluorène, spirobifluorène, naphtaline, indole, benzofurane, benzothiophène, carbazole, dibenzofurane, dibenzothiophène, indénocarbazole, indolocarbazole phénanthrène, triphénylène, ou une combinaison de deux ou trois de ces groupes, qui peuvent chacun être substitués par un ou plusieurs radicaux R.

**10.** Procédé de fabrication d'un composé selon une ou plusieurs des revendications 1 à 9 par réaction d'une dichloropyrimidine ou dichlorotriazine, fonctionnalisée avec $Ar^1$, avec un acide 1-dibenzofurane-boronique ou ses esters ou avec un acide 1-dibenzothiophène-boronique ou ses esters dans un couplage de Suzuki.

**11.** Formulation contenant au moins un composé selon une ou plusieurs des revendications 1 à 9 et au moins un autre composé et/ou un solvant.

**12.** Utilisation d'un composé selon une ou plusieurs des revendications 1 à 9 ou d'une formulation selon la revendication 11 dans un dispositif électronique.

**13.** Dispositif électronique contenant au moins un composé selon une ou plusieurs des revendications 1 à 9 ou une formulation selon la revendication 11.

**14.** Dispositif électronique selon la revendication 13, le dispositif électronique étant un dispositif électroluminescent organique, **caractérisé en ce que** le composé selon une ou plusieurs des revendications 1 à 9 est employé dans une couche d'émission en tant que matériau de matrice pour des émetteurs phosphorescents ou pour des émetteurs à fluorescence retardée thermiquement activée, et/ou dans une couche de transport d'électrons et/ou dans une couche de blocage de trous.

**15.** Dispositif électronique selon la revendication 14, **caractérisé en ce que** le composé selon une ou plusieurs des revendications 1 à 9 est employé dans une couche d'émission en tant que matériau de matrice pour un émetteur phosphorescent, le composé étant employé en combinaison avec un ou plusieurs autres matériaux de matrice qui sont choisis dans le groupe comprenant les cétones aromatiques, oxydes de phosphine aromatiques, sulfoxydes aromatiques, sulfones aromatiques, triarylamines, dérivés de carbazole, dérivés d'indolocarbazole, dérivés d'indénocarbazole, dérivés d'azacarbazole, matériaux de matrice bipolaires, azaborolène, esters boroniques, dérivés de triazine, complexes de zinc, dérivés de diazasilol ou de tétraazasilol, dérivés de diazaphosphole, dérivés de carbazole pontés, dérivés de triphénylène, dérivés de dibenzofurane, dérivés de dibenzothiophène, matériaux à large bande interdite et/ou composés phosphorescents, lesquels émettent des ondes courtes en tant qu'émetteur à proprement parler.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2015169412 A
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- WO 2017032439 A
- WO 2018011186 A
- WO 2018041769 A
- WO 2019020538 A
- WO 2018178001 A
- WO 2019115423 A
- WO 2019158453 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- WO 2013041176 A
- WO 2007063754 A
- WO 2008056746 A
- WO 2010136109 A
- WO 2011000455 A
- WO 2013056776 A
- EP 1617710 A
- EP 1617711 A
- EP 1731584 A
- JP 2005347160 A
- WO 2007137725 A
- WO 2005111172 A
- WO 2006117052 A
- WO 2010015306 A
- WO 2011057706 A
- WO 2011060859 A
- WO 2011060877 A
- EP 652273 A
- WO 2009062578 A
- WO 2010054729 A
- WO 2010054730 A
- WO 2011042107 A
- WO 2011060867 A
- WO 2011088877 A
- WO 2012143080 A
- WO 2012048781 A
- WO 2016015810 A
- WO 2016023608 A
- WO 2017148564 A
- WO 2017148565 A
- WO 2010108579 A
- WO 2016184540 A
- EP 19152285
- WO 2004058911 A
- WO 2004037887 A
- WO 2010097155 A

### In der Beschreibung aufgeführte Nicht-Patentliteratur

- **M. S. ARNOLD et al.** *Appl. Phys. Lett.*, 2008, vol. 92, 053301
- *CHEMICAL ABSTRACTS,* 1822311-11-4
- *CHEMICAL ABSTRACTS,* 1846559-20-3

- *CHEMICAL ABSTRACTS,* 1416814-68-0
- *CHEMICAL ABSTRACTS,* 1547397-15-8
- *CHEMICAL ABSTRACTS,* 1822311-12-5
- *CHEMICAL ABSTRACTS,* 137055-65-9
- *CHEMICAL ABSTRACTS,* 2260995-43-3
- *CHEMICAL ABSTRACTS,* 1427160-10-8
- *CHEMICAL ABSTRACTS,* 112719-97-8
- *CHEMICAL ABSTRACTS,* 1845707-19-8
- *CHEMICAL ABSTRACTS,* 10202-45-6
- *CHEMICAL ABSTRACTS,* 1644054-61-4
- *CHEMICAL ABSTRACTS,* 1700-02-3
- *CHEMICAL ABSTRACTS,* 2102042-41-9